# EUROPEAN PATENT APPLICATION

(11) **EP 4 636 089 A1**
(43) Date of publication of application: **22.10.2025**
(21) Application number: 23891595.3
(22) Date of filing: 15.11.2023
(51) Int. Cl.: C12N 15/63, A61K 48/00, A61P 35/00, A61P 43/00, C12Q 1/04, C12Q 1/68

(54) **NUCLEIC ACID CONSTRUCT CAPABLE OF SELECTIVELY DETECTING OR KILLING MISMATCH REPAIR-DEFICIENT CELL, AND USE THEREOF**

(30) Priority: 18.11.2022 JP 2022185011
(71) Applicant: PUBLIC UNIVERSITY CORPORATION YOKOHAMA CITY UNIVERSITY, Yokohama-shi, Kanagawa 236-0027 (JP)
(72) Inventor: ADACHI, Noritaka, Yokohama-shi, Kanagawa 236-0027 (JP)
(74) Representative: Mewburn Ellis LLP
(86) International application number: PCT/JP2023/041021
(87) International publication number: WO 2024/106448

(57) **Abstract**

A nucleic acid construct of the present invention has a structure in which protein expression becomes possible by occurrence of two or more times of recombination reaction by single-strand annealing (SSA). The construct is useful for diagnosis and treatment of mismatch repair (MMR)-deficient cancer, and is applicable to search for factors that regulate MMR or SSA reaction, search for inhibitors or activators of MMR or SSA reaction, and prediction of whether or not a gene mutation identified in a cancer patient is a mutation that impairs MMR activity. A conventional SSA construct that has been previously developed by the present inventor had a structure that allows protein expression by a single SSA reaction, whereas the construct of the present invention tends to exhibit a larger difference in the expression level (activity) of a protein due to the presence or absence of MMR deficiency. Therefore, the construct of the present invention can be expected to act more specifically on MMR-deficient cells, and hence to provide, for example, a therapeutic means with lower side effects.

## Description

### TECHNICAL FIELD

The present invention relates to a nucleic acid construct, and a therapeutic agent or a diagnostic agent for mismatch repair-deficient cancer comprising the nucleic acid construct.

### BACKGROUND ART

Mismatch repair is one of the DNA damage-repairing mechanisms cells possess, and plays an important role in maintenance of stability of the genome (Non-Patent Documents 1 and 2). In human cells, proteins encoded by genes such as *MSH2, MSH6, MLH1,* and *PMS2* are involved in mismatch repair, and cells deficient in these factors lose the mismatch repair activity. Abnormalities of factors that affect gene expression of these mismatch repair proteins also cause mismatch repair deficiency.

Mismatch repair deficiency causes various cancers (when mispairing (mismatch) of bases in DNA remains unrepaired, it leads to accumulation of genomic mutations, resulting in high carcinogenicity). A number of deficiencies of mismatch repair factors have been reported, and their examples include not only Lynch syndrome (also called hereditary non-polyposis colorectal cancer (HNPCC)), which is a well-known familial (genetic) tumor, but also various sporadic (non-genetic) cancers (especially colorectal cancer and endometrial cancer) (Non-Patent Document 3).

Diagnosis of a mismatch repair-deficient cancer is carried out based on microsatellite instability (MSI) or by immunostaining of the four factors described above. However, their results do not show a rate of concordance of 100% (Non-Patent Document 4). Further, in the latter method, mismatch repair deficiencies caused by abnormalities of factors other than the four factors may be missed (this applies also to genetic diagnosis on the four factors). Both methods require several days to several weeks for the diagnosis.

Regarding the treatment, immune checkpoint inhibitors (such as anti-PD-1 antibodies) have been found quite recently to produce a high response rate in mismatch repair-deficient cancers, and are therefore attracting a lot of attention (Non-Patent Documents 3 and 5). However, immune checkpoint inhibitors have side effects of autoimmune diseases due to immune suppression, and lethal cases have been reported. Thus, development of safer and less expensive therapeutic methods is also important.

### PRIOR ART DOCUMENTS

### PATENT DOCUMENTS

Patent Document 1: WO 2021/162121 A1
Patent Document 2: WO 2021/162120 A1

### NON-PATENT DOCUMENTS

Non-Patent Document 1: Jiricny J (2006) The multifaceted mismatch-repair system. Nat Rev Mol Cell Biol7(5):335-346.
Non-Patent Document 2: Harfe BD and Jinks-Robertson S (2000) DNA mismatch repair and genetic instability. Annu Rev Genet 34:359-399.
Non-Patent Document 3: Le DT et al. Science. 2017 Jul 28; 357(6349):409-413
Non-Patent Document 4: Hampel H et al. N Engl J Med 352:1851-1860, 2015
Non-Patent Document 5: Le DT et al. N Engl J Med 2015; 372: 2509-2520.
Non-Patent Document 6: Stark JM et al. Mol Cell Biol. 2004 Nov; 24(21):9305-9316.
Non-Patent Document 7: Mendez-Dorantes C et al. Genes Dev. 2018 Apr 1; 32(7-8):524-536.
Non-Patent Document 8: Ochiai H et al. Genes Cells. 2010 Aug; 15(8):875-885.

### SUMMARY OF THE INVENTION

### PROBLEMS TO BE SOLVED BY THE INVENTION

If there is a method that enables simple and rapid detection of the presence or absence of mismatch repair activity in cells, tissues, or individuals, the method is expected to be extremely useful for diagnosis and treatment of mismatch repair-deficient cancers including Lynch syndrome, irrespective of the types of the cancers. For example, in cancer treatment using an immune checkpoint inhibitor, if whether mismatch repair is normal or not can be diagnosed in advance, the risk of occurrence of a serious side effect due to administration of the immune checkpoint inhibitor to a patient in whom it produces low response rate can be avoided.

As a system capable of detecting the presence or absence of mismatch repair activity, the present inventor has previously developed a nucleic acid construct in which two divided fragments of a gene sequence encoding a protein are incorporated into an expression vector, wherein the fragments are designed to have regions homologous to each other (Patent Document 1). This construct is a construct in which recombination reaction by single-strand annealing (SSA) occurs between the pair of homologous regions to reproduce the gene sequence, to thereby enable expression of the protein. Introduction of the construct into a cell whose mismatch repair activity is deteriorated causes the reproduction of the gene sequence by the action of the SSA mechanism, resulting in the expression of the protein.

The present inventor has also developed a construct having a structure in which a gene sequence encoding a protein is reproduced by homologous recombination (HR) to enable expression of the protein (Patent Document 2).

An object of the present invention is to provide a novel means having better characteristics than those of the constructs that have so far been developed, which means enables simple and rapid detection of the presence or absence of mismatch repair activity and is useful for diagnosis and treatment of mismatch repair-deficient cancers.

### MEANS FOR SOLVING THE PROBLEMS

As a result of a further intensive study, the present inventor successfully developed a nucleic acid construct having a structure that enables protein expression by occurrence of two or more times of recombination reaction by SSA, and discovered that this nucleic acid construct is useful for the diagnosis and treatment of mismatch repair-deficient cancers, thereby completing the present invention.

More specifically, the present invention is an invention related to a nucleic acid construct that enables protein expression by two or more times of SSA reaction and use thereof, and includes the following aspects.

### (Nucleic Acid Construct of First Aspect)

[1] A nucleic acid construct comprising:
   a promoter region;
   a 5'-side region of a gene sequence encoding protein A;
   a 3'-side region of a gene sequence encoding protein A; and
   at least one complementary region comprising: a homologous region α for which a region comprising at least a 3'-end portion of the 5'-side region serves as a substrate in a recombination reaction by single-strand annealing; and a homologous region β for which a region comprising at least a 5'-end portion of the 3'-side region serves as a substrate in said recombination reaction;
   the promoter region, the 5'-side region, the 3'-side region, and the at least one complementary region being placed in one nucleic acid molecule or in two or more different nucleic acid molecules,
   wherein the homologous region α and the homologous region β are contained in the same complementary region or different complementary regions, and in the latter case, at least one complementary region comprises one or more additional sets of a homologous region and a substrate that undergo said recombination reaction,
   wherein the homology between each homologous region and each region that serves as the substrate therefor is not less than 40% and less than 100%, and
   wherein said recombination reaction forms a nucleic acid whose base sequence encodes protein A, or protein B which has the same activity as protein A, resulting in expression of protein A or protein B.
[2] The nucleic acid construct according to [1], wherein the amino acid sequence of protein B has a sequence identity of not less than 80% and less than 100% to the amino acid sequence of protein A.
[3] The nucleic acid construct according to [1] or [2], wherein the promoter region, the 5'-side region, and the 3'-side region are placed in the same nucleic acid molecule.
[4] The nucleic acid construct according to claim [1] or [2], wherein the promoter region and the 5'-side region are placed in one nucleic acid molecule, and the 3'-side region is placed in another nucleic acid molecule.
[5] The nucleic acid construct according to any one of [1] to [4], wherein a poly-A addition signal is functionally linked downstream of the 3'-side region.
[6] The nucleic acid construct according to [3], composed of two nucleic acid molecules and comprising one complementary region,
   wherein:
   a nucleic acid molecule 1 comprises the promoter region, the 5'-side region, and the 3'-side region;
   a nucleic acid molecule 2 comprises a complementary region comprising the homologous region α and the homologous region β;
   the homology between the homologous region α and the region that comprises at least the 3'-end portion of the 5'-side region and that serves as the substrate for the homologous region α in the recombination reaction is not less than 40% and less than 100%, and the homology between the homologous region β and the region that comprises at least the 5'-end portion of the 3'-side region and that serves as the substrate for the homologous region β in the recombination reaction is not less than 40% and less than 100%; and
   the 5'-side region, the complementary region, and the 3'-side region, when arranged in this order, encode an amino acid sequence of protein A or protein B while each homologous region and the substrate therefor overlap each other, and the recombination reaction that occurs between each homologous region and the substrate therefor results in expression of protein A or protein B.
[7] The nucleic acid construct according to [3], composed of *n* nucleic acid molecules and comprising (*n*-1) complementary regions,
   wherein:
   a nucleic acid molecule 1 comprises the promoter region, the 5'-side region, and the 3'-side region;
   a nucleic acid molecule 2 comprises a first complementary region comprising a first homologous region and a second homologous region;
   an *mth* nucleic acid molecule *m* comprises an (*m*-1)th complementary region comprising an (*m*-1)th substrate region and an *m*th homologous region;
   the first homologous region is the homologous region α, and the homology between the first homologous region and the region that comprises at least the 3'-end portion of the 5'-side region and that serves as the substrate for the first homologous region in said recombination reaction is not less than 40% and less than 100%;
   an (*m*-1)th homologous region is a region for which the (*m*-1)th substrate region serves as a substrate in said recombination reaction, and the homology between these regions is not less than 40% and less than 100%;
   an *n*th homologous region contained in a nucleic acid molecule *n* is the homologous region β, and the homology between the *n*th homologous region and the region that comprises at least the 5'-end portion of the 3'-side region and that serves as the substrate for the *n*th homologous region in the recombination reaction is not less than 40% and less than 100%; and
   the 5'-side region, the first complementary region, the *(m-1)th* complementary region, and the 3'-side region, when arranged in this order, encode an amino acid sequence of protein A or protein B while each homologous region and the substrate therefor overlap each other, and the recombination reaction that occurs between each homologous region and the substrate therefor results in expression of protein A or protein B
   (wherein the integer *n* is a constant of 3 ≤ *n* ≤ 10, and the integer *m* is a variable of 3 ≤ *m* ≤*n*).
[8] The nucleic acid construct according to [6] or [7], wherein the nucleic acid molecule 1 is a linear nucleic acid molecule comprising, downstream of the 3'-side region, the promoter region and the 5'-side region in this order, or a circular nucleic acid molecule comprising, downstream of the promoter region, the 5'-side region and the 3'-side region in this order.
[9] The nucleic acid construct according to [8], wherein the circular nucleic acid molecule comprises a cleavage site between the 5'-side region and the 3'-side region.
[10] The nucleic acid construct according to [9], wherein the cleavage site is a restriction enzyme recognition site.
[11] The nucleic acid construct according to [4], composed of three nucleic acid molecules and comprising one complementary region,
   wherein:
   a nucleic acid molecule 1-1 comprises the promoter region and the 5'-side region;
   a nucleic acid molecule 1-2 comprises the 3'-side region;
   a nucleic acid molecule 2 comprises a complementary region comprising the homologous region α and the homologous region β;
   the homology between the homologous region α and the region that comprises at least the 3'-end portion of the 5'-side region and that serves as the substrate for the homologous region α in said recombination reaction is not less than 40% and less than 100%;
   the homology between the homologous region β and the region that comprises at least the 5'-end portion of the 3'-side region and that serves as the substrate for the homologous region β in said recombination reaction is not less than 40% and less than 100%; and
   the 5'-side region, the complementary region, and the 3'-side region, when arranged in this order, encode an amino acid sequence of protein A or protein B while each homologous region and the substrate therefor overlap each other, and the recombination reaction that occurs between each homologous region and the substrate therefor results in expression of protein A or protein B.
[12] The nucleic acid construct according to [4], composed of (*n*+1) nucleic acid molecules and comprising (*n*-1) complementary regions,
   wherein:
   a nucleic acid molecule 1-1 comprises the promoter region and the 5'-side region;
   a nucleic acid molecule 1-2 comprises the 3'-side region;
   a nucleic acid molecule 2 comprises a first complementary region comprising a first homologous region and a second homologous region;
   an *mth* nucleic acid molecule *m* comprises an (*m*-1)th complementary region comprising an (*m*-1)th substrate region and an *m*th homologous region;
   the first homologous region is the homologous region α, and the homology between the first homologous region and the region that comprises at least the 3'-end portion of the 5'-side region and that serves as the substrate for the first homologous region in the recombination reaction is not less than 40% and less than 100%;
   an (*m*-1)th homologous region is a region for which the (*m*-1)th substrate region serves as a substrate in said recombination reaction, and the homology between these regions is not less than 40% and less than 100%;
   an nth homologous region contained in a nucleic acid molecule *n* is the homologous region β, and the homology between the *n*th homologous region and the region that comprises at least the 5'-end portion of the 3'-side region and that serves as the substrate for the *n*th homologous region in said recombination reaction is not less than 40% and less than 100%; and
   the 5'-side region, the first complementary region, the *(m-1)th* complementary region, and the 3'-side region, when arranged in this order, encode an amino acid sequence of protein A or protein B while each homologous region and the substrate therefor overlap each other, and the recombination reaction that occurs between each homologous region and the substrate therefor results in expression of protein A or protein B
   (wherein the integer *n* is a constant of 3 ≤ *n* ≤ 10, and the integer *m* is a variable of 3 ≤ *m* ≤*n*).
[13] The nucleic acid construct according to [3], composed of one circular nucleic acid molecule and comprising one complementary region,
   wherein:
   the circular nucleic acid molecule comprises, downstream of the promoter region, the 5'-side region, a cleavage site, and the 3'-side region in this order, and comprises, upstream of the promoter region but downstream of the 3'-side region, a complementary region comprising the homologous region α and the homologous region β;
   the homology between the homologous region α and the region comprising at least the 3'-end portion of the 5'-side region which serves as a substrate for the homologous region α in said recombination reaction is not less than 40% and less than 100%;
   the homology between the homologous region β and the region comprising at least the 5'-end portion of the 3'-side region which serves as a substrate for the homologous region β in said recombination reaction is not less than 40% and less than 100%;
   the 5'-side region, the complementary region, and the 3'-side region, when arranged in this order, encode an amino acid sequence of protein A or protein B while each homologous region and the substrate therefor overlap each other, and the recombination reaction that occurs between each homologous region and the substrate therefor results in expression of protein A or protein B.
[14] The nucleic acid construct according to [3], composed of one circular nucleic acid molecule and comprising (*n*-1) complementary regions,
   wherein:
   the circular nucleic acid molecule comprises, downstream of the promoter region, the 5'-side region, a cleavage site, and the 3'-side region in this order, and comprises, upstream of the promoter region but downstream of the 3'-side region, (*n-*1) complementary regions;
   a first complementary region comprises a first homologous region and a second homologous region;
   an (*m*-1)th complementary region comprises an (*m*-1)th substrate region and an mth homologous region;
   the first homologous region is the homologous region α for which the region comprising at least the 3'-end portion of the 5'-side region serves as a substrate in said recombination reaction, and the homology between the substrate and the first homologous region is not less than 40% and less than 100%;
   an (*m*-1)th homologous region is a region for which the (*m*-1)th substrate region serves as a substrate in said recombination reaction, and the homology between these regions is not less than 40% and less than 100%;
   an nth homologous region contained in an (*n*-1)th complementary region is the homologous region β for which the region comprising at least the 5'-end portion of the 3'-side region serves as a substrate in said recombination reaction, and the homology between the substrate and the nth homologous region is not less than 40% and less than 100%;
   the 5'-side region, the first complementary region, the *(m-1)th* complementary region, and the 3'-side region, when arranged in this order, encode an amino acid sequence of protein A or protein B while each homologous region and the substrate therefor overlap each other, and the recombination reaction that occurs between each homologous region and the substrate therefor results in expression of protein A or protein B
   (wherein the integer *n* is a constant of 3 ≤ *n* ≤ 10, and the integer *m* is a variable of 3 ≤ *m* ≤*n*).
[15] The nucleic acid construct according to [13] or [14], wherein the cleavage site is a restriction enzyme recognition site.
[16] The nucleic acid construct according to [3], composed of one linear nucleic acid molecule and comprising one complementary region,
   wherein:
   the linear nucleic acid molecule comprises, from upstream to downstream, the 3'-side region, the complementary region, the promoter region, and the 5'-side region in this order;
   the complementary region comprises the homologous region α and the homologous region β;
   the homology between the homologous region α and the region comprising at least the 3'-end portion of the 5'-side region which serves as a substrate for the homologous region α in said recombination reaction is not less than 40% and less than 100%;
   the homology between the homologous region β and the region comprising at least the 5'-end portion of the 3'-side region which serves as a substrate for the homologous region β in said recombination reaction is not less than 40% and less than 100%;
   the 5'-side region, the complementary region, and the 3'-side region, when arranged in this order, encode an amino acid sequence of protein A or protein B while each homologous region and the substrate therefor overlap each other, and the recombination reaction that occurs between each homologous region and the substrate therefor results in expression of protein A or protein B.
[17] The nucleic acid construct according to [3], composed of one linear nucleic acid molecule and comprising (*n*-1) complementary regions,
   wherein:
   the linear nucleic acid molecule comprises, from upstream to downstream, the 3'-side region, the promoter region, and the 5'-side region in this order, and comprises, between the 3'-side region and the promoter region, (*n*-1) complementary regions;
   a first complementary region comprises a first homologous region and a second homologous region;
   an (*m*-1)th complementary region comprises an (*m*-1)th substrate region and an mth homologous region;
   the first homologous region is the homologous region α for which the region comprising at least the 3'-end portion of the 5'-side region serves as a substrate in said recombination reaction, and the homology between the substrate and the first homologous region is not less than 40% and less than 100%;
   an (*m*-1)th homologous region is a region for which the (*m*-1)th substrate region serves as a substrate in said recombination reaction, and the homology between these regions is not less than 40% and less than 100%;
   an *n*th homologous region contained in an (*n*-1)th complementary region is the homologous region β for which the region comprising at least the 5'-end portion of the 3'-side region serves as a substrate in said recombination reaction, and the homology between the substrate and the nth homologous region is not less than 40% and less than 100%;
   the 5'-side region, the first complementary region, the (*m*-1)th complementary region, and the 3'-side region, when arranged in this order, encode an amino acid sequence of protein A or protein B while each homologous region and the **substrate** therefor overlap each other, and the recombination reaction that occurs between each homologous region and the substrate therefor results in expression of protein A or protein B
   (wherein the integer *n* is a constant of 3 ≤ *n* ≤ 10, and the integer *m* is a variable of 3 ≤ *m* ≤*n*).
[18] The nucleic acid construct according to any one of [1] to [17], wherein the chain lengths of all homologous regions are at least 20 bases.
[19] The nucleic acid construct according to any one of [1] to [18], wherein the gene sequence encoding protein A is a gene sequence encoding a protein that acts to decrease cell survival rate, or a gene sequence encoding a protein whose intracellular expression is detectable.
[20] The nucleic acid construct according to [19], wherein the gene sequence encoding protein A is a sequence of a suicide gene, a DNA damage-inducing gene, a DNA repair-inhibiting gene, a luminescent enzyme gene, a fluorescent protein gene, a cell surface antigen gene, a secretory protein gene, or a membrane protein gene.
[21] A therapeutic agent for mismatch repair-deficient cancer, the agent comprising the nucleic acid construct according to any one of [1] to [20], wherein the gene sequence encoding protein A is a gene sequence encoding a protein that acts to decrease cell survival rate.
[22] The therapeutic agent according to [21], wherein the gene sequence encoding protein A is a sequence of a suicide gene, a DNA damage-inducing gene, or a DNA repair-inhibiting gene.
[23] A diagnostic agent for mismatch repair-deficient cancer, the agent comprising the nucleic acid construct according to any one of [1] to [20].
[24] The diagnostic agent according to [23], wherein the gene sequence encoding protein A is a gene sequence encoding a protein whose intracellular expression is detectable.
[25] A companion diagnostic agent for predicting an effect of an anticancer drug for mismatch repair-deficient cancer, the agent comprising the nucleic acid construct according to any one of [1] to [20].
[26] The companion diagnostic agent according to [25], wherein the anticancer drug is an immune checkpoint inhibitor.
[27] The companion diagnostic agent according to [25] or [26], wherein the gene sequence encoding protein A is a gene sequence encoding a protein whose intracellular expression is detectable.
[28] A therapeutic method for mismatch repair-deficient cancer, the method comprising administering the nucleic acid construct according to any one of [1] to [20] to a patient with the mismatch repair-deficient cancer, wherein the gene sequence encoding protein A is a gene sequence encoding a protein that acts to decrease cell survival rate.
[29] A diagnostic method for mismatch repair-deficient cancer, the method comprising:
   introducing the nucleic acid construct according to any one of [1] to [20] into cancer cells of a cancer patient; and
   measuring expression of protein A or protein B.
[30] The method according to [29], wherein the expression of protein A or protein B is measured by directly or indirectly measuring the activity of protein A or protein B.
[31] The method according to [29] or [30], wherein the cancer cells are cells separated from the cancer patient, and the introduction of the nucleic acid construct into the cancer cells is carried out *in vitro.*
[32] The method according to [29] or [30], wherein: protein A or protein B is a protein whose intracellular expression is detectable as a signal; the introduction of the nucleic acid construct into the cancer cells is carried out by administering the nucleic acid construct to the cancer patient; and whether or not the signal of the protein is detected from a cancer lesion is investigated.
[33] The method according to [29] or [30], wherein: protein A or protein B is a secretory protein whose intracellular expression is detectable; the introduction of the nucleic acid construct into the cancer cells is carried out by administering the nucleic acid construct to the cancer patient; and the activity of the protein in blood separated from the patient after the administration of the nucleic acid construct is measured.
[34] A method of predicting an effect of an anticancer drug for mismatch repair-deficient cancer, the method comprising:
   introducing the nucleic acid construct according to any one of [1] to [20] into cancer cells of a cancer patient; and
   measuring expression of protein A or protein B.
[35] The method according to [34], wherein the anticancer drug is an immune checkpoint inhibitor.
[36] The method according to [34] or [35], wherein the cancer cells are cells separated from the patient, and the introduction of the nucleic acid construct into the cancer cells is carried out *in vitro.*
[37] The method according to [34] or [35], wherein: protein A or protein B is a protein whose intracellular expression is detectable as a signal; the introduction of the nucleic acid construct into the cancer cells is carried out by administering the nucleic acid construct to the patient; and whether or not the signal of the protein is detected from a cancer lesion is investigated.
[38] The method according to [34] or [35], wherein: protein A or protein B is a secretory protein whose intracellular expression is detectable; the introduction of the nucleic acid construct into the cancer cells is carried out by administering the nucleic acid construct to the cancer patient; and the activity of the protein in blood separated from the patient after the administration of the nucleic acid construct is measured.

### (Nucleic Acid Construct of Second Aspect)

[39] A nucleic acid construct comprising:
   a promoter region;
   two substrate regions α and β;
   a gene sequence encoding protein X; and
   at least one complementary region comprising two homologous regions, wherein said two substrate regions serve as substrates for the two homologous regions, respectively, in a recombination reaction by single-strand annealing;
   the promoter region, the two substrate regions, the gene sequence, and the at least one complementary region being placed in one nucleic acid molecule or in two or more different nucleic acid molecules,
   wherein the two homologous regions are contained in the same complementary region or different complementary regions, and in the latter case, at least one complementary region comprises one or more additional sets of a homologous region and a substrate that undergo said recombination reaction,
   wherein the homology between each substrate region and its corresponding homologous region is not less than 40% and less than 100%, and
   wherein said recombination reaction results in expression of protein X.
[40] The nucleic acid construct according to [39], wherein the promoter region, the substrate region α, the substrate region β, and the gene sequence are placed in the same nucleic acid molecule.
[41] The nucleic acid construct according to [39], wherein the promoter region and the substrate region α are placed in one nucleic acid molecule, and the substrate region β and the gene sequence are placed in another nucleic acid molecule.
[42] The nucleic acid construct according to any one of [39] to [41], wherein a poly-A addition signal is functionally linked downstream of the gene sequence encoding protein X.
[43] The nucleic acid construct according to [40], composed of two nucleic acid molecules and comprising one complementary region,
   wherein:
   a nucleic acid molecule 1 comprises the promoter region, the substrate region α, the substrate region β, and the gene sequence encoding protein X;
   a nucleic acid molecule 2 comprises one complementary region comprising a first homologous region and a second homologous region;
   the first homologous region is a region for which the substrate region α serves as a substrate in said recombination reaction, and the homology between these regions is not less than 40% and less than 100%;
   the second homologous region is a region for which the substrate region β serves as a substrate in said recombination reaction, and the homology between these regions is not less than 40% and less than 100%; and
   the recombination reaction that occurs between each homologous region and the substrate therefor results in expression of protein X.
[44] The nucleic acid construct according to [40], composed of *n* nucleic acid molecules and comprising (*n*-1) complementary regions,
   wherein:
   a nucleic acid molecule 1 comprises the promoter region, the substrate region α, the substrate region β, and the gene sequence;
   a nucleic acid molecule 2 comprises a first complementary region comprising a first homologous region and a second homologous region;
   an *mth* nucleic acid molecule *m* comprises an (*m*-1)th complementary region comprising an (*m*-1)th substrate region and an *m*th homologous region;
   the first homologous region is a region for which the substrate region α serves as a substrate in said recombination reaction, and the homology between these regions is not less than 40% and less than 100%;
   an (*m*-1)th homologous region is a region for which the (*m*-1)th substrate region serves as a substrate in said recombination reaction, and the homology between these regions is not less than 40% and less than 100%;
   an *n*th homologous region contained in a nucleic acid molecule *n* is a region for which the substrate region β serves as a substrate in said recombination reaction, and the homology between these regions is not less than 40% and less than 100%; and
   the recombination reaction that occurs between each homologous region and the substrate therefor results in expression of protein X
   (wherein the integer *n* is a constant of 3 ≤ *n* ≤ 10, and the integer *m* is a variable of 3 ≤ *m* ≤*n*).
[45] The nucleic acid construct according to [43] or [44], wherein the nucleic acid molecule 1 is a circular nucleic acid molecule comprising, downstream of the promoter region, the substrate region α, the substrate region β, and the gene sequence encoding protein X in this order, or a linear nucleic acid molecule comprising, from upstream to downstream, the substrate region β, the gene sequence encoding protein X, the promoter region, and the substrate region α in this order.
[46] The nucleic acid construct according to [45], wherein the circular nucleic acid molecule comprises a cleavage site between the substrate region α and the substrate region β.
[47] The nucleic acid construct according to [46], wherein the cleavage site is a restriction enzyme recognition site.
[48] The nucleic acid construct according to [41], composed of three nucleic acid molecules and comprising one complementary region, wherein:
   a nucleic acid molecule 1-1 comprises the promoter region and the substrate region α;
   a nucleic acid molecule 1-2 comprises the substrate region β and the gene sequence;
   a first homologous region is a region for which the substrate region α serves as a substrate in said recombination reaction, and the homology between these regions is not less than 40% and less than 100%;
   a second homologous region is a region for which the substrate region β serves as a substrate in said recombination reaction, and the homology between these regions is not less than 40% and less than 100%; and
   the recombination reaction that occurs between each homologous region and the substrate therefor results in expression of protein X.
[49] The nucleic acid construct according to [41], composed of (*n*+1) nucleic acid molecules and comprising (*n*-1) complementary regions,
   wherein:
   a nucleic acid molecule 1-1 comprises the promoter region and the substrate region α;
   a nucleic acid molecule 1-2 comprises the substrate region β and the gene sequence;
   a nucleic acid molecule 2 comprises a first complementary region comprising a first homologous region and a second homologous region;
   an *mth* nucleic acid molecule *m* comprises an (*m*-1)th complementary region comprising an (*m*-1)th substrate region and an *m*th homologous region;
   the first homologous region is a region for which the substrate region α serves as a substrate in said recombination reaction, and the homology between these regions is not less than 40% and less than 100%;
   an (*m*-1)th homologous region is a region for which the (*m*-1)th substrate region serves as a substrate in said recombination reaction, and the homology between these regions is not less than 40% and less than 100%;
   an nth homologous region contained in a nucleic acid molecule *n* is a region for which the substrate region β serves as a substrate in said recombination reaction, and the homology between these regions is not less than 40% and less than 100%; and
   the recombination reaction that occurs between each homologous region and the substrate therefor results in expression of protein X
   (wherein the integer *n* is a constant of 3 ≤ *n* ≤ 10, and the integer *m* is a variable of 3 ≤ *m* ≤*n*).
[50] The nucleic acid construct according to [40], composed of one circular nucleic acid molecule and comprising one complementary region,
   wherein:
   the circular nucleic acid molecule comprises, downstream of the promoter region, the substrate region α, a cleavage site, the substrate region β, and the gene sequence encoding protein X in this order, and comprises, upstream of the promoter region but downstream of the gene sequence, a complementary region comprising a first homologous region and a second homologous region;
   the first homologous region is a region for which the substrate region α serves as a substrate in said recombination reaction, and the homology between these regions is not less than 40% and less than 100%;
   the second homologous region is a region for which the substrate region β serves as a substrate in said recombination reaction, and the homology between these regions is not less than 40% and less than 100%; and
   the recombination reaction that occurs between each homologous region and the substrate therefor results in expression of protein X.
[51] The nucleic acid construct according to [40], composed of one circular nucleic acid molecule and comprising (n-1) complementary regions,
   **wherein:**
   the circular nucleic acid molecule comprises, downstream of the promoter region, the substrate region α, a cleavage site, the substrate region β, and the gene sequence encoding protein X in this order, and comprises, upstream of the promoter region but downstream of the gene sequence, (*n*-1) complementary regions;
   a first complementary region comprises a first homologous region and a second homologous region;
   an (*m*-1)th complementary region comprises an (*m*-1)th substrate region and an *m*th homologous region;
   the first homologous region is a region for which the substrate region α serves as a substrate in the recombination reaction, and the homology between these regions is not less than 40% and less than 100%;
   an (*m*-1)th homologous region is a region for which the (*m*-1)th substrate region serves as a substrate in said recombination reaction, and the homology between these regions is not less than 40% and less than 100%;
   an *n*th homologous region contained in an (*n*-1)th complementary region is a region for which the substrate region β serves as a substrate in said recombination reaction, and the homology between these regions is not less than 40% and less than 100%; and
   the recombination reaction that occurs between each homologous region and the substrate therefor results in expression of protein X
   (wherein the integer *n* is a constant of 3 ≤ *n* ≤ 10, and the integer *m* is a variable of 3 ≤ *m* ≤*n*).
[52] The nucleic acid construct according to either one of [50] and [51], wherein the cleavage site is a restriction enzyme recognition site.
[53] The nucleic acid construct according to [40], composed of one linear nucleic acid molecule and comprising one complementary region, wherein:
   the linear nucleic acid molecule comprises, from upstream to downstream, the substrate region β, the gene sequence encoding protein X, the complementary region, the promoter region, and the substrate region α in this order;
   the complementary region comprises a first homologous region and a second homologous region;
   the first homologous region is a region for which the substrate region α serves as a substrate in said recombination reaction, and the homology between these regions is not less than 40% and less than 100%;
   the second homologous region is a region for which the substrate region β serves as a substrate in said recombination reaction, and the homology between these regions is not less than 40% and less than 100%; and
   the recombination reaction that occurs between each homologous region and the substrate therefor results in expression of protein X.
[54] The nucleic acid construct according to [40], composed of one linear nucleic acid molecule and comprising (*n*-1) complementary regions,
   wherein:
   the linear nucleic acid molecule comprises, from upstream to downstream, the substrate region β, the gene sequence encoding protein X, the promoter region, and the substrate region α in this order, and comprises (*n*-1) complementary regions between the gene sequence and the promoter region;
   a first complementary region comprises a first homologous region and a second homologous region;
   an (*m*-1)th complementary region comprises an (*m*-1)th substrate region and an mth homologous region;
   the first homologous region is a region for which the substrate region α serves as a substrate in said recombination reaction, and the homology between these regions is not less than 40% and less than 100%;
   an (*m*-1)th homologous region is a region for which the (*m*-1)th substrate region serves as a substrate in said recombination reaction, and the homology between these regions is not less than 40% and less than 100%;
   an *n*th homologous region contained in an (*n*-1)th complementary region is a region for which the substrate region β serves as a substrate in said recombination reaction, and the homology between these regions is not less than 40% and less than 100%; and
   the recombination reaction that occurs between each homologous region and the substrate therefor results in expression of protein X
   (wherein the integer *n* is a constant of 3 ≤ *n* ≤ 10, and the integer *m* is a variable of 3 ≤ *m* ≤*n*).
[55] The nucleic acid construct according to any one of [39] to [54], wherein protein X is a protein that acts to decrease cell survival rate, or a protein whose intracellular expression is detectable.
[56] The nucleic acid construct according to [55], wherein the gene sequence encoding protein X is a sequence of a suicide gene, a DNA damage-inducing gene, a DNA repair-inhibiting gene, a luminescent enzyme gene, a fluorescent protein gene, a cell surface antigen gene, a secretory protein gene, or a membrane protein gene.
[57] The nucleic acid construct according to any one of [39] to [56], wherein the base sequence generated by said recombination reaction between each substrate region and each homologous region is a gene sequence encoding protein Y, and the recombination reaction results in expression of protein X and protein Y.
[58] The nucleic acid construct according to [57], wherein a sequence that enables polycistronic expression is placed between the substrate region β and the gene sequence encoding protein X.
[59] The nucleic acid construct of [58], wherein the sequence that enables polycistronic expression is an IRES sequence or a 2A peptide-coding sequence.
[60] The nucleic acid construct according to any one of [57] to [59], wherein one of protein X and protein Y is a protein that acts to decrease cell survival rate, and the other is a protein whose intracellular expression is detectable.
[61] The nucleic acid construct according to [60], wherein one of the gene sequence encoding protein X and the gene sequence encoding protein Y is a sequence of a suicide gene, a DNA damage-inducing gene, or a DNA repair-inhibiting gene, and the other is a sequence of a luminescent enzyme gene, a fluorescent protein gene, a cell surface antigen gene, a secretory protein gene, or a membrane protein gene.
[62] A therapeutic agent for mismatch repair-deficient cancer, the agent comprising the nucleic acid construct according to any one of [39] to [61], wherein the gene sequence encoding protein X is a gene sequence encoding a protein that acts to decrease cell survival rate.
[63] The therapeutic agent according to [62], wherein the gene sequence encoding protein X is a sequence of a suicide gene, a DNA damage-inducing gene, or a DNA repair-inhibiting gene.
[64] An agent that detects and treats mismatch repair-deficient cancer, the agent comprising the nucleic acid construct according to [60] or [61].
[65] A diagnostic agent for mismatch repair-deficient cancer, the agent comprising the nucleic acid construct according to any one of [39] to [61].
[66] The diagnostic agent according to [65], wherein the gene sequence encoding protein X is a gene sequence encoding a protein whose intracellular expression is detectable.
[67] A companion diagnostic agent for predicting an effect of an anticancer drug for mismatch repair-deficient cancer, the agent comprising the nucleic acid construct according to any one of [39] to [61].
[68] The companion diagnostic agent according to [67], wherein the anticancer drug is an immune checkpoint inhibitor.
[69] The companion diagnostic agent according to [67] or [68], wherein the gene sequence encoding protein X is a gene sequence encoding a protein whose intracellular expression is detectable.
[70] A therapeutic method for mismatch repair-deficient cancer, the method comprising administering the nucleic acid construct according to any one of [39] to
[61] to a patient with the mismatch repair-deficient cancer, wherein the gene sequence encoding protein X is a gene sequence encoding a protein that acts to decrease cell survival rate.
[71] A method of detecting and treating mismatch repair-deficient cancer, the method comprising: administering, to a cancer patient, a nucleic acid construct according to [60] or [61] in which one of protein X and protein Y is a protein whose intracellular expression is detectable as a signal; and investigating whether or not the signal of the protein is detected from a cancer lesion.
[72] A method of detecting and treating mismatch repair-deficient cancer, the method comprising: administering, to a cancer patient, a nucleic acid construct according to [60] or [61] in which one of protein X and protein Y is a secretory protein whose intracellular expression is detectable; and measuring the activity of the protein in blood separated from the patient after the administration.
[73] A diagnostic method for mismatch repair-deficient cancer, the method comprising:
   introducing the nucleic acid construct according to any one of [39] to [61] into cancer cells of a cancer patient; and
   measuring expression of protein X.
[74] The method according to [73], wherein the expression of protein X is measured by directly or indirectly measuring the activity of protein X.
[75] The method according to [73] or [74], wherein the cancer cells are cells separated from the cancer patient, and the introduction of the nucleic acid construct into the cancer cells is carried out *in vitro.*
[76] The method according to [73] or [74], wherein: protein X is a protein whose intracellular expression is detectable as a signal; the introduction of the nucleic acid construct into the cancer cells is carried out by administering the nucleic acid construct to the cancer patient; and whether or not the signal of the protein is detected from a cancer lesion is investigated.
[77] The method according to [73] or [74], wherein: protein X is a secretory protein whose intracellular expression is detectable; the introduction of the nucleic acid construct into the cancer cells is carried out by administering the nucleic acid construct to the cancer patient; and the activity of the protein in blood separated from the patient after the administration of the nucleic acid construct is measured.
[78] A method of predicting an effect of an anticancer drug for mismatch repair-deficient cancer, the method comprising:
   introducing the nucleic acid construct according to any one of [39] to [61] into cancer cells of a cancer patient; and
   measuring expression of protein X.
[79] The method according to [78], wherein the anticancer drug is an immune checkpoint inhibitor.
[80] The method according to [78] or [79], wherein the cancer cells are cells separated from the patient, and the introduction of the nucleic acid construct into the cancer cells is carried out *in vitro.*
[81] The method according to [78] or [79], wherein: protein X is a protein whose intracellular expression is detectable as a signal; the introduction of the nucleic acid construct into the cancer cells is carried out by administering the nucleic acid construct to the patient; and whether or not the signal of the protein is detected from a cancer lesion is investigated.
[82] The method according to [78] or [79], wherein: protein X is a secretory protein whose intracellular expression is detectable; the introduction of the nucleic acid construct into the cancer cells is carried out by administering the nucleic acid construct to the cancer patient; and the activity of the protein in blood separated from the patient after the administration of the nucleic acid construct is measured.

### EFFECT OF THE INVENTION

By the present invention, a novel nucleic acid construct with which mismatch repair deficiency can be detected utilizing two or more times of SSA reaction is provided. By the nucleic acid construct of the present invention, the presence or absence of mismatch repair activity can be detected in a transient expression system in a very short time. By utilizing a gene sequence encoding a protein that acts to decrease the cell survival rate, such as a suicide gene, mismatch repair-deficient cancer cells can be efficiently killed. Further, by utilizing a gene sequence encoding a protein whose intracellular expression is detectable, such as a luciferase gene or a fluorescent protein gene, a mismatch repair-deficient cancer can be detected. The present invention significantly contributes to diagnosis and treatment of various mismatch repair-deficient cancers including Lynch syndrome irrespective of the types of the cancers. Further, the nucleic acid construct of the present invention can also be applied to search for factors that positively or negatively regulate, and inhibitors or activators of, the reaction of mismatch repair or single-strand annealing, and to prediction of whether or not a gene mutation identified in a cancer patient is a mutation that deteriorates the mismatch repair activity.

The SSA construct previously developed by the present inventor (Patent Document 1) had a structure that results in expression of a protein due to formation of a normal gene sequence by a single SSA reaction. In contrast, the construct of the present invention has a structure that requires two or more SSA reactions for allowing the expression of the protein. By the construct of the present invention, a difference in the expression level (activity) of a protein due to the presence or absence of mismatch repair deficiency can be more clearly detected compared to conventional SSA constructs (see the Examples below). Therefore, the construct of the present invention can be expected to act more specifically on mismatch repair-deficient cells, and hence to provide, for example, a therapeutic means with lower side effects.

### BRIEF DESCRIPTION OF THE DRAWINGS

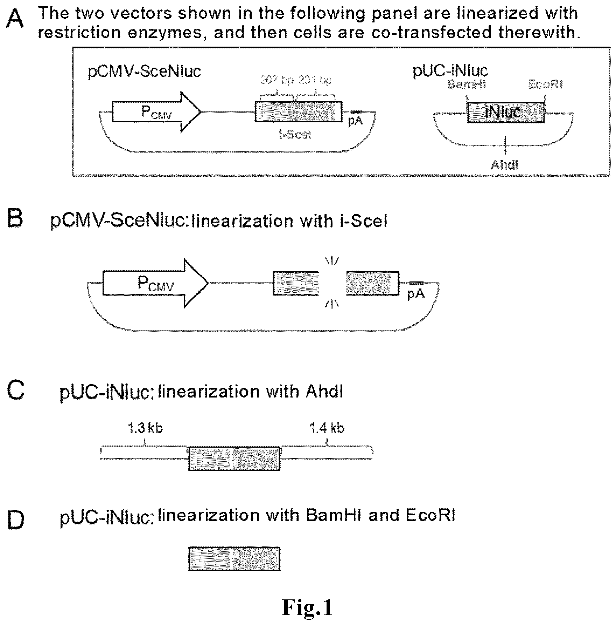

[Fig. 1] A diagram illustrating the structure of a two-molecule, two-SSA-reaction type Nluc construct (construct of the first aspect) prepared in Example A-I. Two nucleic acid molecules constituting the construct (A); an SceNluc fragment comprising a promoter region (B); an iNluc fragment that is a complementary region, with (C) or without (D) additional sequences at both ends.

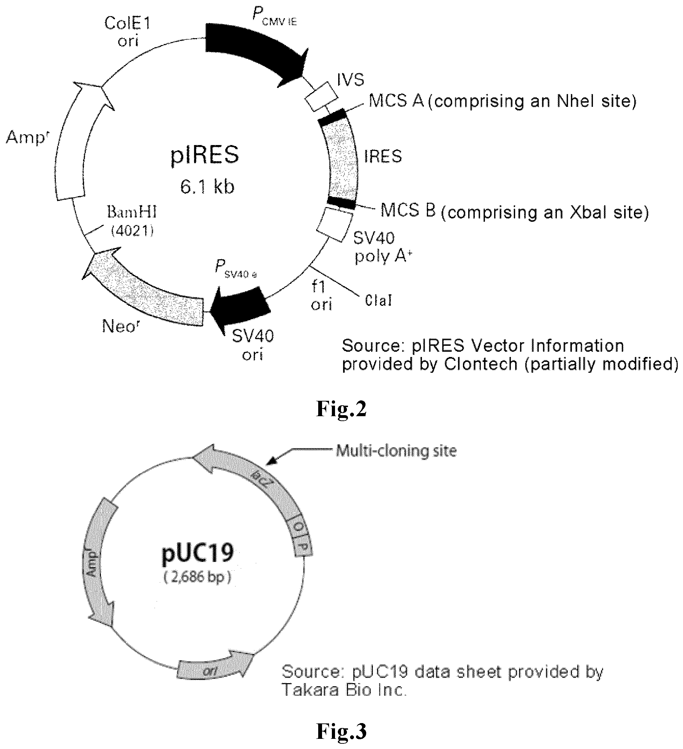

[Fig. 2] A structural drawing of the plasmid vector pIRES used in Examples (partially modified from the structural drawing in Clontech's pIRES Vector Information).
[Fig. 3] A structural drawing of the plasmid vector pUC19 used in Examples (source: pUC19 DNA data sheet provided by Takara Bio Inc.).

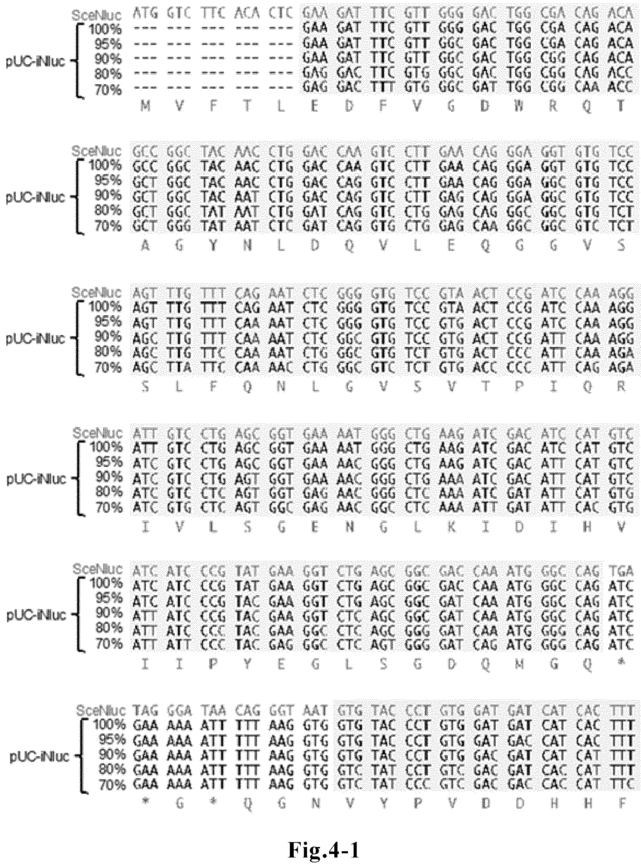

[Fig. 4-1] Sequences of the iNluc fragments prepared in Example A-I, which have decreased homologies of 95%, 90%, 80%, and 70% between the homologous region and the substrate. As shown in the figure, silent mutations were introduced such that they were dispersed throughout the homologous sequences.

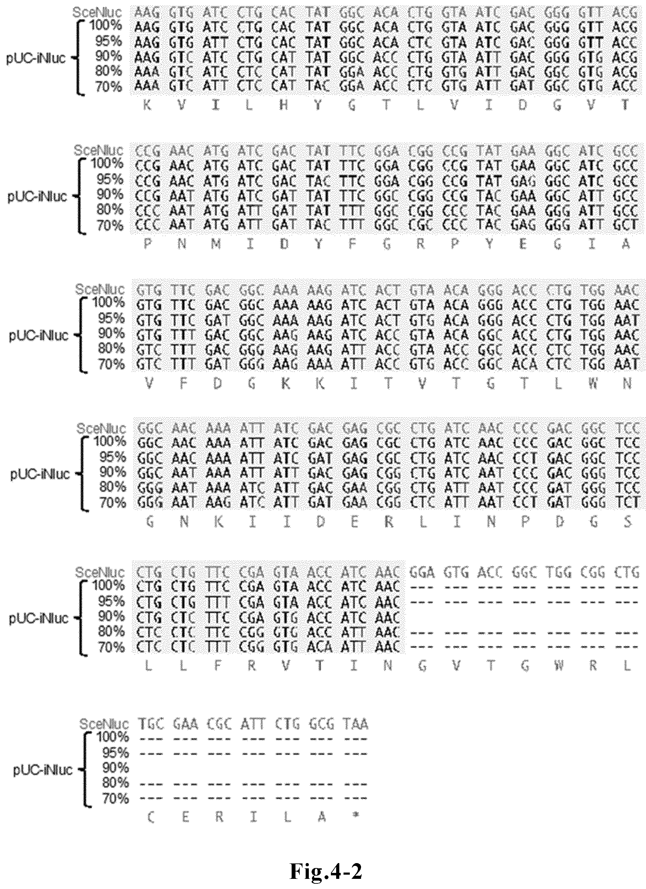

[Fig. 4-2] (continued from Fig. 4-1) Sequences of the iNluc fragments prepared in Example A-I, which have decreased homologies of 95%, 90%, 80%, and 70% between the homologous region and the substrate. As shown in the figure, silent mutations were introduced such that they were dispersed throughout the homologous sequences.

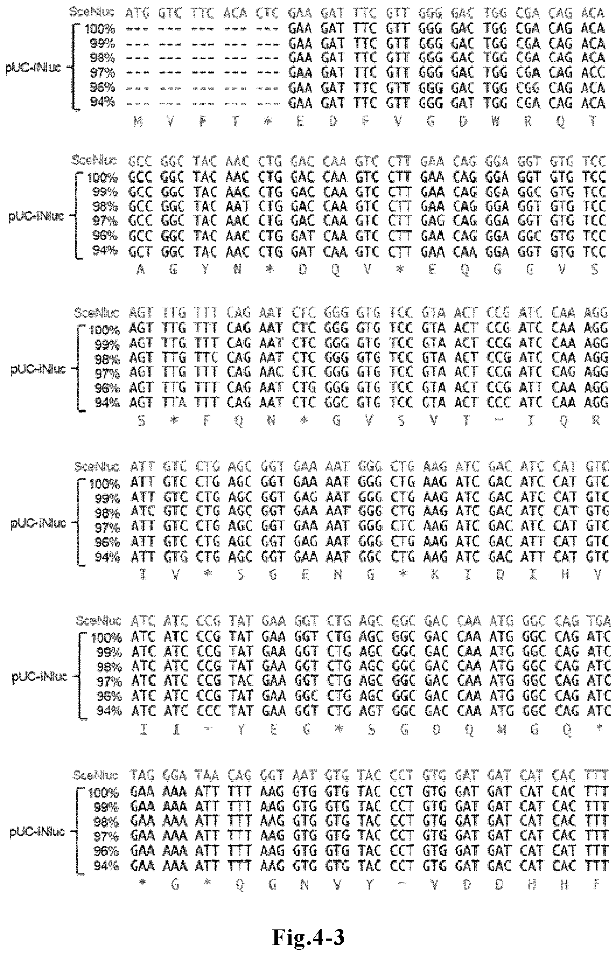

[Fig. 4-3] Sequences of the iNluc fragments prepared in Example A-I, which have decreased homologies of 99 to 96% and 94% between the homologous region and the substrate. As shown in the figure, silent mutations were introduced such that they were dispersed throughout the homologous sequences.

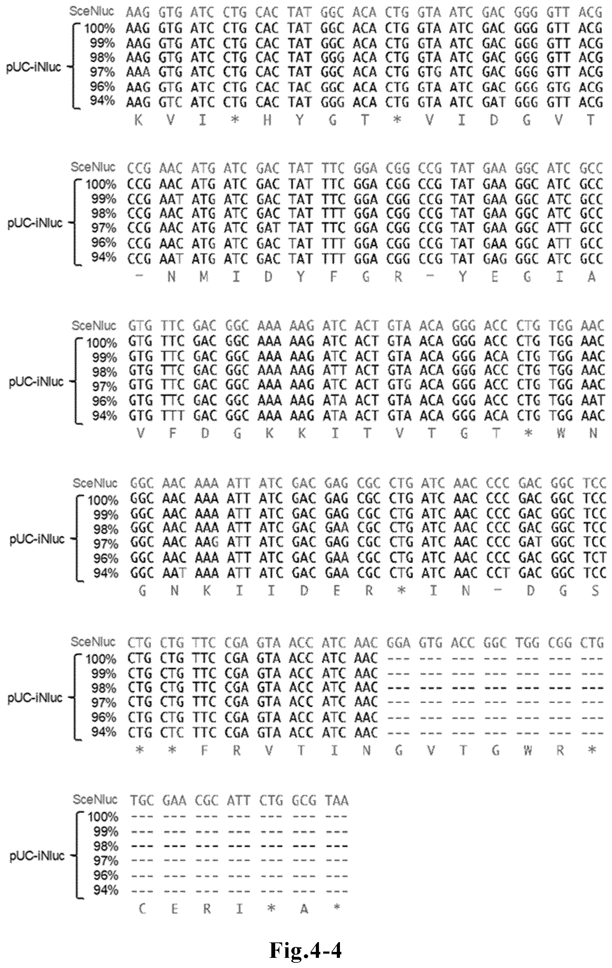

[Fig. 4-4] (continued from Fig. 4-3) Sequences of the iNluc fragments prepared in Example A-I, which have decreased homologies of 99 to 96% and 94% between the homologous region and the substrate. As shown in the figure, silent mutations were introduced such that they were dispersed throughout the homologous sequences.

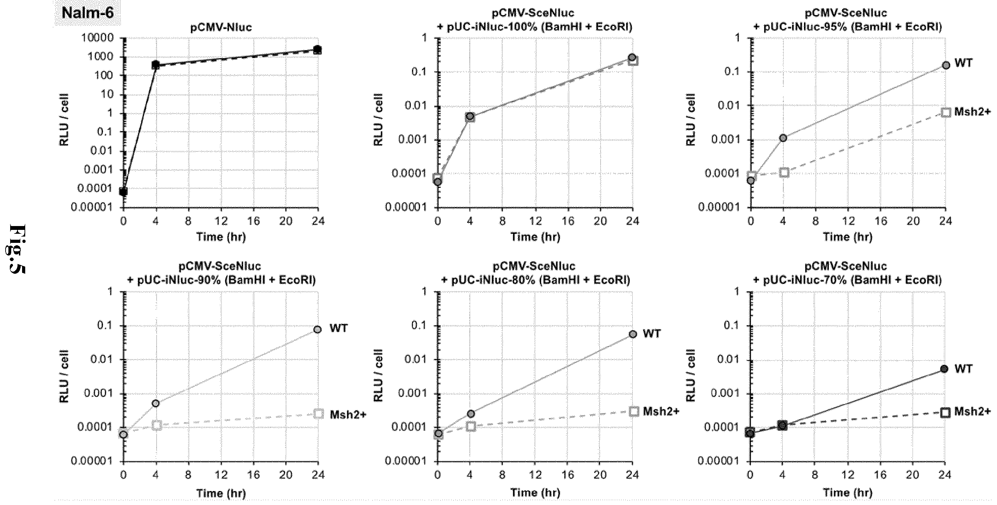

[Fig. 5] One example of the results of luciferase assays of Nalm-6 cells and MSH2 revertant cells thereof into which two-molecule, two-SSA-reaction type Nluc constructs have been introduced. Each iNluc fragment (with a homology of 100%, 95%, 90%, 80%, or 70%) without additional sequences was co-transfected with pCMV-SceNluc, and the luciferase activity was measured over time.

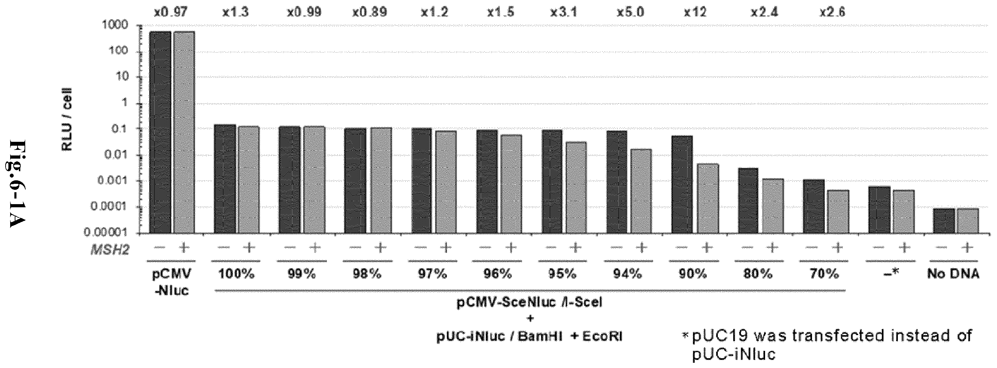

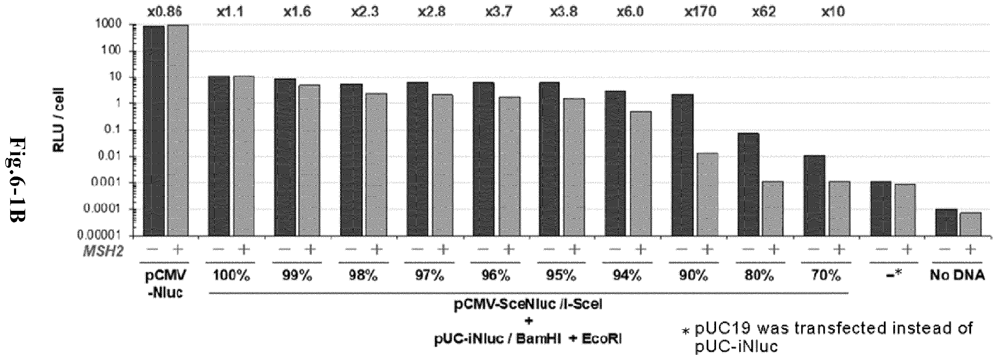

[Fig. 6-1A] One example of the results of luciferase assays of Nalm-6 cells and MSH2 revertant cells thereof into which two-molecule, two-SSA-reaction type Nluc constructs have been introduced. The luciferase activity 4 hours after the gene transfection of each construct (with a homology of 100 to 94%, 90%, 80%, or 70%) is shown.
[Fig. 6-1B] One example of the results of luciferase assays of Nalm-6 cells and MSH2 revertant cells thereof into which two-molecule, two-SSA-reaction type Nluc constructs have been introduced. The luciferase activity 24 hours after the gene transfection of each construct (with a homology of 100 to 94%, 90%, 80%, or 70%) is shown.

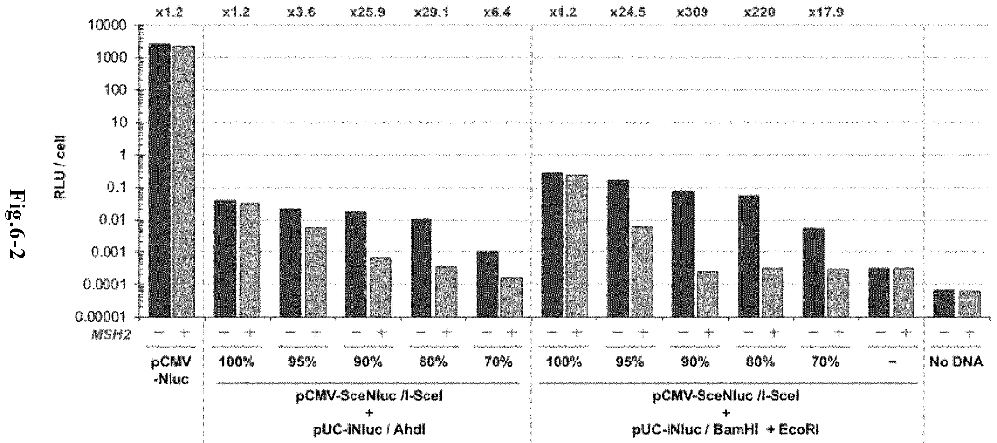

[Fig. 6-2] One example of the results of luciferase assays of Nalm-6 cells and MSH2 revertant cells thereof into which two-molecule, two-SSA-reaction type Nluc constructs have been introduced. The luciferase activity 24 hours after the gene transfection was compared between iNluc fragments with additional sequences (pUC-iNluc / AhdI, left side in the graph) and without the additional sequences (pUC-iNluc / BamHI + EcoRI, right side in the graph).

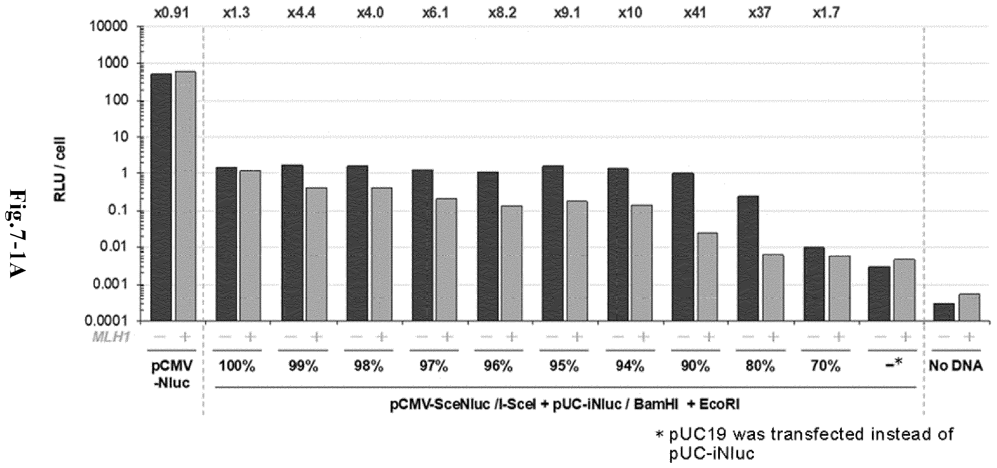

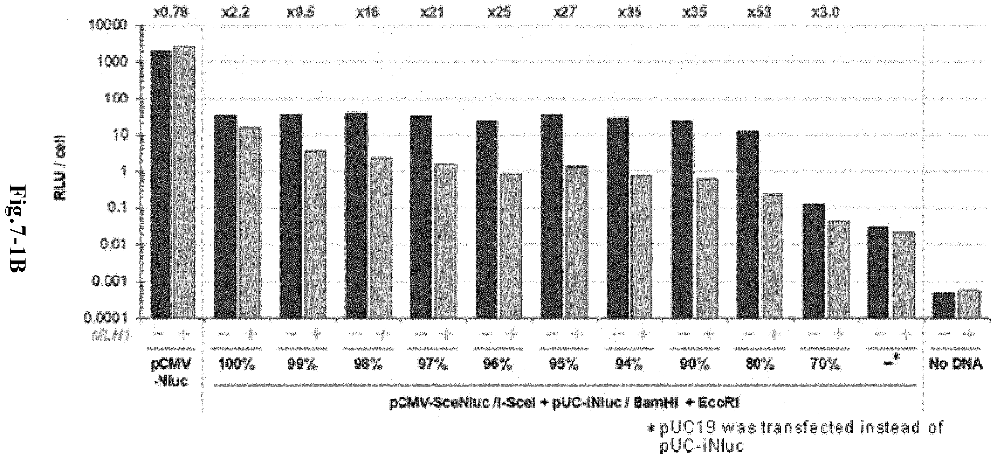

[Fig. 7-1A] One example of the results of luciferase assays of HCT116 cells and MLH1 revertant cells thereof into which two-molecule, two-SSA-reaction type Nluc constructs have been introduced. The luciferase activity 4 hours after the gene transfection of each construct (with a homology of 100 to 94%, 90%, 80%, or 70%) is shown.
[Fig. 7-1B] One example of the results of luciferase assays of HCT116 cells and MLH1 revertant cells thereof into which two-molecule, two-SSA-reaction type Nluc constructs have been introduced. The luciferase activity 24 hours after the gene transfection of each construct (with a homology of 100 to 94%, 90%, 80%, or 70%) is shown.
[Fig. 7-2] One example of the results of luciferase assays of HCT116 cells and MLH1 revertant cells thereof into which two-molecule, two-SSA-reaction type Nluc constructs have been introduced. The luciferase activity 24 hours after the gene transfection was compared between iNluc fragments with additional sequences (pUC-iNluc / AhdI, left side in the graph) and without the additional sequences (pUC-iNluc / BamHI + EcoRI, right side in the graph).
[Fig. 8] The structure of the single-molecule, two-SSA-reaction type, integrated-type Nluc construct (construct of the first aspect) prepared in Example A-II.
[Fig. 9] The luciferase activities of Nalm-6 cells and MSH2 revertant cells thereof 24 hours after introduction of integrated-type Nluc constructs.
[Fig. 10] The structure of the two-molecule, two-SSA-reaction type NlucP construct (construct of the first aspect) prepared in Example A-III.
[Fig. 11] The luciferase activities of Nalm-6 cells and MSH2 revertant cells thereof 24 hours after introduction of two-molecule, two-SSA-reaction type NlucP constructs (with a homology of 100%, 98%, 96%, 94%, or 90%).
[Fig. 12] A diagram illustrating the structure and the mechanism of the three-molecule, three-SSA-reaction type Nluc construct (construct of the first aspect) prepared in Example A-IV.
[Fig. 13] A diagram illustrating the structure and the mechanism of the four-molecule, three-SSA-reaction type Nluc construct (construct of the first aspect) prepared in Example A-IV.
[Fig. 14-1] Sequences of the iNluc3-1 fragments prepared in Example A-IV, which have decreased homologies of 90% and 80% between the homologous region and the substrate (sequences of the A portion in Fig. 13). As shown in the figure, silent mutations were introduced such that they were dispersed throughout the homologous sequences.
[Fig. 14-2] Sequences of the iNluc3-1 fragments and the iNluc3-2 fragments prepared in Example A-IV, which have decreased homologies of 90% and 80% between the homologous region and the substrate (sequences of the B portion in Fig. 13). As shown in the figure, silent mutations were introduced such that they were dispersed throughout the homologous sequences.
[Fig. 14-3] Sequences of the iNluc3-2 fragments prepared in Example A-IV, which have decreased homologies of 90% and 80% between the homologous region and the substrate (sequences of the C portion in Fig. 13). As shown in the figure, silent mutations were introduced such that they were dispersed throughout the homologous sequences.
[Fig. 15] One example of the results of luciferase assays of Nalm-6 cells and MSH2 revertant cells thereof into which three-molecule, three-SSA-reaction type Nluc constructs have been introduced (results obtained 4 hours after the gene transfection).
[Fig. 16] One example of the results of luciferase assays of Nalm-6 cells and MSH2 revertant cells thereof into which three-molecule, three-SSA-reaction type Nluc constructs have been introduced (results obtained 24 hours after the gene transfection).
[Fig. 17] The results of measurement of the luciferase activity 4 hours or 24 hours after introduction of three-molecule, three-SSA-reaction type Nluc constructs into HCT116 cells and MLH1 revertant cells thereof.
[Fig. 18] The results of measurement of the luciferase activity 24 hours after introduction of four-molecule, three-SSA-reaction type Nluc constructs into Nalm-6 cells and MSH2 revertant cells thereof.
[Fig. 19] The results of measurement of the luciferase activity 24 hours after introduction of four-molecule, three-SSA-reaction type Nluc constructs into HCT116 cells and MLH1 revertant cells thereof.
[Fig. 20] A diagram illustrating the structure of the two-molecule, two-SSA-reaction type GeNL construct (construct of the first aspect) prepared in Example A-V.
[Fig. 21-1] The results of measurement of the luciferase activity 4 hours after introduction of two-molecule, two-SSA-reaction type GeNL constructs into Nalm-6 cells and MSH2 revertant cells thereof.
[Fig. 21-2] The results of measurement of the luciferase activity 24 hours after introduction of two-molecule, two-SSA-reaction type GeNL constructs into Nalm-6 cells and MSH2 revertant cells thereof.
[Fig. 22] The structure of the two-molecule, two-SSA-reaction type Nluc-homeo DTA-linked construct (construct of the second aspect) prepared in Example B, wherein SSA causes polycistronic expression of two kinds of proteins.
[Fig. 23-1] The results of comparison of the survival rate 96 hours after introduction of two-molecule, two-SSA-reaction type Nluc-homeo DTA-linked constructs, between Nalm-6 cells and MSH2 revertant cells thereof.
[Fig. 23-2] The results of comparison of the survival rate 72 hours after introduction of two-molecule, two-SSA-reaction type Nluc-homeo DTA-linked constructs, between HCT116 cells and MLH1 revertant cells thereof.
[Fig. 24] The structure of the two-molecule, two-SSA-reaction type Nluc-homeo TK-linked construct (construct of the second aspect) prepared in Example C, wherein SSA causes polycistronic expression of two kinds of proteins.
[Fig. 25] The results of comparison of the survival rate 96 hours after introduction of two-molecule, two-SSA-reaction type Nluc-homeo TK-linked constructs, between Nalm-6 cells and MSH2 revertant cells thereof.
[Fig. 26] The structure of the two-molecule, two-SSA-reaction type DTA construct (construct of the first aspect) prepared in Example D-I.
[Fig. 27-1] Sequences of the iDTA2 fragments prepared in Example D-I, which have decreased homologies of 99 to 94%, 90%, 80%, and 70% between the homologous region and the substrate. As shown in the figure, silent mutations were introduced such that they were dispersed throughout the homologous sequences.
[Fig. 27-2] (continued from Fig. 27-1) Sequences of the iDTA2 fragments prepared in Example D-I, which have decreased homologies of 99 to 94%, 90%, 80%, and 70% between the homologous region and the substrate. As shown in the figure, silent mutations were introduced such that they were dispersed throughout the homologous sequences.
[Fig. 28] The results of comparison of the survival rate 96 hours after introduction of two-molecule, two-SSA-reaction type DTA constructs, between Nalm-6 cells and MSH2 revertant cells thereof.
[Fig. 29] The results of comparison of the survival rate 72 hours after introduction of two-molecule, two-SSA-reaction type DTA constructs, between HCT116 cells and MLH1 revertant cells thereof.
[Fig. 30] A diagram illustrating the structure and the mechanism of the three-molecule, three-SSA-reaction type DTA construct (construct of the first aspect) prepared in Example D-II.
[Fig. 31] A diagram illustrating the structure and the mechanism of the four-molecule, three-SSA-reaction type DTA construct (construct of the first aspect) prepared in Example D-II.
[Fig. 32-1] Sequences of the iDTA3-1 fragments prepared in Example D-II, which have decreased homologies of 98%, 94%, and 90% between the homologous region and the substrate (sequences of the A portion in Fig. 30 and Fig. 31). As shown in the figure, silent mutations were introduced such that they were dispersed throughout the homologous sequences.
[Fig. 32-2] Sequences of the iDTA3-1 fragments and the iDTA3-2 fragments prepared in Example D-II, which have decreased homologies of 98%, 94%, and 90% between the homologous region and the substrate (sequences of the B portion in Fig. 30 and Fig. 31). As shown in the figure, silent mutations were introduced such that they were dispersed throughout the homologous sequences.
[Fig. 32-3] Sequences of the iDTA3-2 fragments prepared in Example D-II, which have decreased homologies of 98%, 94%, and 90% between the homologous region and the substrate (sequences of the C portion in Fig. 30 and Fig. 31). As shown in the figure, silent mutations were introduced such that they were dispersed throughout the homologous sequences.
[Fig. 33] The results of comparison of the survival rate 96 hours after introduction of three-molecule, three-SSA-reaction type DTA constructs, between Nalm-6 cells and MSH2 revertant cells thereof (left panel), and the results of comparison of the survival rate 72 hours after introduction of these constructs, between HCT116 cells and MLH1 revertant cells thereof (right panel).
[Fig. 34] The results of comparison of the survival rate after introduction of four-molecule, three-SSA-reaction type DTA constructs into Nalm-6 cells and MSH2 revertant cells thereof (96 hours after the introduction of the constructs).
[Fig. 35] A diagram illustrating the structure and the mechanism of the four-molecule, four-SSA-reaction type TK construct (construct of the first aspect) prepared in Example E-I.
[Fig. 36-1] Sequences of the iTK3-1 fragments, the iTK3-2 fragments, and the iTK3-3 fragments prepared in Example E-I, which have decreased homologies of 98%, 94%, and 90% between the homologous region and the substrate (sequences of the A and D portions in Fig. 35). As shown in the figure, silent mutations were introduced such that they were dispersed throughout the homologous sequences.
[Fig. 36-2] Sequences of the iTK3-1 fragments and the iTK3-2 fragments prepared in Example E-I, which have decreased homologies of 98%, 94%, and 90% between the homologous region and the substrate (sequences of the B portion in Fig. 35). As shown in the figure, silent mutations were introduced such that they were dispersed throughout the homologous sequences.
[Fig. 36-3] Sequences of the iTK3-2 fragments and the iTK3-3 fragments prepared in Example E-I, which have decreased homologies of 98%, 94%, and 90% between the homologous region and the substrate (sequences of the C portion in Fig. 35). As shown in the figure, silent mutations were introduced such that they were dispersed throughout the homologous sequences.
[Fig. 37] The results of comparison of the survival rate 96 hours after introduction of four-molecule, four-SSA-reaction type TK constructs, between Nalm-6 cells and MSH2 revertant cells thereof.
[Fig. 38] The results of comparison of the survival rate 96 hours after introduction of four-molecule, four-SSA-reaction type TK constructs, between HCT116 cells and MLH1 revertant cells thereof.
[Fig. 39] A diagram illustrating mutation introduction sites in TK30.
[Fig. 40] A diagram illustrating the structure and the mechanism of the four-molecule, four-SSA-reaction type TK30 construct (construct of the first aspect) prepared in Example E-II.
[Fig. 41-1] Sequences of the iTK3-1 fragments, the iTK303-2 fragments, and the iTK3-3 fragments prepared in Example E-II, which have decreased homologies of 98%, 94%, and 90% between the homologous region and the substrate (sequences of the A and D portions in Fig. 40). As shown in the figure, silent mutations were introduced such that they were dispersed throughout the homologous sequences.
[Fig. 41-2] Sequences of the iTK3-1 fragments and the iTK303-2 fragments prepared in Example E-II, which have decreased homologies of 98%, 94%, and 90% between the homologous region and the substrate (sequences of the B portion in Fig. 40). As shown in the figure, silent mutations were introduced such that they were dispersed throughout the homologous sequences.
[Fig. 41-3] Sequences of the iTK303-2 fragments and the iTK3-3 fragments prepared in Example E-II, which have decreased homologies of 98%, 94%, and 90% between the homologous region and the substrate (sequences of the C portion in Fig. 40). As shown in the figure, silent mutations were introduced such that they were dispersed throughout the homologous sequences.
[Fig. 42] The results of comparison of the survival rate 96 hours after introduction of four-molecule, four-SSA-reaction type TK30 constructs, between Nalm-6 cells and MSH2 revertant cells thereof.
[Fig. 43] The results of comparison of the survival rate 72 hours after introduction of four-molecule, four-SSA-reaction type TK30 constructs, between HCT116 cells and MLH1 revertant cells thereof.
[Fig. 44] A diagram illustrating the structure and the mechanism of the four-molecule, four-SSA-reaction type CDUPRT construct (construct of the first aspect) prepared in Example E-III.
[Fig. 45-1] Sequences of the iCDUPRTA3-1 fragments, the iCDUPRTA3-2 fragments, and the iCDUPRTA3-3 fragments prepared in Example E-III, which have decreased homologies of 98%, 94%, and 90% between the homologous region and the substrate (sequences of the A and D portions in Fig. 44). As shown in the figure, silent mutations were introduced such that they were dispersed throughout the homologous sequences.
[Fig. 45-2] Sequences of the iCDUPRTA3-1 fragments and the iCDUPRTA3-2 fragments prepared in Example E-III, which have decreased homologies of 98%, 94%, and 90% between the homologous region and the substrate (sequences of the B portion in Fig. 44). As shown in the figure, silent mutations were introduced such that they were dispersed throughout the homologous sequences.
[Fig. 45-3] Sequences of the iCDUPRTA3-2 fragments and the iCDUPRTA3-3 fragments prepared in Example E-III, which have decreased homologies of 98%, 94%, and 90% between the homologous region and the substrate (sequences of the C portion in Fig. 44). As shown in the figure, silent mutations were introduced such that they were dispersed throughout the homologous sequences.
[Fig. 46] The results of comparison of the survival rate 96 hours after introduction of four-molecule, four-SSA-reaction type CDUPRT constructs, between Nalm-6 cells and MSH2 revertant cells thereof.
[Fig. 47] The results of comparison of the survival rate 72 hours after introduction of four-molecule, four-SSA-reaction type CDUPRT constructs, between HCT116 cells and MLH1 revertant cells thereof.

### MODE FOR CARRYING OUT THE INVENTION

In the present invention, recombination reaction by single-strand annealing refers to a reaction that is different from the Rad51-dependent standard homologous recombination, and that occurs between homologous base sequences through a pathway independent of Rad51. This recombination reaction does not require 100% homology between the homologous base sequences as long as those sequences have not less than a certain level of homology. In particular, in the nucleic acid constructs of the present invention, the homology between the homologous base sequences is not less than 40% and less than 100%.

In the present invention, the chain length of a nucleic acid is expressed using the unit "bases". In cases where the nucleic acid is a double-stranded nucleic acid, the term "bases" means "base pairs".

In the present invention, "homology" of a base sequence has the same meaning as "sequence identity" of a base sequence, and is determined by aligning two base sequences to be compared such that their bases match as much as possible, and dividing the number of matched bases by the total number of bases, wherein the resulting value is expressed as a percentage. In the alignment, a gap(s) is/are inserted to one or both of the two sequences to be compared, when necessary. Such alignment of sequences can be carried out, for example, by using a well-known program such as BLAST, FASTA, or CLUSTAL W. In cases where a gap(s) is/are inserted, the total number of bases is the number of bases determined such that one gap is counted as one base. In cases where the thus counted total number of bases is different between the two sequences to be compared, the homology (%) is calculated by dividing the number of matched bases by the total number of bases of the longer sequence. "Sequence identity" of an amino acid sequence is calculated in the same manner.

Although the nucleic acid construct of the present invention may be either DNA or RNA, the nucleic acid construct is preferably prepared as DNA from the viewpoint of stability of the nucleic acid molecule. The nucleic acid construct of the present invention may comprise a nucleotide analog in part thereof. Examples of the nucleotide analog include, but are not limited to, bridged nucleic acids such as LNA, ENA, and PNA; and modified bases such as Super T (registered trademark) and Super G (registered trademark).

The nucleic acid construct of the present invention encompasses two major aspects.

A nucleic acid construct of the first aspect is a construct in which recombination reaction by single-strand annealing (SSA) (which may hereinafter be simply referred to as "SSA reaction") occurs to form a gene sequence encoding a protein, to thereby enable expression of the protein.

A nucleic acid construct of the second aspect is a construct of a type originally comprising a gene sequence encoding a protein, in which occurrence of SSA reaction causes the gene sequence to be operably linked to a promoter, to thereby enable expression of the protein from the gene sequence.

First, the first aspect is described below.

### [Structure of Nucleic Acid Construct of First Aspect]

The nucleic acid construct of the first aspect comprises: a promoter region; a 5'-side region of a gene sequence encoding protein A (hereinafter simply referred to as "5'-side region"); a 3'-side region of a gene sequence encoding protein A (hereinafter simply referred to as "3'-side region"); and at least one complementary region; which are placed in one nucleic acid molecule or in two or more different nucleic acid molecules. The promoter region and the 5'-side region are operably linked to each other, and contained in the same nucleic acid molecule. The other elements may be in the same nucleic acid molecule as these regions, or may be in (an)other nucleic acid molecule(s). Focusing on the placement of the three elements (the promoter region, the 5'-side region, and the 3'-side region) other than the complementary region, nucleic acid constructs of the first aspect can be divided into the following two types of constructs: constructs in which the three elements are placed in the same nucleic acid molecule, and constructs in which the promoter region and the 5'-side region are placed in one nucleic acid molecule, and the 3'-side region is placed in another nucleic acid molecule. The complementary region may be placed in a nucleic acid molecule that is different from the nucleic acid molecule(s) of the other elements, or may be placed in a nucleic acid molecule together with at least one of the other elements.

The 5'-side region and the 3'-side region may be composed of regions formed by dividing the entire gene sequence encoding protein A into two parts, or may be composed of partial regions of both ends of the gene sequence in a manner in which the middle portion of the gene sequence is not included. In the latter case, one or more complementary regions comprise a coding sequence that complements the middle portion, and a gene sequence whose middle portion has been complemented is generated by the SSA reaction.

The size of the 5'-side region needs to be set such that a protein fragment capable of exerting the activity of protein A is not expressed from the 5'-side region in a cell lacking the SSA activity. The size of the 5'-side region is not limited, and may be appropriately set depending on the type of protein A. In general, the size of the 5'-side region may be not more than about 60/100, for example, not more than 50/100, not more than 40/100, not more than 30/100, or not more than 25/100 of the full length of protein A. In cases where protein A is a fusion protein of protein X and protein Y, wherein protein X is a protein that acts to decrease the cell survival rate or emit a signal, wherein protein Y is a protein that functions to enhance the action of protein X, and wherein protein Y is fused to the C-terminal side of the protein X, the size of the 5'-side region may be not more than about 60/100, for example, not more than 50/100, not more than 40/100, not more than 30/100, or not more than 25/100 of the protein X portion. Or, the protein A fragment consisting of the 5'-side region can be prevented from exerting the activity by placing the cleavage site upstream of a base sequence encoding amino acids of the active center of protein A (when protein A is a fusion protein prepared by fusing protein Y to the C-terminal side of protein X, the active center of protein X) so that a region on the N-terminal side of the active center is employed as the 5'-side region. Although there is no lower limit of the size of the 5'-side region, the size is usually set to not less than 50 bases.

A poly-A addition signal may be functionally linked downstream of the 3'-side region. The functional linkage herein means that a poly-A addition signal is placed downstream of the 3'-side region such that an mRNA molecule having a poly-A tail is expressed from the base sequence formed after the recombination reaction by the single-strand annealing, and no other element such as a complementary region is placed between the 3'-side region and the poly-A addition signal. However, since mRNAs that appropriately function without a poly-A, such as a histone mRNA, are known, the poly-A addition signal may be omitted also in the nucleic acid construct of the present invention.

At least one complementary region comprises: one homologous region (homologous region α) for which a region comprising at least a 3'-end portion of the 5'-side region serves as a substrate in the SSA reaction; and one homologous region (homologous region β) for which a region comprising at least a 5'-end portion of the 3'-side region serves as a substrate in the SSA reaction.

The homologous region α and the homologous region β are contained in the same complementary region or different complementary regions. In the case of a two-SSA-reaction type construct, in which the protein is expressed by two times of SSA reaction, one complementary region is present, and the one complementary region comprises the homologous region α and the homologous region β. In the case of a three- or more-SSA-reaction type construct, in which the protein is expressed by three or more times of SSA reaction, two or more complementary regions are included, and the homologous region α and the homologous region β are included in different complementary regions. Two or more complementary regions of a three- or more-SSA-reaction type construct thus contain, in addition to the homologous region α and the homologous region β, one or more pairs of a further homologous region and the substrate therefor to allow the occurrence of SSA reaction between the complementary regions.

The homology between each homologous region and the substrate therefor is not limited as long as it is sufficient for allowing single-strand annealing therebetween and within the range of not less than 40% and less than 100%. In cases where the homology between the homologous region and the substrate is 100%, recombination by homologous recombination (HR) reaction is predominant over the SSA reaction, so that the difference in the protein expression depending on the presence or absence of a mismatch repair factor such as MSH2 can be hardly observed (see, for example, A-1-3 in the Examples below). Therefore, in the construct of the present invention, the homology between each homologous region and the substrate therefor is set to less than 100%. Regarding the upper limit of the homology, the homology may be, for example, not more than 99%, not more than 98%, not more than 97%, not more than 96%, or not more than 95%. Regarding the lower limit, the homology may be, for example, not less than 45%, not less than 50%, not less than 55%, not less than 60%, not less than 65%, not less than 70%, not less than 75%, not less than 80%, not less than 85%, or not less than 90%.

The bases mismatched between a homologous region and the substrate therefor are preferably dispersed throughout the homologous region or its substrate therefor, to make the SSA reaction more likely to occur. In general, the number of consecutive mismatched bases may be not more than 10. In a homologous region or the substrate therefor, the number of consecutive mismatched bases may be, for example, not more than 9, not more than 8, not more than 7, not more than 6, not more than 5, not more than 4, not more than 3, or not more than 2, or the mismatched bases may be singly dispersed.

The homologous region or substrate having a mismatched base(s) is preferably simply designed by using a gene sequence encoding protein A and introducing a silent mutation(s) thereto, and it is also possible to introduce a mutation(s) that change(s) the encoded amino acid sequence. In the latter case, while the gene sequence generated by the SSA reaction may encode protein B whose amino acid sequence is different from that of protein A, this is acceptable if protein B has the same activity as protein A. Such protein B has a sequence identity of not less than 80%, for example, not less than 85%, not less than 90%, not less than 95%, or not less than 98% to protein A.

Conservative substitution, that is, substitution with an amino acid having similar chemical properties, is highly unlikely to cause deterioration of properties and activity of a protein. Amino acids having similar side chains have similar chemical properties. Based on the similarities among the side chains, amino acids can be classified into, for example, a group of amino acids having an aliphatic side chain (glycine, alanine, valine, leucine, and isoleucine), a group of amino acids having an aliphatic hydroxyl side chain (serine and threonine), a group of amino acids having an amide-containing side chain (asparagine and glutamine), a group of amino acids having an aromatic side chain (phenylalanine, tyrosine, and tryptophan), a group of amino acids having a basic side chain (arginine, lysine, and histidine), a group of amino acids having an acidic side chain (aspartic acid and glutamic acid), and a group of amino acids having a sulfur-containing side chain (cysteine and methionine). Substitution with another amino acid belonging to the same group is conservative substitution. In cases where a mismatched base is not a silent mutation, a mutation that results in a conservative amino acid substitution may be preferably selected.

The chain lengths of each homologous region and the substrate therefor are not limited as long as they are sufficient for causing the recombination reaction by SSA in a cell. The chain lengths are, for example, not less than 4 bases, not less than 10 bases, or not less than 15 bases, or may be not less than 20 bases, not less than 30 bases, not less than 40 bases, or not less than 50 bases. A construct that causes SSA in a homologous region of 20 bases is known (WO 2021/162121 A1). The upper limit is also not limited. The chain length is usually not more than 10,000 bases, and may be, for example, not more than 5000 bases, not more than 1000 bases, not more than 700 bases, not more than 500 bases, or not more than 200 bases.

The promoter is not limited, and may be any promoter as long as a promoter activity can be exerted in a cell (typically a mammalian cell such as a human cell). In general, a promoter that constitutively exerts a strong promoter activity in the cell into which the nucleic acid construct of the present invention is introduced may be preferably used. Or, an inducible promoter that exerts the promoter activity under certain conditions may be used. In the following Examples, a human cytomegalovirus promoter that constitutively exerts a strong promoter activity in a cell was used. However, the promoter is not limited thereto. Further, the promoter is not limited to a virus-derived promoter, and the origin of the promoter is not limited as long as it is a sequence having a promoter activity in a cell, preferably in a human cell.

The gene sequence encoding protein A or protein B (which may be hereinafter simply referred to as protein A/B) generated by the SSA reaction may be a sequence containing the entire coding region of a naturally occurring gene, or may be a sequence composed of part thereof or a modified sequence thereof. For example, only a region encoding a domain required for the exertion of the activity of the protein (for example, a sequence encoding a protein fragment obtained by removal of domains that are not necessary for the protein activity itself, such as a domain required for membrane localization) in a naturally occurring gene sequence may be used as the gene sequence in the present invention. Further, for fluorescent proteins and luminescent proteins, genes with various modifications have been widely commercially utilized. Such gene sequences encoding non-naturally occurring proteins may also be used. Further, a gene sequence encoding a fusion protein in which two or more proteins are fused may be used as the gene sequence encoding protein A/B. That is, protein A/B may be a fusion protein. In cases where a gene sequence encoding a fusion protein is used in the present invention, typical examples of the gene sequence include a gene sequence encoding a fusion protein in which a protein whose activity can be measured directly, or indirectly using as an index, for example, a phenotypic change that occurs in the cell due to the protein activity, is fused with a protein that acts to enhance the activity of the beforementioned protein.

Protein A/B is preferably a protein whose expression level can be rapidly and simply measured. For example, the protein used as protein A/B is preferably a protein whose activity can be measured directly, or indirectly using as an index, for example, a phenotypic change that occurs in the cells due to the activity of the protein, rather than a protein for which the amount of the protein itself produced or accumulated is measured. Examples of such a protein include proteins whose intracellular expression is detectable based on a signal such as luminescence due to reaction with a substrate, or fluorescence of the protein itself. Another example is a protein that acts to decrease the cell survival rate. In this case, the activity of the protein can be indirectly measured as a decrease in the cell survival rate.

The term "measurement of expression (the expression level) of protein A/B" includes measurement of the amount of protein A/B produced or accumulated, and measurement of the activity of protein A/B. As described above, in the present invention, it is preferred to employ, as protein A/B, a protein whose expression can be measured by directly or indirectly measuring the activity of the protein. In other words, it is preferred to employ, as protein A/B, a protein whose activity can be directly or indirectly measured.

Specific examples of the gene encoding a protein that acts to decrease the cell survival rate include, but are not limited to, suicide genes, DNA damage-inducing genes, and DNA repair-inhibiting genes.

Examples of the suicide genes include genes encoding a protein which by itself is toxic or injurious to cells, genes encoding a protein which acts on another compound to produce a toxic substance, and genes encoding a naturally occurring or non-naturally occurring protein that inhibits an endogenous protein to exert cytotoxicity by the dominant negative effect. Specific examples of the suicide genes include the diphtheria toxin A fragment gene (*DT-A*) (the DT-A protein itself has toxicity to cells, and kills the cells), the herpesvirus-derived thymidine kinase gene (*HSV-tk*) (HSV-tk protein acts on ganciclovir, 5-iodo-2'-fluoro-2'deoxy-1-beta-D-arabino-furanosyl-uracil (FIAU), or the like to convert it to a toxic substance having a DNA synthesis-inhibiting activity; use of this gene as a suicide gene requires treatment of the cells with ganciclovir, FIAU, or the like), the *p53* gene (its overexpression induces cell cycle arrest or apoptosis to cause cell death), and the *Fcy1* gene derived from budding yeast (encoding cytosine deaminase (CD); CD converts 5-fluorocytosine (5-FC) to 5-fluorouracil by deamination; 5-fluorouracil is metabolized in the cells to become a substance that inhibits DNA synthesis and RNA synthesis, to induce cell death (Austin EA and Huber BE. Mol Pharmacol. 1993 Mar; 43(3): 380-387.)); and modified genes thereof prepared such that the actions of these genes are enhanced. Specific examples of the modified genes include the *TK30* gene, which is a mutant of the *HSV-TK* gene (see Example E-II below), and the *CD::UPRT* gene, which is a fusion gene of the *Fcy1* gene and the *Fur1* gene derived from budding yeast (see Example E-III below). The *TK30* gene is a mutant of the *HSV-TK* gene, prepared by introducing mutations in amino acids near the active center of the *HSV-TK* gene (specifically, mutations that substitute the alanine at position 152 with valine, the leucine at position 159 with isoleucine, the isoleucine at position 160 with leucine, the phenylalanine at position 161 with alanine, the alanine at position 168 with tyrosine, and the leucine at position 169 with phenylalanine in HSV-TK protein) so as to enhance the action on ganciclovir. The *TK30* gene is known to exhibit a stronger cytotoxicity than that of the wild-type *HSV-TK* (Kokoris MS et al. Gene Ther. 1999, Aug; 6(8): 1415-1426.). The *Fur1* gene used for the *CD::UPRT* gene is a gene encoding uracil phosphoribosyl transferase (UPRT). UPRT is known to catalyze a metabolic pathway that converts 5-fluorouracil to a toxic substance in the cell. By using UPRT in combination with CD, a cytotoxicity-enhancing effect and a bystander effect due to 5-fluorocytosine can be obtained (Tiraby M et al. FEMS Microbiol Lett. 1998 Oct 1; 167(1): 41-49., Bourbeau D et al. J Gene Med. 2004 Dec; 6(12): 1320-1332.). In the following Example E-III, the entire length of a codon-optimized base sequence (SEQ ID NO:229) encoding CD (NP_015387, SEQ ID NO:230) was linked through an alanine codon to almost the entire length of a codon-optimized base sequence (SEQ ID NO:231) encoding UPRT (NP_011996, SEQ ID NO:232), to provide, as the *CD::UPRT* gene, a fusion gene encoding a fusion protein (SEQ ID NO: 186) having a structure in which the entire length of CD is linked through one alanine residue to the part from the third residue to the C-terminal residue of UPRT. However, the *CD::UPRT* gene is not limited thereto, and may be arbitrarily designed by subjecting a known *Fcy1* gene sequence (such as NM_001184159 (SEQ ID NO:233)) and a known *Fur1* gene sequence (such as NM_001179258 (SEQ ID NO:234)) to codon optimization as appropriate in a manner suitable for the animal species of the cells into which the construct is to be introduced, and linking those gene sequences, when desired, through a linker residue(s). Further specific examples of the suicide genes include naturally occurring nucleases represented by restriction enzymes and meganucleases; and artificial nucleases represented by ZFN and TALEN, and the CRISPR system (these are also included in specific examples of the DNA damage-inducing genes). However, the suicide genes are not limited to these specific examples.

Examples of the gene encoding a protein whose intracellular expression is detectable include, but are not limited to, luminescent enzyme genes such as luciferase genes (including genes encoding a secretory luciferase); fluorescent protein genes (genes encoding a naturally occurring or an artificial fluorescent protein including GFP, CFP, OFP, RFP, and YFP, and modified types thereof); cell surface antigen genes; secretory protein genes; and membrane protein genes. Luminescent enzyme genes and fluorescent protein genes are genes encoding a protein whose intracellular expression is detectable as a signal such as luminescence or fluorescence, and such genes are included in examples of genes that may be preferably used in the present invention. Besides the suicide genes, modified genes having enhanced actions of the above-described genes may be used. Specific examples of the modified genes include the genes encoding Nano-lantern (NL), in which the chemiluminescent protein Nluc is fused with a fluorescent protein. NL is known to exhibit enhanced luminescence intensity by forester resonance energy transfer (FRET) between Nluc and the fluorescent protein (Suzuki K et al. Nat Commun 2016, Dec 14; 7: 13718.). NLs using fluorescent proteins such as cyan, green, yellow-green, orange, and red fluorescent proteins have so far been reported, but other fluorescent proteins may also be used to design NLs.

Typical examples of the nucleic acid construct of the first aspect include the following.
(1) A construct with a configuration in which the promoter region, the 5'-side region, and the 3'-side region are placed in the same nucleic acid molecule, and all of the at least one complementary region are individually contained in different nucleic acid molecules.
(2) A construct with a configuration in which the promoter region and the 5'-side region are placed in one nucleic acid molecule; the 3'-side region is placed in another nucleic acid molecule; and all of the at least one complementary region are individually contained in different nucleic acid molecules.
(3) A nucleic acid construct with a configuration in which the promoter region, the 5'-side region, the 3'-side region, and all complementary regions are placed in the same nucleic acid molecule.
   The following constructs, which are variants of (1) to (3), are also included in the nucleic acid construct of the first aspect.
(4) (Variant of (1) (3)) Part of the at least one complementary region is placed in a nucleic acid molecule together with the regions including the promoter region, and the remaining complementary region(s) is/are placed in one or more other nucleic acid molecules.
(5) (Variant of (2)) The at least one complementary region is placed in one or both of the nucleic acid molecule comprising the 5'-side region and the nucleic acid molecule comprising the 3'-side region.
(6) (Variant of (2)) Part of the at least one complementary region is placed in one or both of the nucleic acid molecule comprising the 5'-side region and the nucleic acid molecule comprising the 3'-side region, and the remaining complementary region(s) is/are placed in one or more other nucleic acid molecules.

Among the nucleic acid constructs of (1), a construct composed of two nucleic acid molecules and comprising one complementary region has the following configuration.

A nucleic acid molecule 1 comprises a promoter region, a 5'-side region, and a 3'-side region.

A nucleic acid molecule 2 comprises a complementary region comprising a homologous region α and a homologous region β.

The homology between the homologous region α and a region that comprises at least a 3'-end portion of the 5'-side region and that serves as a substrate for the homologous region α in the SSA reaction is not less than 40% and less than 100%.

The homology between the homologous region β and a region that comprises at least a 5'-end portion of the 3'-side region and that serves as a substrate for the homologous region β in the SSA reaction is not less than 40% and less than 100%.

The 5'-side region, the complementary region, and the 3'-side region, when arranged in this order, encode an amino acid sequence of protein A/B while each homologous region and the substrate therefor overlap each other, and the SSA reaction that occurs between each homologous region and the substrate therefor results in expression of protein A/B.

Among the nucleic acid constructs of (1), a construct composed of three or more nucleic acid molecules and comprising two or more complementary regions can be expressed as follows using an integer *n* that is a constant of 3 ≤ *n* ≤ 10 and an integer *m* that is a variable of 3 ≤ *m* ≤ n. *n is* preferably not more than 9, not more than 8, not more than 7, not more than 6, not more than 5, or not more than 4.

The construct is a nucleic acid construct composed of *n* nucleic acid molecules and comprising (*n*-1) complementary regions, wherein:
a nucleic acid molecule 1 comprises a promoter region, a 5'-side region, and a 3'-side region;
a nucleic acid molecule 2 comprises a first complementary region comprising a first homologous region and a second homologous region; and
an *mth* nucleic acid molecule *m* comprises an (*m*-1)th complementary region comprising an (*m*-1)th substrate region and an *m*th homologous region.

The first homologous region is the homologous region α described above, and the homology between the first homologous region and the region that comprises at least a 3'-end portion of the 5'-side region and that serves as a substrate for the first homologous region in the SSA reaction is not less than 40% and less than 100%.

An (*m*-1)th homologous region is a region for which the (*m*-1)th substrate region serves as a substrate in the SSA reaction, and the homology between these regions is not less than 40% and less than 100%.

An *n*th homologous region contained in a nucleic acid molecule *n* is the homologous region β described above, and the homology between the nth homologous region and the region that comprises at least the 5'-end portion of the 3'-side region and that serves as a substrate for the *n*th homologous region in the SSA reaction is not less than 40% and less than 100%.

The 5'-side region, the first complementary region, the (*m*-1)th complementary region, and the 3'-side region, when arranged in this order (wherein the complementary regions are arranged from smaller to larger numbers), encode an amino acid sequence of protein A/B while each homologous region and the substrate therefor overlap each other, and the SSA reaction that occurs between each homologous region and the substrate therefor results in expression of protein A/B.

For example, in a case where *n* = 3, a nucleic acid construct of (1) is composed of three nucleic acid molecules (nucleic acid molecules 1 to 3) and comprises two complementary regions, wherein in the configuration described above,
the nucleic acid molecule 3 comprises a second complementary region comprising a second substrate region and a third homologous region;
the second homologous region is a region for which the second substrate region serves as a substrate in the SSA reaction, and the homology between these regions is not less than 40% and less than 100%;
the third homologous region is the homologous region β described above, and the homology between the third homologous region and the region that comprises at least a 5'-end portion of the 3'-side region and that serves as a substrate for the third homologous region in the SSA reaction is not less than 40% and less than 100%; and
the 5'-side region, the first complementary region, the second complementary region, and the 3'-side region, when arranged in this order, encode an amino acid sequence of protein A/B while each homologous region and the substrate therefor overlap each other, and the SSA reaction that occurs between each homologous region and the substrate therefor results in expression of protein A/B.

In a case where *n* = 4, a nucleic acid construct of (1) is composed of four nucleic acid molecules (nucleic acid molecules 1 to 4) and comprises three complementary regions, wherein in the configuration described above,
the nucleic acid molecule 3 comprises a second complementary region comprising a second substrate region and a third homologous region;
the nucleic acid molecule 4 comprises a third complementary region comprising a third substrate region and a fourth homologous region;
the second homologous region is a region for which the second substrate region serves as a substrate in the SSA reaction, and the homology between these regions is not less than 40% and less than 100%;
the third homologous region is a region for which the third substrate region serves as a substrate in the SSA reaction, and the homology between these regions is not less than 40% and less than 100%;
the fourth homologous region is the homologous region β described above, and the homology between the fourth homologous region and the region that comprises at least a 5'-end portion of the 3'-side region and that serves as a substrate for the fourth homologous region in the SSA reaction is not less than 40% and less than 100%; and
the 5'-side region, the first complementary region, the second complementary region, the third complementary region, and the 3'-side region, when arranged in this order, encode an amino acid sequence of protein A/B while each homologous region and the substrate therefor overlap each other, and the SSA reaction that occurs between each homologous region and the substrate therefor results in expression of protein A/B.

In the nucleic acid construct of (1), the nucleic acid molecule 1 may be a linear nucleic acid molecule comprising, downstream of the 3'-side region, the promoter region and the 5'-side region in this order, or a circular nucleic acid molecule comprising, downstream of the promoter region, the 5'-side region and the 3'-side region in this order. In cases where a poly-A addition signal is contained, the nucleic acid molecule 1 may be a linear nucleic acid molecule comprising, downstream of the 3'-side region, the poly-A addition signal, the promoter region, and the 5'-side region in this order, or a circular nucleic acid molecule comprising, downstream of the promoter region, the 5'-side region, the 3'-side region, and the poly-A addition signal in this order.

All of the nucleic acid molecules comprising a complementary region, other than the nucleic acid molecule 1, are preferably linear molecules from the viewpoint of simplicity in both the production and the use.

In cases where the nucleic acid molecule 1 is a circular molecule, it may be introduced into the cell as the circular molecule. However, since the nucleic acid molecule 1 is usually linearized by cleavage between the 5'-side region and the 3'-side region before the introduction into the cell, it preferably has a cleavage site between the 5'-side region and the 3'-side region. A typical example of the cleavage site is a restriction enzyme recognition site. However, the cleavage site is not limited thereto. The cleavage may be carried out using a restriction enzyme, or may be carried out, for example, using a genome editing technique that causes a DNA strand break. Specific examples of the cleavage include cleavage by a CRISPR/Cas system, i.e., cleavage by a complex of a guide RNA and a Cas protein such as Cas9. In cases where the complex is used, a PAM sequence is introduced at an appropriate position between the 5'-side region and the 3'-side region. In cases of *Streptococcus pyogenes* type II-derived Cas9, which is the most commonly used system among the known CRISPR/Cas systems, the PAM sequence is 5'-NGG (wherein N is A, T, G, or C). By the complex, the nucleic acid construct is cleaved several bases upstream of the PAM sequence. In the case of the cleavage by the CRISPR/Cas system, the PAM sequence and the actual position of cleavage located several bases upstream thereof constitute the cleavage site in the present invention. A stop codon may be added to the 3'-end of the 5'-side region. By this, in a cell deficient in the SSA activity, expression of a protein having the activity of protein A from the portion of [5'-side region] + [cleavage site (optional)] + [3'-side region] in the circular molecule can be securely prevented. However, the addition of the stop codon to the 5'-side region is not indispensable since the SSA reaction requires a double-strand break end, and since, when a nucleic acid construct of the present invention comprising the circular molecule is introduced into a cell in order to use the construct for evaluation of the SSA activity or the like, a position between the 5'-side region and the 3'-side region is usually cleaved.

Among the nucleic acid constructs of (2), a construct composed of three nucleic acid molecules and comprising one complementary region has the following configuration.

A nucleic acid molecule 1-1 comprises the promoter region and the 5'-side region.

A nucleic acid molecule 1-2 comprises the 3'-side region.

A nucleic acid molecule 2 comprises a complementary region comprising a homologous region α and a homologous region β.

The homology between the homologous region α and a region that comprises at least a 3'-end portion of the 5'-side region and that serves as a substrate for the homologous region α in the SSA reaction is not less than 40% and less than 100%.

The homology between the homologous region β and a region that comprises at least a 5'-end portion of the 3'-side region and that serves as a substrate for the homologous region β in the SSA reaction is not less than 40% and less than 100%.

The 5'-side region, the complementary region, and the 3'-side region, when arranged in this order, encode an amino acid sequence of protein A/B while each homologous region and the substrate therefor overlap each other, and the SSA reaction that occurs between each homologous region and the substrate therefor results in expression of protein A/B.

Among the nucleic acid constructs of (2), a construct composed of four or more nucleic acid molecules and comprising two or more complementary regions can be expressed as follows using an integer *n* that is a constant of 3 ≤ *n* ≤ 10 and an integer *m* that is a variable of 3 ≤ *m* ≤ *n*. *n is* preferably not more than 9, not more than 8, not more than 7, not more than 6, not more than 5, or not more than 4.

The construct is a nucleic acid construct composed of (*n*+1) nucleic acid molecules and comprising (*n*-1) complementary regions,
wherein:
a nucleic acid molecule 1-1 comprises the promoter region and the 5'-side region;
a nucleic acid molecule 1-2 comprises the 3'-side region;
a nucleic acid molecule 2 comprises a first complementary region comprising a first homologous region and a second homologous region; and
an *mth* nucleic acid molecule *m* comprises an (*m*-1)th complementary region comprising an (*m*-1)th substrate region and an mth homologous region.

The first homologous region is the homologous region α described above, and the homology between the first homologous region and the region that comprises at least a 3'-end portion of the 5'-side region and that serves as a substrate for the first homologous region in the SSA reaction is not less than 40% and less than 100%.

An (*m*-1)th homologous region is a region for which the (*m*-1)th substrate region serves as a substrate in the SSA reaction, and the homology between these regions is not less than 40% and less than 100%.

An nth homologous region contained in a nucleic acid molecule *n* is the homologous region β described above, and the homology between the *n*th homologous region and the region that comprises at least the 5'-end portion of the 3'-side region and that serves as a substrate for the *n*th homologous region in the SSA reaction is not less than 40% and less than 100%.

The 5'-side region, the first complementary region, the (*m*-1)th complementary region, and the 3'-side region, when arranged in this order (wherein the complementary regions are arranged from smaller to larger numbers), encode an amino acid sequence of protein A/B while each homologous region and the substrate therefor overlap each other, and the SSA reaction that occurs between each homologous region and the substrate therefor results in expression of protein A/B.

For example, in a case where *n* = 3, a nucleic acid construct of (2) is composed of four nucleic acid molecules (nucleic acid molecules 1-1, 1-2, 2, and 3) and comprises two complementary regions, wherein in the configuration described above,
the nucleic acid molecule 3 comprises a second complementary region comprising a second substrate region and a third homologous region;
the second homologous region is a region for which the second substrate region serves as a substrate in the SSA reaction, and the homology between these regions is not less than 40% and less than 100%;
the third homologous region is the homologous region β described above, and the homology between the third homologous region and the region that comprises at least a 5'-end portion of the 3'-side region and that serves as a substrate for the third homologous region in the SSA reaction is not less than 40% and less than 100%; and
the 5'-side region, the first complementary region, the second complementary region, and the 3'-side region, when arranged in this order, encode an amino acid sequence of protein A/B while each homologous region and the substrate therefor overlap each other, and the SSA reaction that occurs between each homologous region and the substrate therefor results in expression of protein A/B.

In a case where *n* = 4, a nucleic acid construct of (2) is composed of five nucleic acid molecules (nucleic acid molecules 1-1, 1-2, 2, 3, and 4) and comprises three complementary regions, wherein in the configuration described above,
the nucleic acid molecule 3 comprises a second complementary region comprising a second substrate region and a third homologous region;
the nucleic acid molecule 4 comprises a third complementary region comprising a third substrate region and a fourth homologous region;
the second homologous region is a region for which the second substrate region serves as a substrate in the SSA reaction, and the homology between these regions is not less than 40% and less than 100%;
the third homologous region is a region for which the third substrate region serves as a substrate in the SSA reaction, and the homology between these regions is not less than 40% and less than 100%;
the fourth homologous region is the homologous region β described above, and the homology between the fourth homologous region and the region that comprises at least a 5'-end portion of the 3'-side region and that serves as a substrate for the fourth homologous region in the SSA reaction is not less than 40% and less than 100%; and
the 5'-side region, the first complementary region, the second complementary region, the third complementary region, and the 3'-side region, when arranged in this order, encode an amino acid sequence of protein A/B while each homologous region and the substrate therefor overlap each other, and the SSA reaction that occurs between each homologous region and the substrate therefor results in expression of protein A/B.

From the viewpoint of simplicity in both the production and the use, all nucleic acid molecules constituting the nucleic acid construct of (2) are preferably prepared as linear nucleic acid molecules. However, part or all of the nucleic acid molecules may be prepared as circular molecules. In cases where the nucleic acid construct of (2) comprises a poly-A addition signal, the nucleic acid molecule 1-2, which contains the 3'-side region, comprises the poly-A addition signal downstream of the 3'-side region.

The nucleic acid construct of (3) is composed of one circular or linear nucleic acid molecule.

A construct of (3) composed of one circular nucleic acid molecule and comprising one complementary region has the following configuration.

The circular nucleic acid molecule comprises, downstream of the promoter region, the 5'-side region, a cleavage site, and the 3'-side region in this order. A complementary region comprising a homologous region α and a homologous region β is contained at a position upstream of the promoter region and downstream of the 3'-side region. Thus, in the one circular nucleic acid molecule, the elements are placed in the order of [promoter]-[5'-side region]-[cleavage site]-[3'-side region]-[complementary region] from upstream to downstream. The complementary region may be placed in the direction in which the homologous region α is positioned upstream, or may be placed in the direction in which the homologous region β is positioned upstream. In cases where a poly-A addition signal is contained, the poly-A addition signal is placed such that it is operably linked to the 3'-side region, and therefore the complementary region is placed downstream of the poly-A addition signal.

The homology between the homologous region α and the region comprising at least the 3'-end portion of the 5'-side region which serves as a substrate for the homologous region α in the SSA reaction is not less than 40% and less than 100%.

The homology between the homologous region β and the region comprising at least the 5'-end portion of the 3'-side region which serves as a substrate for the homologous region β in the SSA reaction is not less than 40% and less than 100%.

The 5'-side region, the complementary region, and the 3'-side region, when arranged in this order with the complementary region being in the direction of "complementary region α → complementary region β", encode an amino acid sequence of protein A/B while each homologous region and the substrate therefor overlap each other, and the SSA reaction that occurs between each homologous region and the substrate therefor results in expression of protein A/B.

The cleavage site is as described for the nucleic acid construct of (1), and includes, for example, a restriction enzyme recognition site.

In cases where the construct is of a type in which a complementary region is contained in a nucleic acid molecule together with other elements, the complementary region is preferably cleaved out from the nucleic acid molecule, and used as a separate nucleic acid molecule. Therefore, in the nucleic acid construct of (3), cleavage sites are preferably placed on both sides of the complementary region in the nucleic acid molecule. In cases where these cleavage sites as well as the cleavage site between the 5'-side region and the 3'-side region are of the same type, all cleavage sites can be simply cleaved by one time of cleavage treatment (for example, by treatment with one type of restriction enzyme).

Among the nucleic acid constructs of (3), a construct composed of one circular nucleic acid molecule and comprising two or more complementary regions can be expressed as follows using an integer *n* that is a constant of 3 ≤ *n* ≤ 10 and an integer *m* that is a variable of 3 ≤ *m* ≤ n. *n* is preferably not more than 9, not more than 8, not more than 7, not more than 6, not more than 5, or not more than 4.

A nucleic acid construct composed of one circular nucleic acid molecule and comprising (*n*-1) complementary regions has the following configuration.

The circular nucleic acid molecule comprises, downstream of the promoter region, the 5'-side region, a cleavage site, and the 3'-side region in this order, and comprises, upstream of the promoter region but downstream of the 3'-side region, (*n-*1) complementary regions. In cases where two or more complementary regions are contained, the order of arrangement of the complementary regions is not limited at all. They may be arranged in the order of numbering, or may be arranged in a random order. The direction of the complementary regions is also not limited at all.

A first complementary region comprises a first homologous region and a second homologous region; and
an (*m*-1)th complementary region comprises an (*m*-1)th substrate region and an mth homologous region.

The first homologous region is the homologous region α for which the region comprising at least the 3'-end portion of the 5'-side region serves as a substrate in the SSA reaction, and the homology between the substrate and the first homologous region is not less than 40% and less than 100%.

An (*m*-1)th homologous region is a region for which the (*m*-1)th substrate region serves as a substrate in the SSA reaction, and the homology between these regions is not less than 40% and less than 100%.

An *n*th homologous region contained in an (*n*-1)th complementary region is the homologous region β for which the region comprising at least the 5'-end portion of the 3'-side region serves as a substrate in the SSA reaction, and the homology between the substrate and the nth homologous region is not less than 40% and less than 100%.

The 5'-side region, the first complementary region, the (*m*-1)th complementary region, and the 3'-side region, when arranged in this order (wherein the complementary regions are arranged from smaller to larger numbers), encode an amino acid sequence of protein A/B while each homologous region and the substrate therefor overlap each other, and the SSA reaction that occurs between each homologous region and the substrate therefor results in expression of protein A /B.

For example, in a case where *n* = 3, a nucleic acid construct of (3) is composed of one circular nucleic acid molecule and comprises two complementary regions, wherein in the configuration described above,
the circular nucleic acid molecule comprises, at a position upstream of the promoter region but downstream of the 3'-side region, the two complementary regions, that is, the first and second complementary regions;
the second complementary region comprises a second substrate region and a third homologous region;
the second homologous region is a region for which the second substrate region serves as a substrate in the SSA reaction, and the homology between these regions is not less than 40% and less than 100%;
the third homologous region is the homologous region β described above, and the homology between the third homologous region and the region that comprises at least a 5'-end portion of the 3'-side region and that serves as a substrate for the third homologous region in the SSA reaction is not less than 40% and less than 100%; and
the 5'-side region, the first complementary region, the second complementary region, and the 3'-side region, when arranged in this order, encode an amino acid sequence of protein A/B while each homologous region and the substrate therefor overlap each other, and the SSA reaction that occurs between each homologous region and the substrate therefor results in expression of protein A/B.

In a case where *n* = 4, a nucleic acid construct of (3) is composed of one circular nucleic acid molecule and comprises three complementary regions, wherein in the configuration described above,
the circular nucleic acid molecule comprises, at a position upstream of the promoter region but downstream of the 3'-side region, the three complementary regions, that is, the first, second, and third complementary regions;
the second complementary region comprises a second substrate region and a third homologous region;
the third complementary region comprises a third substrate region and a fourth homologous region;
the second homologous region is a region for which the second substrate region serves as a substrate in the SSA reaction, and the homology between these regions is not less than 40% and less than 100%;
the third homologous region is a region for which the third substrate region serves as a substrate in the SSA reaction, and the homology between these regions is not less than 40% and less than 100%;
the fourth homologous region is the homologous region β described above, and the homology between the fourth homologous region and the region that comprises at least a 5'-end portion of the 3'-side region and that serves as a substrate for the fourth homologous region in the SSA reaction is not less than 40% and less than 100%; and
the 5'-side region, the first complementary region, the second complementary region, the third complementary region, and the 3'-side region, when arranged in this order, encode an amino acid sequence of protein A/B while each homologous region and the substrate therefor overlap each other, and the SSA reaction that occurs between each homologous region and the substrate therefor results in expression of protein A/B.

A construct of (3) composed of one linear nucleic acid molecule and comprising one complementary region has the following configuration.

The linear nucleic acid molecule comprises, from upstream to downstream, the 3'-side region, the complementary region, the promoter region, and the 5'-side region in this order. The complementary region comprises a homologous region α and a homologous region β. Similarly to the construct of (3) composed of a circular nucleic acid molecule, the direction of the complementary region is not limited. In cases where a poly-A addition signal is contained, the complementary region is placed downstream of the "3'-side region + poly-A addition signal".

The homology between the homologous region α and the region comprising at least the 3'-end portion of the 5'-side region which serves as a substrate for the homologous region α in the SSA reaction is not less than 40% and less than 100%.

The homology between the homologous region β and the region comprising at least the 5'-end portion of the 3'-side region which serves as a substrate for the homologous region β in the SSA reaction is not less than 40% and less than 100%.

The 5'-side region, the complementary region, and the 3'-side region, when arranged in this order with the complementary region being in the direction of "complementary region α → complementary region β", encode an amino acid sequence of protein A/B while each homologous region and the substrate therefor overlap each other, and the SSA reaction that occurs between each homologous region and the substrate therefor results in expression of protein A/B.

Similarly to the construct of (3) composed of a circular molecule, cleavage sites are preferably placed on both sides of the complementary region.

Among the nucleic acid constructs of (3), a construct composed of one linear nucleic acid molecule and comprising two or more complementary regions can be expressed as follows using an integer *n* that is a constant of 3 ≤ *n* ≤ 10 and an integer *m* that is a variable of 3 ≤ *m* ≤ n. *n* is preferably not more than 9, not more than 8, not more than 7, not more than 6, not more than 5, or not more than 4.

A nucleic acid construct composed of one linear molecule and comprising (*n-*1) complementary regions has the following configuration.

The linear nucleic acid molecule comprises, from upstream to downstream, the 3'-side region, the promoter region, and the 5'-side region in this order, and comprises, between the 3'-side region and the promoter region, (*n*-1) complementary regions. Similarly to the case of a circular molecule, the order of arrangement and the direction of the complementary regions are not limited at all.

A first complementary region comprises a first homologous region and a second homologous region; and
An (*m*-1)th complementary region comprises an (*m*-1)th substrate region and an mth homologous region.

The first homologous region is the homologous region α for which the region comprising at least the 3'-end portion of the 5'-side region serves as a substrate in the SSA reaction, and the homology between the substrate and the first homologous region is not less than 40% and less than 100%.

An (*m*-1)th homologous region is a region for which the (*m*-1)th substrate region serves as a substrate in the SSA reaction, and the homology between these regions is not less than 40% and less than 100%.

An nth homologous region contained in an (*n*-1)th complementary region is the homologous region β for which the region comprising at least the 5'-end portion of the 3'-side region serves as a substrate in the SSA reaction, and the homology between the substrate and the *n*th homologous region is not less than 40% and less than 100%.

The 5'-side region, the first complementary region, the (*m*-1)th complementary region, and the 3'-side region, when arranged in this order (wherein the complementary regions are arranged from smaller to larger numbers), encode an amino acid sequence of protein A/B while each homologous region and the substrate therefor overlap each other, and the SSA reaction that occurs between each homologous region and the substrate therefor results in expression of protein A/B.

For example, in a case where *n* = 3, a nucleic acid construct of (3) is composed of one linear nucleic acid molecule and comprises two complementary regions, wherein in the configuration described above,
the linear nucleic acid molecule comprises, between the 3'-side region and the promoter region, two complementary regions, that is, the first and second complementary regions;
the second complementary region comprises a second substrate region and a third homologous region;
the second homologous region is a region for which the second substrate region serves as a substrate in the SSA reaction, and the homology between these regions is not less than 40% and less than 100%;
the third homologous region is the homologous region β described above, and the homology between the third homologous region and the region that comprises at least a 5'-end portion of the 3'-side region and that serves as a substrate for the third homologous region in the SSA reaction is not less than 40% and less than 100%; and
the 5'-side region, the first complementary region, the second complementary region, and the 3'-side region, when arranged in this order, encode an amino acid sequence of protein A/B while each homologous region and the substrate therefor overlap each other, and the SSA reaction that occurs between each homologous region and the substrate therefor results in expression of protein A/B.

In a case where *n* = 4, a nucleic acid construct of (3) is composed of one linear nucleic acid molecule and comprises three complementary regions, wherein in the configuration described above,
the linear nucleic acid molecule comprises, between the 3'-side region and the promoter region, three complementary regions, that is, the first, second, and third complementary regions;
the second complementary region comprises a second substrate region and a third homologous region;
the third complementary region comprises a third substrate region and a fourth homologous region;
the second homologous region is a region for which the second substrate region serves as a substrate in the SSA reaction, and the homology between these regions is not less than 40% and less than 100%;
the third homologous region is a region for which the third substrate region serves as a substrate in the SSA reaction, and the homology between these regions is not less than 40% and less than 100%;
the fourth homologous region is the homologous region β described above, and the homology between the fourth homologous region and the region that comprises at least a 5'-end portion of the 3'-side region and that serves as a substrate for the fourth homologous region in the SSA reaction is not less than 40% and less than 100%; and
the 5'-side region, the first complementary region, the second complementary region, the third complementary region, and the 3'-side region, when arranged in this order, encode an amino acid sequence of protein A/B while each homologous region and the substrate therefor overlap each other, and the SSA reaction that occurs between each homologous region and the substrate therefor results in expression of protein A/B.

The nucleic acid constructs of (4) to (6), which are variants of (1) to (3), have configurations that can be employed for constructs having two or more complementary regions. The following are examples of specific configurations of (4) to (6) in which each construct has up to four complementary regions. Any of these can be designed according to the nucleic acid constructs of (1) to (3).

Configuration examples of the nucleic acid construct of (4), which is a variant of (1) (3):
a construct having two complementary regions, wherein one complementary region is placed in a nucleic acid molecule (nucleic acid molecule 1) together with the regions including the promoter region, and the remaining one complementary region is placed in another nucleic acid molecule;
a construct having three complementary regions, wherein two complementary regions are placed in nucleic acid molecule 1, and wherein the remaining one complementary region is placed in another nucleic acid molecule;
a construct having three complementary regions, wherein one complementary region is placed in nucleic acid molecule 1, and wherein the remaining two complementary regions are (i) placed in one nucleic acid molecule, or (ii) placed separately in two nucleic acid molecules;
a construct having four complementary regions, wherein one complementary region is placed in nucleic acid molecule 1, and wherein the remaining three complementary regions are (i) placed in one nucleic acid molecule, (ii) placed separately in two nucleic acid molecules, or (iii) placed one by one in three nucleic acid molecules;
a construct having four complementary regions, wherein two complementary regions are placed in nucleic acid molecule 1, and wherein the remaining two complementary regions are (i) placed in one nucleic acid molecule, or (ii) placed separately in two nucleic acid molecules; and
a construct having four complementary regions, wherein three complementary regions are placed in nucleic acid molecule 1, and the remaining one complementary region is placed in another nucleic acid molecule.

Configuration examples of the nucleic acid construct of (5), which is a variant of (2):
a construct having two complementary regions, wherein (i) one complementary region is placed in one of the nucleic acid molecule comprising the 5'-side region (nucleic acid molecule 1-1) and the nucleic acid molecule comprising the 3'-side region (nucleic acid molecule 1-2), and one complementary region is placed in the other; or (ii) both of the two complementary regions are placed in one of nucleic acid molecule 1-1 and nucleic acid molecule 1-2;
a construct having three complementary regions, wherein (i) one complementary region is placed in one of nucleic acid molecule 1-1 and nucleic acid molecule 1-2, and two complementary regions are placed in the other; or (ii) all of the three complementary regions are placed in one of nucleic acid molecule 1-1 and nucleic acid molecule 1-2; and
a construct having four complementary regions, wherein (i) one complementary region is placed in one of nucleic acid molecule 1-1 and nucleic acid molecule 1-2, and three complementary regions are placed in the other; (ii) two complementary regions are placed in each of nucleic acid molecule 1-1 and nucleic acid molecule 1-2; or (iii) all of the four complementary regions are placed in one of nucleic acid molecule 1-1 and nucleic acid molecule 1-2.

Configuration examples of the nucleic acid construct of (6), which is a variant of (2):
a construct having two complementary regions, wherein one complementary region is placed in one of nucleic acid molecule 1-1 and nucleic acid molecule 1-2, and wherein one complementary region is placed in a nucleic acid molecule other than nucleic acid molecule 1-1 and nucleic acid molecule 1-2;
a construct having three complementary regions, wherein one complementary region is placed in one of nucleic acid molecule 1-1 and nucleic acid molecule 1-2, and wherein the remaining two complementary regions are placed in one nucleic acid molecule other than nucleic acid molecule 1-1 and nucleic acid molecule 1-2, or individually placed in different nucleic acid molecules;
a construct having three complementary regions, wherein two complementary regions are placed (i) in nucleic acid molecule 1-1, (ii) in nucleic acid molecule 1-2, or (iii) one by one in nucleic acid molecule 1-1 and nucleic acid molecule 1-2, and wherein the remaining one complementary region is placed in a nucleic acid molecule other than nucleic acid molecule 1-1 and nucleic acid molecule 1-2;
a construct having four complementary regions, wherein one complementary region is placed in one of nucleic acid molecule 1-1 and nucleic acid molecule 1-2, and wherein the remaining three complementary regions are (i) placed in one nucleic acid molecule other than nucleic acid molecule 1-1 and nucleic acid molecule 1-2, (ii) placed dividedly in two nucleic acid molecules other than nucleic acid molecule 1-1 and nucleic acid molecule 1-2, or (iii) placed one by one in three nucleic acid molecules other than nucleic acid molecule 1-1 and nucleic acid molecule 1-2;
a construct having four complementary regions, wherein two complementary regions are placed (i) in nucleic acid molecule 1-1, (ii) in nucleic acid molecule 1-2, or (iii) one by one in nucleic acid molecule 1-1 and nucleic acid molecule 1-2, and wherein the remaining two complementary regions are placed in one nucleic acid molecule other than nucleic acid molecule 1-1 and nucleic acid molecule 1-2, or individually placed in different nucleic acid molecules; and
a construct having four complementary regions, wherein three complementary regions are placed (i) in nucleic acid molecule 1-1, (ii) in nucleic acid molecule 1-2, or (iii) dividedly in nucleic acid molecule 1-1 and nucleic acid molecule 1-2, and wherein the remaining one complementary region is placed in a nucleic acid molecule other than nucleic acid molecule 1-1 and nucleic acid molecule 1-2.

The 5'-side region, the 3'-side region, and the complementary regions may be prepared by PCR amplification using appropriate primers, from a known plasmid in which a gene encoding protein A is incorporated, or from cultured cells or a cDNA library of an organism species that expresses protein A. Nucleic acid molecules having decreased homologies between the homologous region and the substrate may be prepared by performing PCR using primers to which mutations have been introduced. Or, nucleic acid molecules whose sequences have been determined may be prepared by a well-known chemical synthesis method. Other essential elements and optional elements, such as the promoter region, may be prepared from known plasmids.

The circular nucleic acid molecule may be prepared by incorporating the essential elements in the present invention and, when desired, an optional element(s), into a plasmid vector. The linear nucleic acid molecule may be prepared entirely by chemical synthesis or artificial gene synthesis, or may be prepared by linking the essential elements in the present invention and, when desired, an optional element(s), by fusion PCR or the like. The linear nucleic acid molecule may also be prepared by preparing a circular nucleic acid molecule and then cleaving it at an appropriate site (such as a cleavage site placed in the circular nucleic acid molecule). Further, the nucleic acid construct of the present invention may be prepared by incorporating the essential elements and, when desired, an optional element(s), into a virus vector. In cases where the nucleic acid construct of the present invention is in the form of a vector in which those elements are incorporated, the nucleic acid construct may comprise, in addition to the above-described elements in the present invention, general elements depending on the type of the vector, such as a replication origin, a translation initiation site, and a selection marker gene including a drug resistance gene. The same applies to circular nucleic acid molecules and linear nucleic acid molecules of nucleic acid constructs of the second aspect.

### [Uses of Nucleic Acid Construct of First Aspect)

The nucleic acid construct of the first aspect can be preferably used as a therapeutic agent or diagnostic agent for mismatch repair-deficient cancer, or as a companion diagnostic agent for predicting an effect of an anticancer drug for mismatch repair-deficient cancer. In the following description of various uses of the construct of the first aspect, the protein encoded by the gene sequence formed by the SSA reaction is referred to as "protein A". However, when a gene sequence of protein B is formed, "protein A" is read as "protein B".

Mismatch repair-deficient cancers have been reported in various cancers. Specific examples of the mismatch repair-deficient cancers that have been reported so far include colorectal cancer, endometrial cancer, gastric cancer, esophagus cancer, ovarian cancer, breast cancer, pancreatic cancer, hepatobiliary cancer, urinary tract cancer, small intestinal cancer, prostate cancer, liver cancer, brain tumor, small intestinal cancer, cervical cancer, neuroendocrine tumor, bile duct cancer, uterine sarcoma, thyroid cancer, retroperitoneal or peritoneal sarcoma, uveal malignant melanoma, lung cancer, other malignant tumors of the female genital organs, cancers of unknown primary, and malignant soft tissue tumors (for example, Non-Patent Document 3). It is expected that mismatch repair-deficient cancers will be found also in various cancers other than these (including cancers of various animal species including non-human animals) in the future. It has also been reported that the mismatch repair activity is decreased by molecular targeted cancer therapies using an EGFR inhibitor, a BRAF inhibitor, or the like (Science 20 Dec 2019: Vol. 366, Issue 6472, pp 1473-1480, DOI:10.1126/science.aav4474). Another well-known example of a mismatch repair-deficient cancer is Lynch syndrome, which is a familial (genetic) cancer (another name: hereditary non-polyposis colorectal cancer (HNPCC)). Examples of the mismatch repair-deficient cancer to be targeted when the nucleic acid construct of the present invention is used as a therapeutic agent or diagnostic agent include various cancers including the specific examples described above. When the term "colorectal cancer" is simply used in the present invention, it includes not only nonhereditary colorectal cancer, but also Lynch syndrome (hereditary non-polyposis colorectal cancer). In the present invention, the term "cancer" has the same meaning as malignant tumor, and includes malignant tumors that have occurred from epithelial cells (cancers in the narrow sense), as well as sarcomas. The term "mismatch repair deficiency" means decreased mismatch repair activity compared to those of cells having normal mismatch repair activity (for example, the later-described MMR-proficient control cells), and is not limited to the complete deficiency of mismatch repair.

In cases where the nucleic acid construct of the present invention is used as a therapeutic agent for mismatch repair-deficient cancer, the gene sequence encoding protein A may be a gene sequence encoding a protein that acts to decrease the cell survival rate. In cases of a cancer which is not deficient in mismatch repair, single-strand annealing of the nucleic acid construct is blocked by the mismatch repair mechanism, so that the gene sequence encoding protein A is not reconstructed in the cells, and protein A is not expressed. On the other hand, in cases of a cancer which is deficient in mismatch repair, single-strand annealing of the nucleic acid construct occurs in the cells to reconstruct the gene sequence encoding protein A in the cells, so that protein A is expressed to enable efficient killing of the cancer cells. The effect to kill cancer cells cannot be obtained in the case of a cancer in which mismatch repair is not deficient, and therefore, before the administration of the therapeutic agent of the present invention, whether or not the cancer is a mismatch repair-deficient cancer may be investigated using, for example, the diagnostic agent of the present invention, and the therapeutic agent of the present invention may then be administered to the patient when the patient is diagnosed with mismatch repair-deficient cancer. In cases where protein A is a protein that acts on another compound to produce a toxic substance, the compound that is the target of the action is also administered to the patient. For example, in cases where protein A is HSV-tk, the nucleic acid construct of the present invention may be administered in combination with ganciclovir, FIAU, or the like.

In cases where the nucleic acid construct of the present invention is used as a diagnostic agent for mismatch repair-deficient cancer, the nucleic acid construct of the present invention is introduced into cancer cells of the cancer patient, and expression of protein A is measured. The measurement of the expression of protein A is preferably carried out by directly or indirectly measuring the activity of protein A as described above. As the gene sequence encoding protein A employed for the 5'-side region and the 3'-side region, a gene sequence encoding a protein whose intracellular expression is detectable may be preferably used, and a gene sequence encoding a protein that acts to decrease the cell survival rate may also be used. In cases where mismatch repair is not deficient in the cell, single-strand annealing of the nucleic acid construct is blocked by the mismatch repair mechanism, so that the gene sequence encoding protein A is not reconstructed in the cell, and the activity of protein A is not detected. In cases where mismatch repair is deficient in the cell, single-strand annealing of the nucleic acid construct occurs to reconstruct the gene sequence encoding protein A in the cell, so that the protein A activity is detected.

In one mode of the diagnostic agent for mismatch repair-deficient cancer, the cancer cells are cells separated from a cancer patient, and the introduction of the nucleic acid construct into the cancer cells is carried out *in vitro.* Among the modes of use described later, (a) and (c) are specific examples of this mode. In this mode, either a protein whose intracellular expression is detectable or a protein that acts to decrease the cell survival rate may be used as protein A, and the former is more preferred. In this mode, the cancer patient includes solid cancer patients and blood cancer patients. Cells in a cancer tissue separated from a cancer patient by biopsy or surgery, or, in cases of leukemia, cancer cells in blood collected from a cancer patient, may be used. If desired, the cancer cells may be concentrated before the introduction of the nucleic acid construct. The introduction of the nucleic acid construct may be transient.

After the introduction of the nucleic acid construct, expression of protein A, preferably the activity of protein A, in the cancer cells is measured. In cases where protein A is a protein whose intracellular expression is detectable, the expression (activity) of protein A can be measured by measuring a signal of protein A. In cases where protein A is a protein that acts to decrease the cell survival rate, the expression (activity) of protein A can be measured by measuring the survival rate of the cancer cells after the introduction of the nucleic acid construct. Preferably, in the measurement of the expression of protein A in cancer cells, cells which have normal mismatch repair activity or which are not deficient in mismatch repair activity are used as an MMR-proficient control, and, after introducing the nucleic acid construct into the cancer cells and the control cells, the expression of protein A is compared between the cancer cells and the control cells.

Preferred examples of the control cells include non-cancerous cells (cells not deficient in the mismatch repair activity) collected from the same cancer patient. Or, a known cell line not deficient in the mismatch repair activity may be used as the control cells. A number of wild-type mammalian cell lines having no mutation in MMR-related genes (such as *MSH2, MSH6, MLH1, PMS2, EPCAM, MSH3, PMS1, MLH3, ARID1A, SETD2, EXO1, RPA1, RPA2, RPA3, POLD, LIG1,* and *LIG3*) are known, and any of such known lines may be used as the MMR-proficient control cells. Specific examples of known mammalian cell lines having normal mismatch repair activity include, but are not limited to, human cell lines such as HT1080 (a human fibrosarcoma cell line), U2OS (a human osteosarcoma-derived cell line), HeLa (a human cervical cancer-derived cell line), MCF-7 (a human breast adenocarcinoma-derived cell line), HAP1 (a human chronic myelogenous leukemia-derived cell line), HEK293 (a human embryonic kidney-derived cell line), TIG-7, TIG-3 (a human lung-derived cell line), iPS cells (human induced pluripotent stem cells; established from normal human cells), and ES cells (human embryonic stem cells).

Further, if desired, cells deficient in the mismatch repair activity may be used as an additional control. Examples of such MMR-deficient control cells include cell lines derived from mismatch repair-deficient cancers. Various cell lines derived from mismatch repair-deficient cancers having a mutation(s) in the MMR-related genes described above are known, and any of such cells may be used as the MMR-deficient control cells. Or, a cell line prepared by introducing a mutation(s) to one or more MMR-related genes can also be used. Since the technique for gene knockout has been established, MMR-deficient cells of various animal species can be prepared by knocking out one or more MMR-related genes in mammalian cultured cells having normal mismatch repair activity. Specific examples of known MMR-deficient human cell lines include Nalm-6 (a human B-cell leukemia-derived cell line), LoVo (a human colon adenocarcinoma-derived cell line), Jurkat (a human T-cell leukemia-derived cell line), and C-33 A (a human cervical cancer-derived cell line), which are deficient in MSH2; HCT116 (a human colon adenocarcinoma-derived cell line), SK-OV-3 (a human ovarian cancer-derived cell line), and DU-145 (a human prostate cancer-derived cell line), which are deficient in MLH1; DLD-1(a human colon adenocarcinoma-derived cell line), which is deficient in MSH6; and MMR-deficient human cell lines available from Horizon Discovery (the following gene-deficient cells are available as genetically modified HAP1 cell lines (human chronic myelogenous leukemia-derived cell lines): an MSH2-deficient line, an MLH1-deficient line, an MSH6-deficient line, and a PMS2-deficient line). Specific examples of the non-human animal cell lines include, but are not limited to, MMR-deficient mice (frozen embryos) available from The NCI Mouse Repository (Msh2-deficient mice, Mlh1-deficient mice, and Msh6-deficient mice); the canine cancer cell line TYLER2 (Dodd G. Sledge et al., Proceedings of the 103rd Annual Meeting of the American Association for Cancer Research; 2012 Mar 31-Apr 4; Chicago, IL. Philadelphia (PA): AACR; Cancer Res 2012; 72 (8 Suppl): Abstract nr 5258. doi:1538-7445. AM2012-5258); and the MMR-deficient canine cell Angus (MSH6-deficient) (Sunetra Das et al., Mol Cancer Ther. Author manuscript; available in PMC 2020 Feb 1. Published in final edited form as: Mol Cancer Ther. 2019 Aug; 18(8): 1460-1471. Published online 2019 Jun 7. doi: 10.1158/1535-7163. MCT-18-1346).

When the expression level or activity level of protein A in the cancer cells is higher than the expression level or activity level of protein A in the MMR-proficient control cells, the cancer in the cancer patient is indicated to be a mismatch repair-deficient cancer. In cases where protein A is a protein whose intracellular expression is detectable as a signal, when the detected signal of protein A is higher in the cancer cells than in the MMR-proficient control cells, the cancer in the cancer patient is indicated to be a mismatch repair-deficient cancer. In cases where protein A is a protein that acts to decrease the cell survival rate, when the survival rate of the cancer cells is lower than the survival rate of the MMR-proficient control cells, the cancer in the cancer patient is indicated to be a mismatch repair-deficient cancer.

When the expression level or activity level of protein A in the cancer cells is not higher than (about the same as, or lower than) the expression level or activity level of protein A in the MMR-proficient control cells, the cancer in the cancer patient is indicated not to be a mismatch repair-deficient cancer. In cases where protein A is a protein whose intracellular expression is detectable as a signal, when the level of the signal detected in the cancer cells is about the same as or lower than the level in the MMR-proficient control cells, the cancer in the cancer patient is indicated not to be a mismatch repair-deficient cancer. In cases where protein A is a protein that acts to decrease the cell survival rate, when the survival rate of the cancer cells is about the same as or higher than the survival rate of the MMR-proficient control cells, the cancer in the cancer patient is indicated not to be a mismatch repair-deficient cancer.

In another mode of the diagnostic agent for mismatch repair-deficient cancer, protein A is a protein whose intracellular expression is detectable as a signal; the introduction of the nucleic acid construct into the cancer cells is carried out by administering the nucleic acid construct to the patient; and whether or not the signal of protein A is detected from a cancer lesion is investigated. Among the modes of use described later, (b) is a specific example of this mode. The nucleic acid construct may be topically administered into the tumor or into the vicinity of the tumor of the cancer patient, or may be systemically administered. Topical administration is more preferred. In this mode, the cancer patient is typically a solid cancer patient. From the viewpoint of detecting the signal of protein A from the cancer lesion in the body of the cancer patient, protein A is preferably a protein that generates the signal without a reaction with a substrate, for example, a fluorescent protein. When the signal of protein A is detected from the cancer lesion (for example, when the signal from the cancer lesion is clearly higher than the signal from a non-cancerous site in the vicinity of the tumor), the cancer of the cancer patient is indicated to be a mismatch repair-deficient cancer. When no signal of protein A is detected from the cancer lesion, or when a signal as low as that of a non-cancerous site in the vicinity of the tumor is detected, a possibility that the cancer of the cancer patient is not a mismatch repair-deficient cancer is indicated. If desired, cancer cells may be collected from the patient, and the nucleic acid construct may be introduced thereto in vitro to further confirm whether or not the cancer is a mismatch repair-deficient cancer.

In still another mode, protein A is a secretory protein whose intracellular expression is detectable; the introduction of the nucleic acid construct into the cancer cells is carried out by administering the nucleic acid construct to the cancer patient; and the activity of protein A in blood separated from the patient after the administration of the nucleic acid construct is measured. Among the modes of use described later, (d) is a specific example of this mode. The nucleic acid construct may be topically administered into the tumor or into the vicinity of the tumor of the cancer patient, or may be systemically administered. Topical administration is more preferred. In this mode, the cancer patient is typically a solid cancer patient. In cases where the cancer of the patient is deficient in mismatch repair, secretory protein A is expressed from the protein A gene reconstructed in the cancer cells, and secreted to the outside of the cancer cells. Therefore, the activity of protein A can be detected using a blood sample of the cancer patient. Since the activity of protein A secreted into the blood is detected in vitro, a protein that generates a signal by reaction with a substrate can also be preferably used. Typical examples of such a protein include secretory luminescent enzymes, especially secretory luciferases. As the gene sequence encoding the secretory protein such as a secretory luciferase, a known secretory protein coding sequence may be used as it is, or the gene sequence may be prepared by adding a sequence encoding a secretory signal to a protein coding sequence. As a negative control sample, for example, a blood sample collected from a patient before the administration of the nucleic acid construct, or a culture supernatant obtained by introducing the nucleic acid construct to an animal cell line not deficient in mismatch repair and culturing the resulting cells may be used. As a positive control, for example, a culture supernatant obtained by introducing the nucleic acid construct into a cell line deficient in the mismatch repair activity (such as a cell line derived from a mismatch repair-deficient cancer) and culturing the resulting cells may be used. When the signal of protein A is detected in a blood sample (for example, when a signal of protein A higher than that of a negative control sample is detected in a blood sample), the cancer of the patient is indicated to be a mismatch repair-deficient cancer. When the signal of protein A is not detected in a blood sample, in particular, when a signal higher than that of a negative control is not detected (in other words, when the level of the signal detected is about the same as or lower than the level in a negative control sample), a possibility that the cancer of the cancer patient is not a mismatch repair-deficient cancer is indicated. If desired, cancer cells may be collected from the patient, and the nucleic acid construct may be introduced thereto *in vitro* to further confirm whether or not the cancer is a mismatch repair-deficient cancer.

In cases where a patient is diagnosed with mismatch repair-deficient cancer by the diagnostic technique for mismatch repair-deficient cancer according to the present invention, an immune checkpoint inhibitor is likely to be effective for the patient, so that an immune checkpoint inhibitor may be preferably administered to the patient. In cases where a patient is diagnosed as not having mismatch repair-deficient cancer, severe side effects are likely to occur without production of a sufficient cancer therapeutic effect by the immune checkpoint inhibitor, so that an anticancer drug other than an immune checkpoint inhibitor may be preferably administered to the patient.

Examples of modes of the diagnostic agent of the present invention include, but are not limited to, the following modes.
(a) Cancer cells collected from a cancer patient are cultured, and the nucleic acid construct of the present invention is introduced into the cultured cancer cells, followed by investigating whether or not a signal of protein A is detected (or investigating whether or not a cytocidal effect by protein A is found). If desired, non-cancerous cells (such as peripheral blood cells) collected from the same cancer patient may be used as a control. In cases where protein A is a luciferase, a substrate of the luciferase is added for the detection reaction. In cases where protein A is a fluorescent protein, a fluorescence signal may be detected using a luminometer or the like. When the signal of protein A is detected (in particular, when a signal of protein A higher than that of control non-cancerous cells is detected), the cancer of the patient can be judged to be deficient in mismatch repair. When no signal of protein A is detected, or when a signal as low as that of control non-cancerous cells is detected, the cancer of the patient can be judged not to be deficient in mismatch repair.
   The method of measuring the cell survival rate is well known in the art, and the measurement can be easily carried out using a commercially available kit or the like. When cancer cells into which the nucleic acid construct has been introduced show a decreased survival rate (in particular, when the survival rate is lower than that of control non-cancerous cells), the cancer of the patient can be judged to be deficient in mismatch repair. When no decrease in the survival rate of cancer cells is found, or when the survival rate is almost the same as that of control non-cancerous cells, the cancer of the patient can be judged not to be deficient in mismatch repair.
(b) The nucleic acid construct of the present invention is administered to a patient, and whether or not a signal of protein A is detected from a cancer lesion is investigated. When the signal is detected, the cancer of the patient can be judged to be deficient in mismatch repair. When no signal is detected, it can be judged that the cancer of the patient may not be deficient in mismatch repair. In this mode, a protein whose intracellular expression is detectable is used as protein A. Biotoxicity of the protein employed needs to be sufficiently low.
(c) Cancer cells present in blood are isolated from a cancer patient, and then concentrated when necessary, followed by introducing the nucleic acid construct of the present invention to the cells and investigating whether or not a signal of protein A is detected (or whether or not a cytocidal effect (a decreased cell survival rate) due to protein A is found) (in cases of leukemia).
(d) A nucleic acid construct that employs a gene sequence encoding a secretory luciferase as the gene sequence encoding protein A is administered to a patient. Thereafter, a blood sample is collected, and the presence or absence of luciferase reaction is investigated using the blood sample. In cases where the cancer of the patient is deficient in mismatch repair, the secretory luciferase is expressed from the luciferase gene reconstructed in the cancer cells, and secreted to the outside of the cells. Thus, the luciferase reaction can be detected using the blood sample. In cases where the cancer of the patient is not deficient in mismatch repair, the secretory luciferase is not produced, so that the luciferase reaction is not detected in the blood sample.

The diagnostic agent of the present invention is also useful as a companion diagnostic agent for predicting an effect of an anticancer drug for mismatch repair-deficient cancer. Examples of the anticancer drug include immune checkpoint inhibitors. Specific examples of the immune checkpoint inhibitors include, but are not limited to, anti-PD-1 antibodies such as nivolumab, pembrolizumab, spartalizumab, and cemiplimab; anti-PD-L1 antibodies such as avelumab, atezolizumab, and durvalumab; and anti-CTLA-4 antibodies such as ipilimumab and tremelimumab. Specific examples of modes of use as a companion diagnostic agent include those described above for the diagnostic agent. In other words, also in cases where the nucleic acid construct of the present invention is used as a companion diagnostic agent to predict an effect of an anticancer drug against mismatch repair-deficient cancer, similarly to the above-described diagnostic technique for mismatch repair-deficient cancer, the nucleic acid construct of the present invention may be introduced into cancer cells of a cancer patient, and expression of protein A (preferably the activity of protein A) may be measured to diagnose whether or not the patient has mismatch repair-deficient cancer. When the patient is diagnosed with mismatch repair-deficient cancer, the patient can be judged to be a patient in whom a desired anticancer effect is likely to be obtained with an anticancer drug against mismatch repair-deficient cancer, such as an immune checkpoint inhibitor (in other words, it can be predicted that an anticancer drug against mismatch repair-deficient cancer, such as an immune checkpoint inhibitor, is effective for the patient). Thus, to such a cancer patient, an immune checkpoint inhibitor may be preferably administered for the treatment of the cancer. It has been reported that immune checkpoint inhibitors may cause severe side effects. By preliminarily investigating whether or not the cancer is mismatch repair-deficient cancer, administration of an immune checkpoint inhibitor can be avoided in patients in whom it may produce side effects without producing a therapeutic effect.

Further, the nucleic acid construct of the present invention can also be applied to search for factors that positively or negatively regulate, and inhibitors or activators of, the reaction of mismatch repair or single-strand annealing, and to prediction of whether or not a gene mutation identified in a cancer patient is a pathogenic mutation that impairs the mismatch repair activity. By using a nucleic acid construct having a homology of 100% between the homologous regions as a control, and using a nucleic acid construct having a low homology in combination therewith, a drug or a mutation that affects the mismatch repair activity can be identified. For example, prediction of a pathogenic mutation may be carried out as follows. When a mutation is identified in gene Q (gene Q is preferably an MMR-related gene) in cancer cells of a cancer patient, an expression vector that expresses a mutant gene Q having the same mutation and an expression vector that expresses wild-type gene Q having no mutation are constructed, and these expression vectors are introduced into cells deficient in gene Q (the cells may be cells prepared by knocking out gene Q, or, if there is a known cell line completely deficient in the function of gene Q, such a known cell line may be used). Further, nucleic acid constructs of the present invention (the construct having a homology of 100% and the low-homology construct) are introduced into the cells, and the expression level of protein A in the cells is measured. By comparing expression of protein A between the cells into which the nucleic acid construct having a homology of 100% has been introduced and the cells into which the low-homology nucleic acid construct has been introduced, and between the cells into which the mutant gene Q has been introduced and the cells into which wild-type gene Q has been introduced, whether or not the mutation is a pathological mutation that deteriorates the mismatch repair activity can be predicted.

In cases where the agent of the present invention is administered to a patient, the administration route may be either oral administration or parenteral administration. In general, parenteral administration such as intravenous administration, intraarterial administration, subcutaneous administration, or intramuscular administration is preferred. The administration may be systemic administration, administration into the tumor or to the vicinity of the tumor, or administration to a regional lymph node of the tumor. The dose is not limited, and may be about 1 pg to 10 g, for example, about 0.01 mg to 100 mg, in terms of the amount of the nucleic acid construct per day for a patient. The agent may be administered in a single dose or several divided doses per day. The agent may be administered every day, or may be administered every several days or several weeks.

The dosage form of the agent of the present invention to be administered to the patient is not limited. Depending on the administration route, the agent may be prepared by appropriately mixing the nucleic acid construct of the present invention with an additive(s) such as a pharmaceutically acceptable carrier, diluent, or excipient. Examples of the formulation include parenteral formulations such as drops, injection solutions, suppositories, and inhalants; and oral formulations such as tablets, capsules, granules, powders, and syrups. Preparation methods and additives that can be used are well known in the field of pharmaceutical formulations, and any of the methods and additives may be used.

In cases of a diagnostic agent to be used in vitro, the amount of the agent used for the treatment of the cells may be, for example, about 10 pg to 1 mg, e.g. about 0.1 ng to 100 µg, in terms of the amount of the nucleic acid construct per 1,000,000 cells. The dosage form may be a liquid agent supplemented, if desired, with an additive(s) or the like useful for stability or the like of the nucleic acid construct, or may be a powder agent prepared by freeze-drying of the nucleic acid construct.

In cases where the nucleic acid construct of the present invention is used as a therapeutic agent or a diagnostic agent, examples of the target cancer patient include cancer patients of various mammals such as humans, dogs, cats, ferrets, hamsters, mice, rats, horses, and pigs. The nucleic acid construct of the present invention is applicable to various animal species without changing the components except that the origin of the promoter is changed when necessary.

### [Structure of Nucleic Acid Construct of Second Aspect]

The nucleic acid construct of the second aspect is a type of a construct originally comprising a gene sequence encoding a protein, in which occurrence of SSA reaction causes the gene sequence to be operably linked to a promoter, to thereby enable expression of the protein from the gene sequence. This nucleic acid construct comprises: a promoter region; two substrate regions α and β; a gene sequence encoding protein X (which may be hereinafter referred to as gene sequence X); and at least one complementary region comprising two homologous regions for which substrate regions α and β, respectively, serve as substrates in SSA reaction; which are placed in one nucleic acid molecule or in two or more different nucleic acid molecules.

The substrate region α is placed downstream of the promoter region in a single nucleic acid molecule, wherein the other elements may be placed in said nucleic acid molecule or in a different nucleic acid molecule(s). Focusing on the placement of the four elements other than the complementary region (the promoter region, the substrate region α, the substrate region β, and the gene sequence X), nucleic acid constructs of the second aspect can be divided into the following two types of constructs: constructs in which the four elements are placed in the same nucleic acid molecule, and constructs in which the promoter region and the substrate region α are placed in one nucleic acid molecule, and the substrate region β and the gene sequence X are placed in another nucleic acid molecule. The complementary region(s) may be placed in a nucleic acid molecule that is different from the nucleic acid molecule(s) of the other elements, or may be placed in a nucleic acid molecule together with at least one of the other elements.

The definitions, conditions, and preferred examples for the protein X and the gene sequence X encoding it are the same as those for the protein A and the gene sequence encoding it in the nucleic acid construct of the first aspect. Briefly, preferred examples of the protein X include proteins that act to decrease the cell survival rate, or proteins whose intracellular expression is detectable. Preferred specific examples of the gene sequence X encoding such a protein include sequences of a suicide gene, a DNA damage-inducing gene, a DNA repair-inhibiting gene, a luminescent enzyme gene, a fluorescent protein gene, a cell surface antigen gene, a secretory protein gene, or a membrane protein gene.

A poly-A addition signal may be functionally linked downstream of the gene sequence X. In cases where the poly-A addition signal is linked, the other elements, such as a complementary region, are not placed between the gene sequence X and the poly-A addition signal. As in the nucleic acid construct of the first aspect, the poly-A addition signal is not an essential element, and may be omitted.

The conditions and preferred examples of the promoter region are also the same as those of the nucleic acid construct of the first aspect.

The at least one complementary region comprises: one homologous region (hereinafter referred to as homologous region α) for which the substrate region α serves as a substrate in the SSA reaction; and one homologous region (hereinafter referred to as homologous region β) for which the substrate region β serves as a substrate in the SSA reaction. As in the complementary regions in the first nucleic acid construct, the homologous region α and the homologous region β are contained in the same complementary region or different complementary regions. In the case of a two-SSA-reaction type construct, in which the protein is expressed by two times of SSA reaction, one complementary region is present, and the one complementary region comprises the homologous region α and the homologous region β. In the case of a three- or more-SSA-reaction type construct, in which the protein is expressed by three or more times of SSA reaction, two or more complementary regions are included, and the homologous region α and the homologous region β are included in different complementary regions. The two or more complementary regions of a three- or more-SSA-reaction type construct thus contain, in addition to the homologous region α and the homologous region β, one or more pairs of a further homologous region and the substrate therefor to allow the occurrence of the SSA reaction between the complementary regions.

The homology between each homologous region and the substrate region that serves as the substrate therefor is not less than 40% and less than 100% as described in the first aspect, and the upper limit and lower limit thereof may be selected from the values described above. As described in the first aspect, mismatched bases are preferably dispersed throughout the homologous region or the substrate therefor.

The chain lengths of the homologous regions and the substrate regions are basically not limited as long as they are 4 bases to 10,000 bases, similarly to those in the first aspect. Their upper limit and lower limit may be selected from the values described in the first aspect.

The base sequence resulting from the SSA reaction that occurs between each homologous region and the substrate therefor may be a base sequence that does not encode a protein (Type I of the second aspect). In this case, the substrate regions α, β, and the at least one complementary region may be designed based on an arbitrary base sequence that does not encode a protein. A naturally occurring non-coding sequence that has been found in an organism's genome, for example, a repetitive sequence such as a SINE, or an artificial base sequence that is not present in the genome may be used. In a Type I construct, the non-coding sequence resulting from the SSA reaction is used as part of the 5'-untranslated region between the translation initiation site downstream of the promoter and the initiation codon of the gene sequence X. In a nucleic acid construct of the second aspect constructed using a non-coding sequence, occurrence of the SSA reaction causes the gene sequence X to be operably linked to the promoter region, resulting in expression of protein X from the gene sequence X.

Further, the base sequence resulting from the SSA reaction between each homologous region and the substrate therefor may be a base sequence that encodes a protein (Type II of the second aspect). The gene sequence generated by the SSA reaction in the Type II construct, and the protein encoded thereby are hereinafter referred to as gene sequence Y and protein Y, respectively. This type of construct can also be said to be a construct having a structure in which the gene sequence X is placed downstream of the 3'-side region in a construct of the first aspect, wherein the substrate region α, the substrate region β, and the at least one complementary region may be designed by the same method as the 5'-side region, the 3'-side region, and the at least one complementary region in the nucleic acid construct of the first aspect. More strictly speaking, the substrate region α in Type II of the second aspect corresponds to the region comprising at least the 3'-end portion of the 5'-side region (that is, the entire 5'-side region, or a partial region comprising the 3'-end thereof) that serves, in the SSA reaction, as a substrate for the homologous region α in the construct of the first aspect, and the substrate region β corresponds to the region comprising at least the 5'-end portion of the 3'-side region (that is, the entire 3'-side region, or a partial region comprising the 5'-end thereof) that serves, in the SSA reaction, as a substrate for the homologous region β in the construct of the first aspect. The substrate region α, the at least one complementary region, and the substrate region β, when arranged in this order, encode an amino acid sequence of protein Y while each homologous region and the substrate therefor overlap each other, and the SSA reaction results in generation of the gene sequence Y.

In the Type II construct, the SSA reaction generates the gene sequence Y upstream of the gene sequence X, thereby operably linking the promoter region to both the gene sequence Y and the downstream gene sequence X, which results in expression of protein Y and protein X. A base sequence encoding an appropriate polypeptide such as a linker polypeptide may be placed between the substrate region β and the gene sequence X, to allow expression of protein Y and protein X in the state of a fusion protein, or a sequence that enables polycistronic expression may be used to allow polycistronic expression of protein Y and protein X. In the former case, the construct may be designed in a manner in which a protease cleavage sequence to be cleaved by a protease (for example, furin) present in the cell is included between protein Y and protein X, such that the expressed fusion protein undergoes cleavage by the protease to generate the individual proteins.

As sequences that enable polycistronic expression, various sequences including IRES sequences and 2A peptide-coding sequences are known, and any of these sequences may be used. Specifically, examples of the IERS sequences include: the IRES sequence shown in SEQ ID NO:170 and the IRES2 sequence shown SEQ ID NO: 171 (both of these are sequences derived from the internal ribosome entry site of encephalomyocarditis virus (ECMV)). Examples of the 2A peptide-coding sequences include P2A-coding sequences (SEQ ID NOs:164 and 165), E2A-coding sequences (SEQ ID NOs:166 and 167), and F2A-coding sequences (SEQ ID NOs:168 and 169). In cases where an IRES sequence is used, a stop codon needs to be added to the gene sequence Y. On the other hand, in cases where a 2A peptide-coding sequence is used, no stop codon should be added to the gene sequence Y.

The conditions and preferred examples for the gene sequence Y and the protein Y encoded thereby are the same as those for the protein A and the gene sequence encoding it in the nucleic acid construct of the first aspect. Preferably, in the Type II construct, one of the protein X and protein Y is a protein that acts to decrease the cell survival rate, and the other is a protein whose intracellular expression is detectable. Preferably, one of the gene sequence X and gene sequence Y is a sequence of a suicide gene, a DNA damage-inducing gene, or a DNA repair-inhibiting gene, and the other is a sequence of a luminescent enzyme gene, a fluorescent protein gene, a cell surface antigen gene, a secretory protein gene, or a membrane protein gene.

Typical examples of the nucleic acid construct of the second aspect include the following.
(1) A construct with a configuration in which the promoter region, the substrate region α, the substrate region β, and the gene sequence X are placed in the same nucleic acid molecule, and all of the at least one complementary region are individually contained in different nucleic acid molecules.
(2) A construct with a configuration in which the promoter region and the substrate region α are placed in one nucleic acid molecule; the substrate region β and the gene sequence X are placed in another nucleic acid molecule; and all of the at least one complementary region are individually contained in different nucleic acid molecules.
(3) A nucleic acid construct with a configuration in which the promoter region, the substrate region α, the substrate region β, the gene sequence X, and all complementary regions are placed in the same nucleic acid molecule.
   The following constructs, which are variants of (1) to (3), are also included in the nucleic acid construct of the second aspect.
(4) (Variant of (1) (3)) Part of the at least one complementary region is placed in a nucleic acid molecule together with the regions including the promoter region, and the remaining complementary region(s) is/are placed in one or more other nucleic acid molecules.
(5) (Variant of (2)) The at least one complementary region is placed in one or both of the nucleic acid molecule comprising the substrate region α and the nucleic acid molecule comprising the substrate region β.
(6) (Variant of (2)) Part of the at least one complementary region is placed in one or both of the nucleic acid molecule comprising the substrate region α and the nucleic acid molecule comprising the substrate region β, and the remaining complementary region(s) is/are placed in one or more other nucleic acid molecules.

Among the constructs of (1) in the second aspect, a construct composed of two nucleic acid molecules and comprising one complementary region has the following configuration.

A nucleic acid molecule 1 comprises the promoter region, the substrate region α, the substrate region β, and the gene sequence X.

A nucleic acid molecule 2 comprises one complementary region comprising a first homologous region and a second homologous region.

The first homologous region is a region for which the substrate region α serves as a substrate in the SSA reaction, and the homology between these regions is not less than 40% and less than 100%.

The second homologous region is a region for which the substrate region β serves as a substrate in the SSA reaction, and the homology between these regions is not less than 40% and less than 100%.

In cases of a Type I construct, the SSA reaction that occurs between each homologous region and the substrate therefor results in expression of protein X. In cases of a Type II construct, the substrate region α, the complementary region, and the substrate region β, when arranged in this order, encode an amino acid sequence of protein Y while each homologous region and the substrate therefor overlap each other, and the SSA reaction that occurs between each homologous region and the substrate therefor results in expression of protein X and protein Y.

Among the constructs of (1) in the second aspect, a construct composed of three or more nucleic acid molecules and comprising two or more complementary regions can be expressed as follows using an integer *n* that is a constant of 3 ≤ *n* ≤ 10 and an integer *m* that is a variable of 3 ≤ *m* ≤ *n. n* is preferably not more than 9, not more than 8, not more than 7, not more than 6, not more than 5, or not more than 4.

The construct is a nucleic acid construct composed of *n* nucleic acid molecules and comprising (*n*-1) complementary regions,
wherein:
a nucleic acid molecule 1 comprises the promoter region, the substrate region α, the substrate region β, and the gene sequence X;
a nucleic acid molecule 2 comprises a first complementary region comprising a first homologous region and a second homologous region; and
an *m*th nucleic acid molecule *m* comprises an (*m*-1)th complementary region comprising an (*m*-1)th substrate region and an *m*th homologous region.

The first homologous region is a region for which the substrate region α serves as a substrate in the SSA reaction, and the homology between these regions is not less than 40% and less than 100%;
an (*m*-1)th homologous region is a region for which the (*m*-1)th substrate region serves as a substrate in the SSA reaction, and the homology between these regions is not less than 40% and less than 100%; and
an *n*th homologous region contained in a nucleic acid molecule *n* is a region for which the substrate region β serves as a substrate in the SSA reaction, and the homology between these regions is not less than 40% and less than 100%.

In cases of a Type I construct, the SSA reaction that occurs between each homologous region and the substrate therefor results in expression of protein X. In cases of a Type II construct, the substrate region α, the first complementary region, the (*m*-1)th complementary region, and the substrate region β, when arranged in this order (wherein the complementary regions are arranged from smaller to larger numbers), encode an amino acid sequence of protein Y while each homologous region and the substrate therefor overlap each other, and the SSA reaction that occurs between each homologous region and the substrate therefor results in expression of protein X and protein Y.

For example, in a case where *n* = 3, a nucleic acid construct of (1) is composed of three nucleic acid molecules (nucleic acid molecules 1 to 3) and comprises two complementary regions, wherein in the configuration described above,
the nucleic acid molecule 3 comprises a second complementary region comprising a second substrate region and a third homologous region;
the second homologous region is a region for which the second substrate region serves as a substrate in the SSA reaction, and the homology between these regions is not less than 40% and less than 100%; and
the third homologous region is a region for which the substrate region β serves as a substrate in the SSA reaction, and the homology between these regions is not less than 40% and less than 100%.

In cases of a Type I construct, the SSA reaction that occurs between each homologous region and the substrate therefor results in expression of protein X. In cases of a Type II construct, the substrate region α, the first complementary region, the second complementary region, and the substrate region β, when arranged in this order, encode an amino acid sequence of protein Y while each homologous region and the substrate therefor overlap each other, and the SSA reaction that occurs between each homologous region and the substrate therefor results in expression of protein X and protein Y.

In a case where *n* = 4, a nucleic acid construct of (1) is composed of four nucleic acid molecules (nucleic acid molecules 1 to 4) and comprises three complementary regions, wherein in the configuration described above,
the nucleic acid molecule 3 comprises a second complementary region comprising a second substrate region and a third homologous region;
the nucleic acid molecule 4 comprises a third complementary region comprising a third substrate region and a fourth homologous region;
the second homologous region is a region for which the second substrate region serves as a substrate in the SSA reaction, and the homology between these regions is not less than 40% and less than 100%;
the third homologous region is a region for which the third substrate region serves as a substrate in the SSA reaction, and the homology between these regions is not less than 40% and less than 100%; and
the fourth homologous region is a region for which the substrate region β serves as a substrate in the SSA reaction, and the homology between these regions is not less than 40% and less than 100%.

In cases of a Type I construct, the SSA reaction that occurs between each homologous region and the substrate therefor results in expression of protein X. In cases of a Type II construct, the substrate region α, the first complementary region, the second complementary region, the third complementary region, and the substrate region β, when arranged in this order, encode an amino acid sequence of protein Y while each homologous region and the substrate therefor overlap each other, and the SSA reaction that occurs between each homologous region and the substrate therefor results in expression of protein X and protein Y.

In a construct of (1) in the second aspect, the nucleic acid molecule 1 may be a circular nucleic acid molecule comprising, downstream of the promoter region, the substrate region α, the substrate region β, and the gene sequence X in this order, or may be a linear nucleic acid molecule comprising, from upstream to downstream, the substrate region β, the gene sequence X, the promoter region, and the substrate region α in this order. In cases where a poly-A addition signal is contained, the nucleic acid molecule 1 may be a circular nucleic acid molecule comprising, downstream of the promoter region, the substrate region α, the substrate region β, the gene sequence X, and the poly-A addition signal in this order, or may be a linear nucleic acid molecule comprising, from upstream to downstream, the substrate region β, the gene sequence X, the poly-A addition signal, the promoter region, and the substrate region α in this order.

In cases where the nucleic acid molecule 1 is a circular molecule, it is usually linearized by cleavage between the substrate region α and the substrate region β before the introduction into the cell. Therefore, the nucleic acid molecule 1 preferably has a cleavage site between the substrate region α and the substrate region β. Examples of the cleavage site are the same as those for the constructs of the first aspect.

All of the nucleic acid molecules comprising a complementary region, other than the nucleic acid molecule 1, are preferably linear molecules from the viewpoint of simplicity in both the production and the use.

Among the constructs of (2) in the second aspect, a construct composed of three nucleic acid molecules and comprising one complementary region has the following configuration.

A nucleic acid molecule 1-1 comprises the promoter region and the substrate region α.

A nucleic acid molecule 1-2 comprises the substrate region β and the gene sequence X.

The first homologous region is a region for which the substrate region α serves as a substrate in the SSA reaction, and the homology between these regions is not less than 40% and less than 100%.

The second homologous region is a region for which the substrate region β serves as a substrate in the SSA reaction, and the homology between these regions is not less than 40% and less than 100%.

In cases of a Type I construct, the SSA reaction that occurs between each homologous region and the substrate therefor results in expression of protein X. In cases of a Type II construct, the substrate region α, the complementary region, and the substrate region β, when arranged in this order, encode an amino acid sequence of protein Y while each homologous region and the substrate therefor overlap each other, and the SSA reaction that occurs between each homologous region and the substrate therefor results in expression of protein X and protein Y.

Among the constructs of (2) in the second aspect, a construct composed of four or more nucleic acid molecules and comprising two or more complementary regions can be expressed as follows using an integer *n* that is a constant of 3 ≤ *n* ≤ 10 and an integer *m* that is a variable of 3 ≤ *m* ≤ *n. n* is preferably not more than 9, not more than 8, not more than 7, not more than 6, not more than 5, or not more than 4. The construct is a nucleic acid construct composed of (*n*+1) nucleic acid molecules and comprising (*n*-1) complementary regions, wherein:
a nucleic acid molecule 1-1 comprises the promoter region and the substrate region α;
a nucleic acid molecule 1-2 comprises the substrate region β and the gene sequence X;
a nucleic acid molecule 2 comprises a first complementary region comprising a first homologous region and a second homologous region; and
an *m*th nucleic acid molecule *m* comprises an (*m*-1)th complementary region comprising an (*m*-1)th substrate region and an mth homologous region.

The first homologous region is a region for which the substrate region α serves as a substrate in the SSA reaction, and the homology between these regions is not less than 40% and less than 100%;
an (*m*-1)th homologous region is a region for which the (m-1)th substrate region serves as a substrate in the SSA reaction, and the homology between these regions is not less than 40% and less than 100%; and
an *n*th homologous region contained in a nucleic acid molecule n is a region for which the substrate region β serves as a substrate in the SSA reaction, and the homology between these regions is not less than 40% and less than 100%.

In cases of a Type I construct, the SSA reaction that occurs between each homologous region and the substrate therefor results in expression of protein X. In cases of a Type II construct, the substrate region α, the first complementary region, the *(m*-1)th complementary region, and the substrate region β, when arranged in this order (wherein the complementary regions are arranged from smaller to larger numbers), encode an amino acid sequence of protein Y while each homologous region and the substrate therefor overlap each other, and the SSA reaction that occurs between each homologous region and the substrate therefor results in expression of protein X and protein Y.

For example, in a case where *n* = 3, a nucleic acid construct of (2) is composed of four nucleic acid molecules (nucleic acid molecules 1-1, 1-2, 2, and 3) and comprises two complementary regions, wherein in the configuration described above,
the nucleic acid molecule 3 comprises a second complementary region comprising a second substrate region and a third homologous region;
the second homologous region is a region for which the second substrate region serves as a substrate in the SSA reaction, and the homology between these regions is not less than 40% and less than 100%; and
the third homologous region is a region for which the substrate region β serves as a substrate in the SSA reaction, and the homology between these regions is not less than 40% and less than 100%.

In cases of a Type I construct, the SSA reaction that occurs between each homologous region and the substrate therefor results in expression of protein X. In cases of a Type II construct, the substrate region α, the first complementary region, the second complementary region, and the substrate region β, when arranged in this order, encode an amino acid sequence of protein Y while each homologous region and the substrate therefor overlap each other, and the SSA reaction that occurs between each homologous region and the substrate therefor results in expression of protein X and protein Y.

In a case where n = 4, a nucleic acid construct of (2) is composed of five nucleic acid molecules (nucleic acid molecules 1-1, 1-2, 2, 3, and 4) and comprises three complementary regions, wherein in the configuration described above,
the nucleic acid molecule 3 comprises a second complementary region comprising a second substrate region and a third homologous region;
the nucleic acid molecule 4 comprises a third complementary region comprising a third substrate region and a fourth homologous region;
the second homologous region is a region for which the second substrate region serves as a substrate in the SSA reaction, and the homology between these regions is not less than 40% and less than 100%; and
the third homologous region is a region for which the third substrate region serves as a substrate in the SSA reaction, and the homology between these regions is not less than 40% and less than 100%; and
the fourth homologous region is a region for which the substrate region β serves as a substrate in the SSA reaction, and the homology between these regions is not less than 40% and less than 100%.

In cases of a Type I construct, the SSA reaction that occurs between each homologous region and the substrate therefor results in expression of protein X. In cases of a Type II construct, the substrate region α, the first complementary region, the second complementary region, the third complementary region, and the substrate region β, when arranged in this order, encode an amino acid sequence of protein Y while each homologous region and the substrate therefor overlap each other, and the SSA reaction that occurs between each homologous region and the substrate therefor results in expression of protein X and protein Y.

From the viewpoint of simplicity in both the production and the use, all nucleic acid molecules constituting the nucleic acid construct of (2) in the second aspect are preferably prepared as linear nucleic acid molecules. However, part or all of the nucleic acid molecules may be prepared as circular molecules. In cases where the nucleic acid construct of (2) comprises a poly-A addition signal, the nucleic acid molecule 1-2, which contains the gene sequence X, comprises the poly-A addition signal downstream of the gene sequence X.

The nucleic acid constructs of (4) to (6), which are variants of (1) to (3), represent configurations applicable to constructs having two or more complementary regions. Regarding the constructs of (4) to (6) in the first aspect, specific configurations have been illustrated for constructs having up to four complementary regions. The corresponding specific examples for (4) to (6) of the second aspect are understood by replacing "5'-side region" with "substrate region α" and "3'-side region" with "substrate region β", respectively, in the descriptions provided for the first aspect.

### [Uses of Nucleic Acid Construct of Second Aspect]

A type I construct, in which the sequence generated by the SSA reaction is a non-coding sequence, can be used in the same manner as a construct of the first aspect. Various uses for this construct can be understood by replacing "protein A" with "protein X" in the description of [Uses of Nucleic Acid Construct of First Aspect].

A type II construct, in which the sequence generated by the SSA reaction is a coding sequence, and in which the SSA reaction results in expression of the two kinds of proteins, that is, proteins X and Y, can be used in the same manner as a construct of the first aspect.

Further, in a type II construct, if one of the proteins X and Y is a protein that acts to decrease the cell survival rate and the other is a protein whose intracellular expression is detectable, detection and killing of mismatch repair-deficient cells can be carried out with a single construct. Such a dual-type construct can be used as an agent that detects and treats mismatch repair-deficient cancer. In the following Examples B and C, the gene sequence Y which is generated by the SSA reaction is a gene sequence encoding a protein whose intracellular expression is detectable, and the gene sequence X which is originally contained in the nucleic acid construct is a gene sequence encoding a protein that acts to decrease the cell survival rate. However, the reverse configuration may also be employed. In cases where a dual-type construct is used, the step of detecting a mismatch repair-deficient cancer may be carried out in the same manner as the detection of a mismatch repair-deficient cancer using a nucleic acid construct of the first aspect.

### EXAMPLES

The present invention is described below more concretely by way of Examples. However, the present invention is not limited to the following Examples. In the following Examples, the size of each DNA fragment may be expressed as the size excluding the stop codon added to the 3'-end.

### Example A-I: Divided-Type DR-Nluc-Homeo (SceNluc + iNluc) (Two-Molecule, Two-SSA-Reaction Type) (Fig. 1)

Constructs in which a normal *Nluc* gene is formed by single-strand annealing (SSA) at two sites (two times of SSA) were prepared, and evaluated for their luciferase activities.

### A-I-1. Preparation of Vectors

A 513-bp SceNluc fragment (SEQ ID NO:9; a DNA having a structure in which the region of positions 223 to 243 in the *Nluc* gene (SEQ ID NO:7) was replaced with "stop codon (TGA) + I-SceI recognition sequence", and having a stop codon TAA added to the 3'-end) to be used for pCMV-SceNluc and a 459-bp iNluc fragment (SEQ ID NO: 12; the region of positions 16 to 474 in the *Nluc* gene (SEQ ID NO:7), having a stop codon TAA added to the 3'-end) to be used for pUC-iNluc were obtained by PCR amplification using pNL1.1 [Nluc] Vector (Promega, Madison, WI, USA) as a template. As a polymerase for the PCR, PrimeSTAR HS (trade name) DNA Polymerase (Takara Bio Inc.) or Tks Gflex (trade name) DNA Polymerase (Takara Bio Inc.) was used. The primers used are shown in Table 1 below.

The SceNluc fragment was prepared by separately amplifying two fragments: a 5'SceNluc fragment and a 3'SceNluc fragment. In the amplification of the 5'SceNluc fragment, Nluc-Fw (SEQ ID NO:1) and Sce-5'Nluc-Rv (SEQ ID NO:2) were used to amplify a DNA fragment having a structure in which a sequence for In-Fusion reaction was added to the 5'-end of the 5'SceNluc fragment, and in which "stop codon (TGA) + I-SceI recognition sequence" was added to the 3'-end of the 5' SceNluc fragment. In the amplification of the 3' SceNluc fragment, Sce-3'Nluc-Fw (SEQ ID NO:3) and Nluc-Rv (SEQ ID NO:4) were used to amplify a DNA fragment having a structure in which "stop codon (TGA) + I-SceI recognition sequence" was added to the 5'-end of the 3' SceNluc fragment, and in which a stop codon (TAA) and a sequence for In-Fusion reaction were added to the 3'-end of the 3' SceNluc fragment.

In the amplification of the iNluc fragment, iNluc-Fw (SEQ ID NO:5) and iNluc-Rv (SEQ ID NO:6) were used to amplify a DNA fragment having a structure in which a stop codon (TGA) was linked to the 3'-end of the iNluc fragment, and in which sequences for In-Fusion reaction were added to both ends.

Subsequently, pIRES (Takara Bio Inc., Fig. 2) was digested with NheI and XbaI to remove the IRES region, and a fragment containing the CMV promoter and the poly-A addition signal was recovered. Using an In-Fusion HD Cloning Kit (Takara Bio Inc.), the 5'SceNluc fragment and the 3'SceNluc fragment were inserted downstream of the CMV promoter in the recovered fragment, to obtain pCMV-SceNluc, in which [CMV promoter] - [SceNluc fragment] - [poly-A addition signal] were linked together. In the reading frame of the SceNluc fragment (SEQ ID NO:9), there are the stop codon TGA introduced upstream of the I-SceI recognition sequence, and the two stop codons TAG and TAA inside the I-SceI recognition sequence. The amino acid sequences encoded by positions 1 to 225 (the region up to the first stop codon) and positions 235 to 516 (the region located downstream of the third stop codon) of the SceNluc fragment are shown in SEQ ID NOs: 10 and 11, respectively (it should be noted that expression of a polypeptide consisting of the latter sequence does not occur).

pUC-iNluc was obtained by digesting pUC19 (Takara Bio Inc., Fig. 3) at SmaI in the multicloning site, and inserting the iNluc fragment thereto using a DNA Ligation Kit <Mighty Mix> (Takara Bio Inc.).

**[Table 1]**

| Primer name | Sequence (5'→3') | SEQ ID NO: |
|---|---|---|
| Nluc-Fw | TAATACGACTCACTATAGGCTAG | 1 |
| Sce-5'Nluc-Rv | ATTACCCTGTTATCCCTA**TCA**CTGGCCCATTTGGTCGCCGCTC | 2 |
| Sce-3'Nluc-Fw | **TGA**TAGGGATAACAGGGTAATGTGTACCCTGTGGATGATCATCAC | 3 |
| Nluc-Rv | CCGCCCCGACTCTAGAGTCGCGG | 4 |
| iNluc-Fw | AAATCGATAAGGATCGCTAGCATGAAGATTTCGTTGGGGACTGG | 5 |
| iNluc-Rv | CACCATACGCGGATCCTTA**TCA**GTTGATGGTTACTCGGAACAGC | 6 |

| | | |
|---|---|---|
| Single-underline: I-Scel recognition sequence Double-underline: A site that anneals to the target gene sequence (Nluc-Fw and Nluc-Rv target vector sequences located upstream and downstream of the *Nluc* gene in pNL1.1 [Nluc] Vector, respectively.) Bold: Sequence for introduction of a stop codon (TGA) | | |

Subsequently, for preparation of a vector having decreased homology between homologous sequences (between each homologous region - substrate), artificial gene synthesis was performed to prepare a DNA fragment in which silent mutations were introduced in the regions of positions 1 to 207 and positions 229 to 459 of the iNluc sequence (459 bp, SEQ ID NO:12). The silent mutations were introduced such that the homology to SceNluc was 99% (4 sites; 5'-side 2/207, 3'-side 2/231), 98% (8 sites; 5'-side 4/207, 3'-side 4/231), 97% (13 sites; 5'-side 6/207, 3'-side 7/231), 96% (17 sites; 5'-side 8/207, 3'-side 9/231), 95% (23 sites; 5'-side 11/207, 3'-side 12/231), 94% (26 sites; 5'-side 12/207, 3'-side 14/231), 90% (44 sites; 5'-side 21/207, 3'-side 23/231), 80% (94 sites; 5'-side 44/207, 3'-side 50/231), or 70% (140 sites; 5'-side 66/207, 3'-side 74/231), and such that the silent mutations were dispersed as uniformly as possible throughout the sequence (Fig. 4-1 to Fig. 4-4). The sequences of the iNluc fragments having homologies of 95%, 90%, 80% and 70% (Fig. 4-1, Fig. 4-2) are shown in SEQ ID NOs:14 to 17, and the sequences of the iNluc fragments having homologies of 99%, 98%, 97%, 96%, and 94% (Fig. 4-3, Fig. 4-4) are shown in SEQ ID NOs:18 to 22, respectively. Vectors in which each of the nine iNluc fragments having different mutations prepared by the artificial gene synthesis had been cloned were digested with BamHI (Takara Bio Inc.) and EcoRI (Takara Bio Inc.), to cut out each fragment containing each mutated iNluc. Subsequently, pUC19 (Takara Bio Inc.) was digested with BamHI and EcoRI, and each mutated iNluc fragment was inserted therein using a DNA Ligation Kit <Mighty Mix> (Takara Bio Inc.), to obtain each of pUC-iNluc (homology, 99%), pUC-iNluc (homology, 98%), pUC-iNluc (homology, 97%), pUC-iNluc (homology, 96%), pUC-iNluc (homology, 95%), pUC-iNluc (homology, 94%), pUC-iNluc (homology, 90%), pUC-iNluc (homology, 80%), and pUC-iNluc (homology, 70%).

The pCMV-SceNluc was purified using a Qiagen Plasmid Plus Midi Kit (Qiagen K.K.), and linearized by cleavage with I-SceI (New England Biolabs, Ipswich, MA, USA) before use in transfection. The pUC19 and the pUC-iNluc were purified using a Qiagen Plasmid Plus Midi Kit (Qiagen K.K.), and then linearized by cleavage with AhdI, or with BamHI and EcoRI, followed by purification of the fragment containing iNluc using the Wizard SV Gel and PCR Clean-Up System (Promega) before use. Cleavage of pUC-iNluc with AhdI results in a DNA containing additional sequences of not less than 1 kb at both ends of the iNluc fragment (Fig. 1C), while cleavage with BamHI and EcoRI results in a DNA containing no additional sequence (Fig. 1D).

### A-I-2. Cells and Gene Transfection, and Luciferase Assay

The human pre-B cell line Nalm-6 (cancer cells deficient in the mismatch repair mechanism) and cells derived therefrom were cultured at 37°C in a 5% CO₂ incubator (So et al. (2004) Genetic interactions between BLM and DNA ligase IV in human cells. J. Biol. Chem. 279: 55433-55442).

Msh2 revertant cells (Msh2+ cells) were obtained by knocking in MSH2 cDNA into the human pre-B cell line Nalm-6 (cancer cells deficient in the mismatch repair mechanism (MMR); Msh2-). More specifically, a method in a past report (Restoration of mismatch repair functions in human cell line Nalm-6, which has high efficiency for gene targeting. Suzuki T, Ukai A, Honma M, Adachi N, Nohmi T. PLoS One. 2013 Apr 15;8(4):e61189. doi: 10.1371/journal.pone.0061189.) was employed. Msh2+ cells have the mismatch repair mechanism due to recovery by knock-in of MSH2. A construct showing a large difference in the luciferase activity or the cytocidal effect (that is, the recombination efficiency) between Msh2- cells and Msh2+ cells can be evaluated as a construct that responds sharply to the presence or absence of mismatch repair.

Transfection of the Nalm-6 cells (the wild-type line and Msh2 revertant cells thereof) was carried out by the electroporation method according to a past report (Saito et al. (2017) Dual loss of human POLQ and LIG4 abolishes random integration. Nat. Commun. 8: 16112).

The human colorectal-derived cancer cell line HCT116 (Horizon Discovery, Cambridge, UK) and MLH1 revertant cells thereof (MLH1+ HCT116 cells) (Horizon Discovery) were cultured at 37°C in a 5% CO₂ incubator. As a medium, Dulbecco's modified Eagle medium (Nissui Pharmaceutical Co., Ltd., Tokyo, Japan) supplemented with calf serum (Global Life Science Technologies Japan, Tokyo, Japan; 50 ml of calf serum was added per 500 ml of Eagle MEM) and L(+)-glutamine (Fujifilm Wako Pure Chemical Corporation, Osaka, Japan; added to a final concentration of 2 mM) was used. Transfection was carried out using jetPEI (Polyplus-transfection, Illkirch, France) according to a protocol provided by the manufacturer.

A luciferase assay of the Nalm-6 cells (the wild-type line and Msh2 revertant cells thereof) was carried out as follows. With each construct (1 µg), 2×10⁶ Nalm-6 cells were transfected. The cells after the gene transfection were plated on a 12-well dish at 5×10⁵ cells/ml, and the luciferase activity (RLU value) was measured immediately after the gene transfection, 4 hours later, and 24 hours later using the Nano-Glo Luciferase Assay System (Promega).

A luciferase assay of the HCT116 cells (the wild-type line and MLH1 revertant cells thereof) was carried out as follows. On a 24-well dish, 5×10⁴ cells were plated and cultured overnight, followed by transfecting the cells with each construct (1 µg). Immediately after the gene transfection, 4 hours later, and 24 hours later, the luciferase activity (RLU value) was measured using the Nano-Glo Luciferase Assay System (Promega).

### A-I-3. Results

The constructs prepared in the present Example were designed such that a normal *Nluc* gene is generated when homologous recombination (HR) occurs between the SceNluc fragment and the iNluc fragment, or when single-strand annealing (SSA) occurs at two sites, one in the 5'-terminal portion and one in the 3'-terminal portion of the respective fragments. In cases where the homologous regions have sufficiently high homology, HR occurs. However, in cases where the homologous regions have decreased homology, HR does not occur, and a normal *Nluc* gene is generated only when the SSA has occurred. At this time, the SSA reaction is inhibited by the MMR factor, so that the presence or absence of the MMR factor is thought to cause a difference in the expression level of the *Nluc* gene. An SSA construct previously developed by the present inventor (WO 2021/162121 A1; which may be hereinafter referred to as conventional construct) had a structure in which one time of SSA reaction results in formation of a normal *Nluc* gene. On the other hand, the construct of the present Example requires occurrence of SSA at two sites (two times of SSA) for formation of a normal *Nluc* gene. Therefore, compared to the conventional construct, the construct of the present Example can be expected to show a larger difference in the expression level of the *Nluc* gene depending on the presence or absence of the MMR factor.

Fig. 5, Fig. 6-1, and Fig. 6-2 show examples of the results of luciferase assays of Nalm-6 cells and MSH2 revertant cells thereof into which two-molecule, two-SSA-reaction type constructs have been introduced. Fig. 5 shows the results obtained by co-transfecting, with pCMV-SceNluc, the iNluc fragments without the additional sequences, and measuring the luciferase activity over time. The constructs having homologous regions with decreased homologies of 95% to 70% exhibited high Luc activity under the MSH2-deficient condition (in the WT Nalm-6 cells deficient in MMR). In Fig. 6-1, A shows the luciferase activity 4 hours after the gene transfection, and B shows the luciferase activity 24 hours after the gene transfection. In the constructs having homologous regions with decreased homologies of 98% or less, differences in the luciferase activity were clearly detected depending on the presence or absence of MSH2 deficiency (MMR deficiency). The difference in the luciferase activity was more remarkable 24 hours after the gene transfection than 4 hours after the gene transfection. In particular, a 170-fold difference in the luciferase activity occurred in the construct having homologous regions with a homology of 90%, and a 62-fold difference in the luciferase activity occurred in the construct having a homology of 80%. Fig. 6-2 shows a graph comparing the luciferase activity 24 hours after the gene transfection, between the iNluc fragments with additional sequences (pUC-iNluc / AhdI, left side in the graph) and without the additional sequences (pUC-iNluc / BamHI + EcoRI, right side in the graph). The iNluc fragments without the additional sequences showed larger differences in the luciferase activity depending on the presence or absence of MSH2 deficiency (MMR deficiency). In particular, the constructs having homologous regions with homologies of 80% and 90% showed not less than 200-fold differences, indicating very large differences in the luciferase activity.

In the case of a substrate having a homology of 100% to the homologous region, recombination by the HR reaction is predominant over recombination by the SSA reaction, so that the difference in the luciferase activity depending on the presence or absence of MSH2 deficiency (MMR deficiency) can be hardly observed. On the other hand, in the case of a substrate having decreased homology, the formation of the *Nluc* gene due to the SSA reaction proceeds under the MSH2 deficiency (MMR deficiency). This is thought to be the reason why the difference in the luciferase activity occurred depending on the presence or absence of MSH2 deficiency (MMR deficiency).

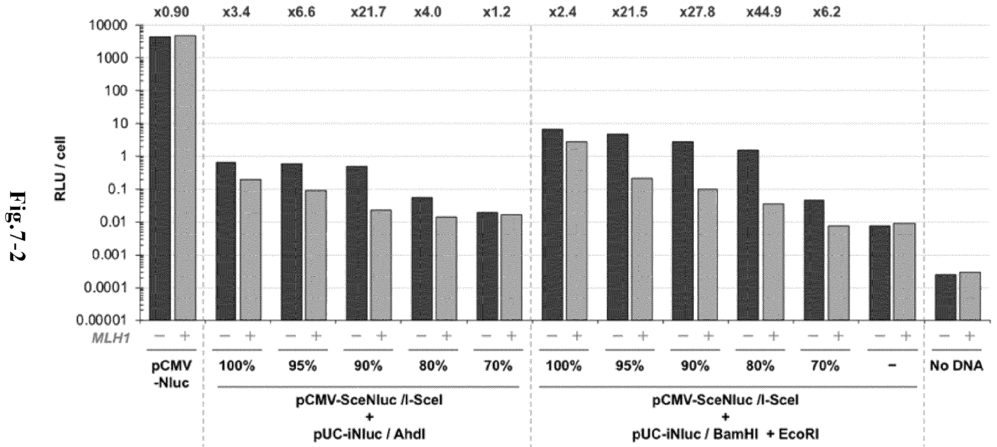

Fig. 7-1 shows the results of measurement of the luciferase activity 4 hours (A) or 24 hours (B) after introduction of two-molecule, two-SSA-reaction type constructs into HCT116, which is a cancer cell line with MMR deficiency due to MLH1 deficiency, and into MLH1 revertant cells thereof. Fig. 7-2 shows a graph comparing the luciferase activity 24 hours after the gene transfection, between the iNluc fragments with additional sequences (pUC-iNluc / AhdI, left side in the graph) and without the additional sequences (pUC-iNluc / BamHI + EcoRI, right side in the graph). It could be confirmed that, depending not only on the presence or absence of mismatch repair deficiency caused by MSH2, but also on the presence or absence of mismatch repair deficiency caused by MLH1, the difference in the gene expression (luciferase activity) increases when the homology of the homologous regions is decreased. Further, also in the MLH1-deficient system, the iNluc fragments without the long additional sequences showed larger differences in the luciferase activity depending on the presence or absence of the deficiency, although the differences were not as remarkable as those in the MSH2-deficient system.

This result suggests that the construct of the present invention is not affected by the cause of mismatch repair deficiency.

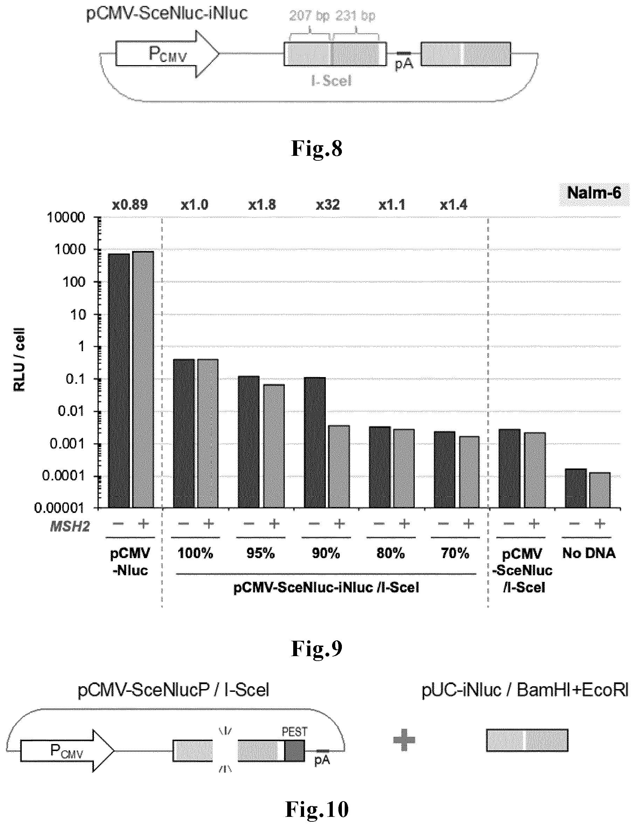

### Example A-II: Integrated-Type DR-Nluc-Homeo (Single-Molecule, Two-SSA-Reaction Type) (Fig. 8)

pCMV-SceNluc-iNluc, which is a two-SSA-reaction type construct comprising the *SceNluc* gene and the *iNluc* gene in one vector, was constructed, and subjected to evaluation of the luciferase activity.

### A-II-1. Preparation of Vectors

pCMV-SceNluc, prepared in A-I-1, was digested with BamHI, and the iNluc fragment was inserted downstream of poly-A using an In-Fusion HD Cloning Kit (Takara Bio Inc.), to obtain pCMV-SceNluc-iNluc, in which [CMV promoter] - [SceNluc fragment] - [poly-A addition signal] - [iNluc fragment] were linked together.

Subsequently, in order to construct pCMV-SceNluc-iNluc in which the *SceNluc* gene and each mutated *iNluc* gene are contained in one vector, each vector in which the mutated *iNluc* gene had been cloned was digested with BamHI and AhdI (New England Biolabs) to cut out a fragment containing the mutated iNluc. Subsequently, pCMV-SceNluc was digested with BamHI and AhdI, and a fragment containing the *SceNluc* gene was cut out. Thereafter, using a DNA Ligation Kit <Mighty Mix> (Takara Bio Inc.), each mutated iNluc fragment was inserted downstream of the poly-A addition signal, to obtain pCMV-SceNluc-iNluc (95% homology), pCMV-SceNluc-iNluc (90% homology), pCMV-SceNluc-iNluc (80% homology), and pCMV-SceNluc-iNluc (70% homology), in each of which [CMV promoter] - [SceNluc fragment] - [poly-A addition signal] - [each mutated iNluc fragment] were linked together.

### A-II-2. Cells and Gene Transfection, and Luciferase Assay

The same operation as described in A-I-2. was carried out.

### A-II-3. Results

Fig. 9 shows the luciferase activities of Nalm-6 cells and MSH2 revertant cells thereof 24 hours after the introduction of the integrated constructs. Although the difference in the luciferase activity depending on the presence or absence of MSH2 deficiency (MMR deficiency) was rather small compared to the two-molecule-type constructs, the integrated types were also found to be capable of detecting the presence or absence of the MMR deficiency.

### Example A-III: Divided-Type NlucP-Homeo (SceNlucP + iNluc) (Two-Molecule, Two-SSA-Reaction Type) (Fig. 10)

In order to prepare a construct that sensitively respond to a change in the expression level of a normal *Nluc* gene, a two-molecule-type expression vector of the *NlucP* gene was constructed. This *NlucP* gene was prepared by fusing the *Nluc* gene with a PEST sequence (protein degradation signal) to decrease stability of the Nluc protein (that is, to make the Nluc protein more susceptible to degradation). As the protein degradation signal, a PEST sequence derived from mouse ornithine decarboxylase with codon optimization for use in human cells (hPEST, SEQ ID NO:27) was used. The hPEST sequence was prepared by artificial gene synthesis (phPEST). The iNluc was prepared by linearizing the pUC-iNluc having a homology of 100%, 98%, 96%, 94% or 90% prepared in Example A-I, by cleavage with BamHI and EcoRI.

### A-III-1. Preparation of Vectors

A 513-bp Nluc fragment used for pCMV-NlucP was obtained by PCR amplification using pNL1.1 [Nluc] Vector (Promega, Madison, WI, USA) as a template, and a 120-bp hPEST fragment (SEQ ID NO:26, having a stop codon added to the 3'-end) was obtained by PCR amplification using hPEST as a template. As a polymerase for the PCR, PrimeSTAR HS (trade name) DNA Polymerase (Takara Bio Inc.) was used. The primers used are shown in Table 2 below. In the amplification of the Nluc fragment, Nluc-Fw (SEQ ID NO: 1) and NlucP-Rv (SEQ ID NO:23) were used to amplify a DNA fragment having a structure in which sequences for In-Fusion reaction were added to both ends of the Nluc fragment. In the amplification of the hPEST fragment, hPEST-Fw (SEQ ID NO:24) and hPEST-Rv (SEQ ID NO:25) were used to amplify a DNA fragment having a structure in which a stop codon (TAA) was linked to the 3'-end of the hPEST fragment, and in which sequences for In-Fusion reaction were added to both ends. Subsequently, pIRES (Takara Bio Inc., Fig. 2) was digested with NheI and XbaI to remove the IRES region, and a fragment containing the CMV promoter and the poly-A addition signal was recovered. Using an In-Fusion HD Cloning Kit (Takara Bio Inc.), the Nluc fragment and the hPEST fragment were inserted downstream of the CMV promoter in the recovered fragment, to obtain pCMV-NlucP, in which [CMV promoter] - [NlucP fragment] - [hPEST fragment] - [poly-A addition signal] were linked together. The base sequence of the NlucP fragment (*NlucP* gene), and the amino acid sequence of the NlucP protein encoded thereby, are shown in SEQ ID NOs:28 and 29.

Subsequently, in order to construct a vector (pCMV-SceNlucP) that expresses the *SceNlucP* gene (SEQ ID NO:30), which had been prepared by fusing the *SceNluc* gene (SEQ ID NO:9, see A-I-1.) with the hPEST sequence, the pCMV-SceNluc prepared in A-I-1. was digested with PpuMI and XbaI to remove the 3'-side of SceNluc including the stop codon, and a fragment containing the CMV promoter, the 5'-side of SceNluc, and the poly-A addition signal was recovered. Subsequently, pCMV-NlucP was digested with PpuMI and XbaI, and a fragment containing the 3'-side of Nluc and the hPEST sequence was recovered. The two recovered sequences were linked to each other using a DNA Ligation Kit <Mighty Mix> (Takara Bio Inc.), to obtain pCMV-SceNlucP, in which [CMV promoter] - [SceNlucP fragment] - [hPEST fragment] - [poly-A addition signal] were linked together. In the reading frame of the *SceNlucP* gene (SEQ ID NO:30), there are the stop codon TGA introduced upstream of the I-SceI recognition sequence, and the two stop codons TAG and TAA inside the I-SceI recognition sequence. The amino acid sequences encoded by positions 1 to 225 (the region up to the first stop codon) and positions 235 to 639 (the region located downstream of the third stop codon) of the *SceNlucP* gene are shown in SEQ ID NOs: 31 and 32, respectively (it should be noted that expression of a polypeptide consisting of the latter sequence does not occur).

pCMV-NlucP and pCMV-SceNlucP were purified using a Qiagen Plasmid Plus Midi Kit (Qiagen K.K.). Thereafter, pCMV-NlucP was used as it is in the circular form in transfection, while pCMV-SceNlucP was linearized by cleavage with I-SceI (New England Biolabs, Ipswich, MA, USA) before use in transfection.

**[Table 2]**

| Primer name | Sequence (5'→3') | SEQ ID NO |
|---|---|---|
| Nluc-Fw | TAATACGACTCACTATAGGCTAG | 1 |
| NlucP-Rv | GCCGTGAGAATTCGCCAGAATGCGTTCGCACAG | 23 |
| hPEST-Fw | TTCTGGCGAATTCTCACGGCTTTCCGCCTG | 24 |
| hPEST-Rv | CGGCCGCCCCGACTCTAGA**TTA**GACGTTGATGCGAGCTGAAG | 25 |

| | | |
|---|---|---|
| Single-underline: A site that anneals to the target gene sequence (Nluc-Fw targets a vector sequence located upstream of the *Nluc* gene in pNL1.1[Nluc] Vector.) Bold: Sequence for introduction of a stop codon (TAA) | | |

### A-III-2. Cells and Gene Transfection, and Luciferase Assay

The same operation as described in A-I-2. was carried out.

### A-III-3. Results

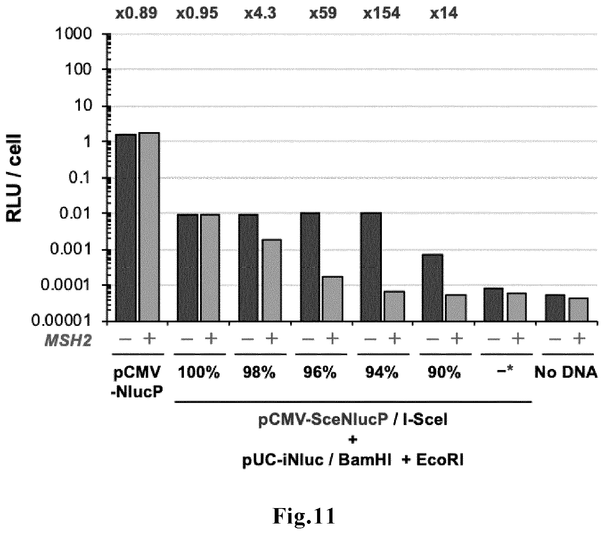

Fig. 11 shows the results of luciferase assays of Nalm-6 cells and MSH2 revertant cells thereof into which the prepared constructs have been introduced (24 hours after the gene transfection). The constructs having homologous regions with homologies of 98% or less exhibited high luciferase activity under the MSH2-deficient condition (in the WT Nalm-6 cells deficient in MMR). In particular, depending on the presence or absence of MSH2, a 154-fold difference occurred in the construct having homologous regions with a homology of 94%.

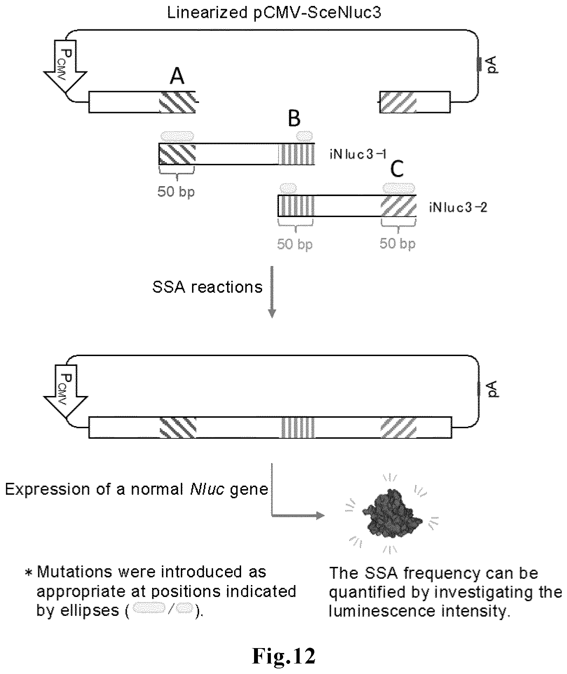

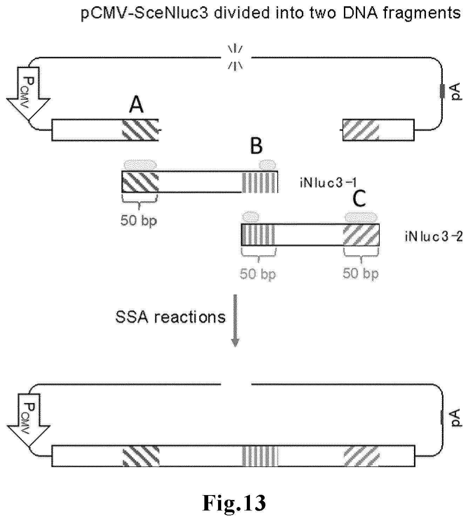

### Example A-IV: Divided-Type SA-Nluc-v3 (Three-Molecule & Four-Molecule, Three-SSA-Reaction Type) (Fig. 12, Fig. 13)

Divided-type constructs in which a normal *Nluc* gene is formed by SSA at three sites (three times of SSA) were prepared, and evaluated for their luciferase activities.

### A-IV-1-1. Preparation of Vectors (Three-Molecule, Three-SSA-Reaction Type) (Fig. 12)

A 280-bp SceNluc3 fragment (SEQ ID NO:47; a DNA having a structure in which the region of positions 151 to 416 in the *Nluc* gene sequence was replaced with "stop codon (TGA) + I-SceI recognition sequence", and having a stop codon added to the 3'-end) to be used for pCMV-SceNluc3, a 220-bp iNluc3-1-100 fragment (the region of positions 101 to 320 in the *Nluc* gene sequence; SEQ ID NO:48), and a 196-bp iNluc3-2-100 fragment (the region of positions 271 to 466 in the *Nluc* gene sequence; SEQ ID NO:49) were obtained by PCR amplification using pNL1.1[Nluc] Vector (Promega, Madison, WI, USA) as a template. As a polymerase for the PCR, PrimeSTAR HS (trade name) DNA Polymerase (Takara Bio Inc.) was used. The primers used are shown in Table 3 below. The SceNluc3 fragment was prepared by separately amplifying two fragments: a 5'SceNluc3 fragment and a 3'SceNluc3 fragment. In the amplification of the 5'SceNluc3 fragment, Nluc-Fw (SEQ ID NO:1) and 5'SceNluc3-Rv (SEQ ID NO:33) were used to amplify a DNA fragment having a structure in which a sequence for In-Fusion reaction was added to the 5'-end of the 5' SceNluc3 fragment, and in which "stop codon (TGA) + I-SceI recognition sequence" was added to the 3'-end of the 5' SceNluc3 fragment. In the amplification of the 3' SceNluc3 fragment, 3' SceNluc3-Fw (SEQ ID NO:34) and Nluc-Rv (SEQ ID NO:4) were used to amplify a DNA fragment having a structure in which "I-SceI recognition sequence" was added to the 5'-end of the 3' SceNluc3 fragment, and in which a stop codon (TAA) and a sequence for In-Fusion reaction were added to the 3'-end of the 3' SceNluc3 fragment. For the iNluc3-1-100 fragment (SEQ ID NO:48), iNluc3-1-100-Fw (SEQ ID NO:35) and iNluc3-1-100-Rv (SEQ ID NO:36) were used. The 50-bp base sequence in the 5'-end region of the iNluc3-1-100 fragment amplified by this PCR has a homology of 100% to the 50 bp in the 3'-end region of pCMV-SceNluc3 digested with I-SceI, and the 50-bp base sequence in the 3'-end region of this fragment has a homology of 100% to the base sequence in the 5'-end region of the iNluc3-2-100 fragment. For the iNluc3-2-100 fragment (SEQ ID NO:49), iNluc3-2-100-Fw (SEQ ID NO:37) and iNluc3-2-100-Rv (SEQ ID NO:38) were used. The 50-bp base sequence in the 5'-end region of the iNluc3-2-100 fragment amplified by this PCR has a homology of 100% to the base sequence in the 3'-end region of the iNluc3-1-100 fragment, and the 50-bp base sequence in the 3'-end region of the iNluc3-2-100 fragment has a homology of 100% to the 50 bp in the 5'-end region of pCMV-SceNluc3 digested with I-SceI.

Subsequently, pIRES (Takara Bio Inc., Fig. 2) was digested with NheI and XbaI to remove the IRES region, and a fragment containing the CMV promoter and the poly-A addition signal was recovered. Using an In-Fusion HD Cloning Kit (Takara Bio Inc.), the 5'SceNluc3 fragment and the 3' SceNluc3 fragment were inserted downstream of the CMV promoter in the recovered fragment, to obtain pCMV-SceNluc3, in which [CMV promoter] - [SceNluc3 fragment] - [poly-A addition signal] were linked together.

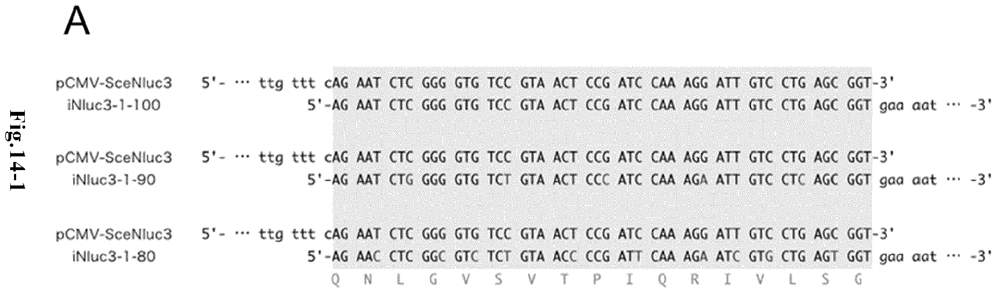

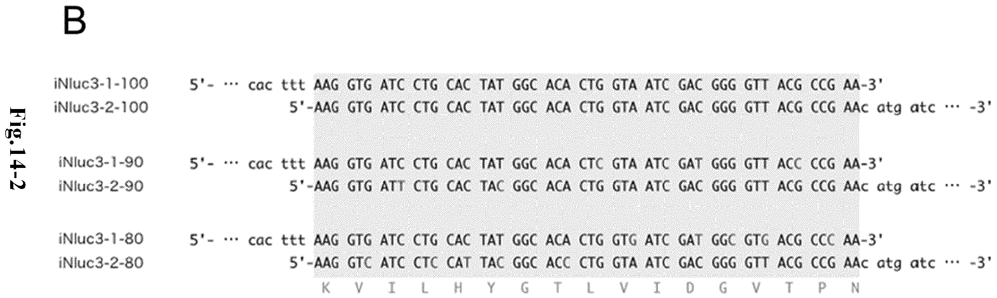

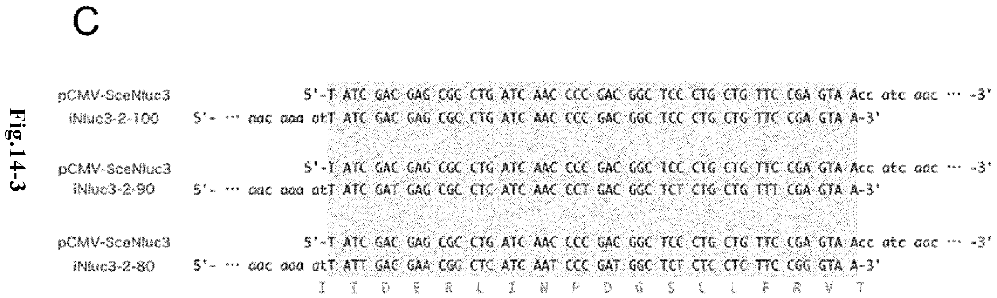

iNluc3-1 fragments and iNluc3-2 fragments having decreased homologies between the homologous sequences were obtained by PCR amplification using the pNL1.1[Nluc] Vector (Promega, Madison, WI, USA) as a template. As a polymerase for the PCR, Tks Gflex (trade name) DNA Polymerase (Takara Bio Inc.) was used. Silent mutations were introduced such that the homologies were 90% (5 sites) and 80% (10 sites), respectively (Fig. 14-1 to Fig. 14-3). The primers used are shown in Table 3 below. iNluc3-1-90-Fw (SEQ ID NO:39) and iNluc3-1-90-Rv (SEQ ID NO:40) were used for the amplification of an iNluc3-1-90 fragment (90% homology, SEQ ID NO:50); iNluc3-1-80-Fw (SEQ ID NO:41) and iNluc3-1-80-Rv (SEQ ID NO:42) were used for the amplification of an iNluc3-1-80 fragment (80% homology, SEQ ID NO:51); iNluc3-2-90-Fw (SEQ ID NO:43) and iNluc3-2-90-Rv (SEQ ID NO:44) were used for the amplification of an iNluc3-2-90 fragment (90% homology, SEQ ID NO:52); and iNluc3-2-80-Fw (SEQ ID NO:45) and iNluc3-2-80-Rv (SEQ ID NO:46) were used for the amplification of an iNluc3-2-80 fragment (80% homology, SEQ ID NO:53).

The pCMV-SceNluc3 was purified using a Qiagen Plasmid Plus Midi Kit (Qiagen K.K.), and linearized by cleavage with I-SceI (New England Biolabs, Ipswich, MA, USA) or with I-SceI and AhdI (New England Biolabs) before use in transfection. The iNluc3-1-100 fragment, iNluc3-2-100 fragment, iNluc3-1-90 fragment, iNluc3-1-80 fragment, iNluc3-2-90 fragment, and iNluc3-2-80 fragment were purified with the Wizard SV Gel and PCR Clean-Up System (Promega) before use in transfection.

**[Table 3]**

| Primer name | Sequence (5'→3') | SEQ ID NO |
|---|---|---|
| Nluc-Fw | TAATACGACTCACTATAGGCTAG | 1 |
| 5'SceNluc3-Rv | CATTACCCTGTTATCCCTACGCG**TCA**ACCGCTCAGGACAATCCTTTGG | 33 |
| 3'SceNluc3-Fw | GTAGGGATAACAGGGTAATGCATATATCGACGAGCGCCTGATCAACC | 34 |
| Nluc-Rv | CCGCCCCGACTCTAGAGTCGCGG | 4 |
| iNluc3-1-100-Fw | AGAATCTCGGGGTGTCCGTAAC | 35 |
| iNluc3-1-100-Rv | TTCGGCGTAACCCCGTCGATTACC | 36 |
| iNluc3-2-100-Fw | AAGGTGATCCTGCACTATGGCAC | 37 |
| iNluc3-2-100-Rv | TTACTCGGAACAGCAGGGAGCCGTC | 38 |
| iNluc3-1-90-Fw | AGAATCTGGGGGTGTCTGTAACTCCCATCCAAAGAATTGTCCTCAGCGG | 39 |
| iNluc3-1-90-Rv | TTCGGGGTAACCCCATCGATTACGAGT | 40 |
| iNluc3-1-80-Fw | AGAACCTCGGCGTCTCTGTAACCCCGATTCAAAGAATCGTGCTGAGTGGT | 41 |
| iNluc3-1-80-Rv | TTGGGCGTCACGCCATCGATCACCAG | 42 |
| iNluc3-2-90-Fw | AAGGTGATTCTGCACTACGGCACAC | 43 |
| iNluc3-2-90-Rv | AAGGTCATCCTCCATTACGGCACCCTGG | 44 |
| iNluc3-2-80-Fw | TTACTCGAAACAGCAGAGAGCCGTCAGGGTTGATGAGGCGCTCATCGA | 45 |
| iNluc3-2-80-Rv | TTACCCGGAAGAGGAGAGAGCCATCGGGATTGATGAGCCGTTCGTCAATA | 46 |

| | | |
|---|---|---|
| Single-underline: I-Scel recognition sequence Double-underline: A site that anneals to the target gene sequence (Nluc-Fw and Nluc-Rv target vector sequences located upstream and downstream of the *Nluc* gene in pNL1.1[Nluc] Vector, respectively.) Bold: Sequence for introduction of a stop codon (TGA) | | |

### A-IV-1-2. Preparation of Vectors (Four-molecule, Three-SSA-Reaction Type) (Fig. 13)

pCMV-SceNluc3 was divided into two molecules by cleavage at two sites using I-SceI and AhdI (a recognition sequence for AhdI is present in the ampicillin resistance gene in the backbone (vector sequence)), and combined with various iNluc3-1 and iNluc3-2 fragments to prepare four-molecule-type constructs.

### A-IV-2. Cells and Gene Transfection, and Luciferase Assay

The same operation as described in A-I-2. was carried out.

### A-IV-3. Results

The three-molecule-type and four-molecule-type constructs prepared in the present Example was designed such that a normal *Nluc* gene is generated when single-strand annealing (SSA) has occurred at the following three sites: between the 3'-side of the SceNluc3 fragment and the 5'-side of the iNluc3-1 fragment; between the 3'-side of the iNluc3-1 fragment and the 5'-side of the iNluc3-2 fragment; and between the 3'-side of the iNluc3-2 fragment and the 5'-side of the SceNluc3 fragment (Fig. 12, Fig. 13).

Since decreased homology in the homologous regions leads to inhibition of the SSA reaction by the MMR factor, a difference is thought to occur depending on the presence or absence of the MMR factor. The SSA constructs prepared in Example A-I have a structure in which a normal *Nluc* gene is formed by two times of SSA reaction, and therefore the difference in the expression level of the *Nluc* gene depending on the presence or absence of the MMR factor was increased compared to a conventional SSA construct (WO 2021/162121 A1). Since the constructs of the present Example require occurrence of SSA at three sites (three times of SSA) for the formation of a normal *Nluc* gene, the difference in the expression level of the *Nluc* gene depending on the presence or absence of the MMR factor is expected to be further increased even compared to the constructs of Example A-I.

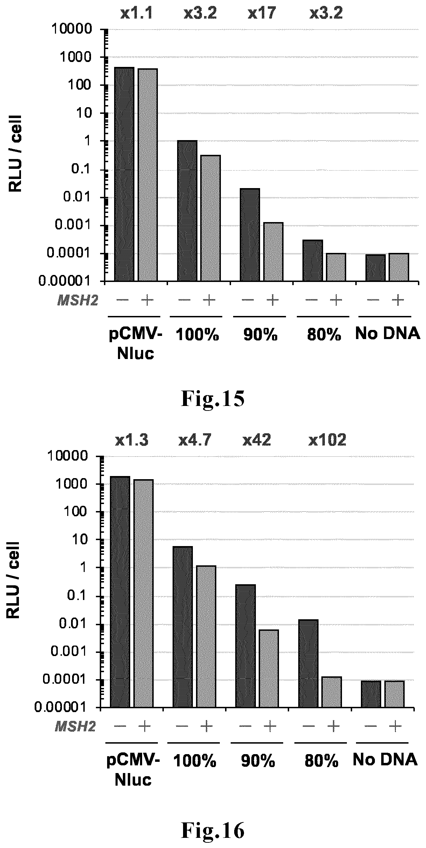

Fig. 15 shows one example of the results of luciferase assays of Nalm-6 cells and MSH2 revertant cells thereof into which three-molecule, three-SSA-reaction type Nluc constructs prepared in the present Example (Fig. 12) have been introduced (results obtained 4 hours after the gene transfection). The constructs having homologous regions with decreased homologies of 90% and 80% exhibited high Luc activity under the MSH2-deficient condition (in the WT Nalm-6 cells deficient in MMR).

Fig. 16 shows the luciferase activities 24 hours after the introduction of the three-molecule, three-SSA-reaction type constructs. The difference in the luciferase activity depending on the presence or absence of MSH2 deficiency (MMR deficiency) was more remarkable in the constructs having homologous regions with a decreased homology than that observed 4 hours after the introduction. In particular, a 102-fold difference in the luciferase activity occurred in the construct having homologous regions with a homology of 80%.

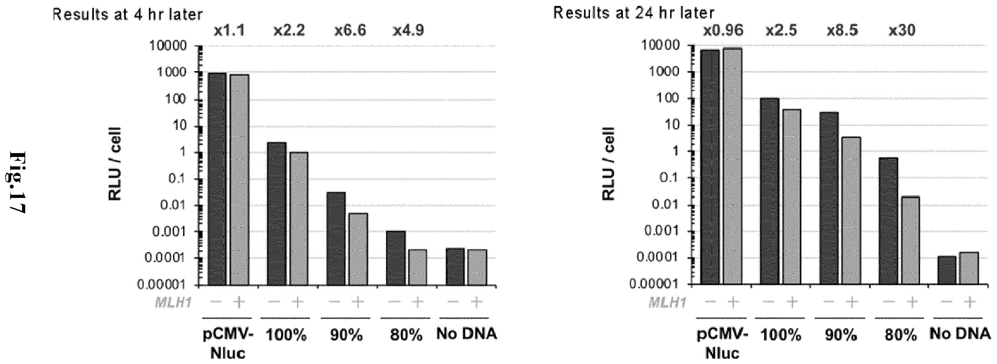

Fig. 17 shows the results of measurement of the luciferase activity 4 hours or 24 hours after the introduction of the three-molecule, three-SSA-reaction type constructs into HCT116, which is a cancer cell line with MMR deficiency due to MLH1 deficiency, and into MLH1 revertant cells thereof. It could be confirmed that, depending not only on the presence or absence of mismatch repair deficiency caused by MSH2, but also on the presence or absence of mismatch repair deficiency caused by MLH1, the difference in gene expression (luciferase activity) increases when the homology of the homologous regions is decreased.

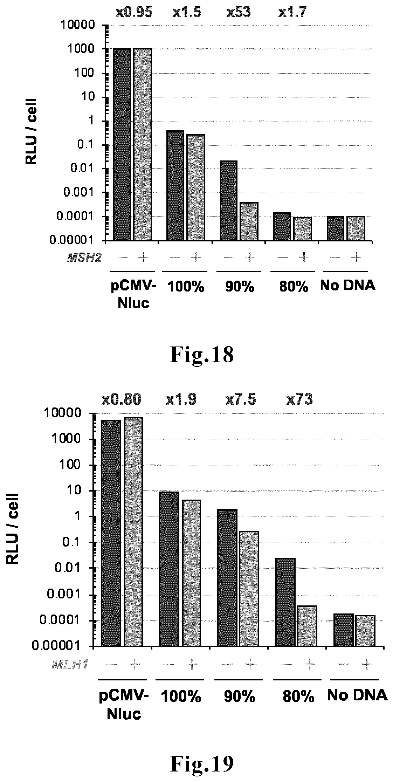

Fig. 18 shows the results of measurement of the luciferase activity 24 hours after the introduction of the four-molecule, three-SSA-reaction type constructs into Nalm-6 cells and MSH2 revertant cells thereof. Although the absolute value of luciferase activity decreased to 1/10 or less compared to the three-molecule, three-SSA-reaction type prepared by digesting pCMV-SceNluc3 with only I-SceI, the construct having homologous regions with a homology of 90% showed a 53-fold difference in the luciferase activity depending on the presence or absence of MSH2 deficiency (MMR deficiency).

Also when the four-molecule, three-SSA-reaction type constructs were introduced into HCT116 cells and MLH1 revertant cells thereof, the absolute value of luciferase activity decreased to 1/10 or less. It was found, however, that the difference in the luciferase activity increases when the homology of the homologous regions is decreased (Fig. 19).

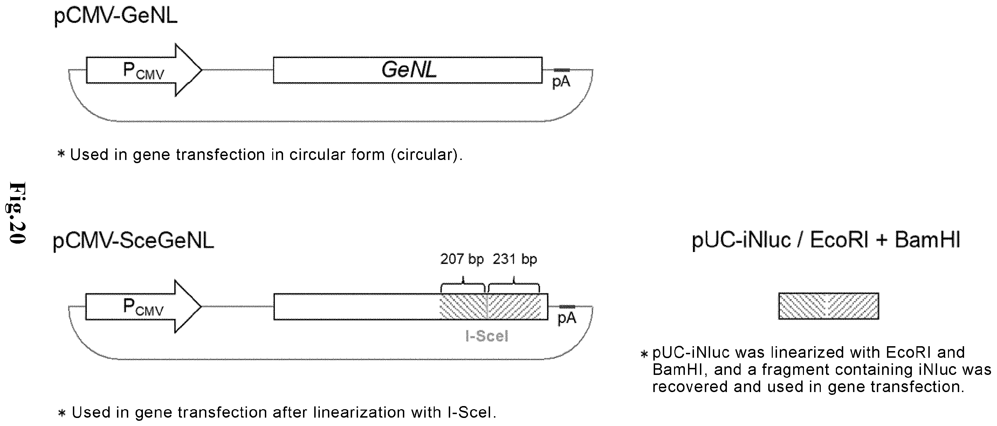

### Example A-V: Divided-Type SA-GeNL-v2 (SceGeNL + iNluc) (Two-Molecule, Two-SSA-Reaction Type) (Fig. 20)

The *GeNL* (green enhanced nano-lantern) gene encodes a fusion protein of the chemiluminescent protein Nluc and the green fluorescent protein mNeonGreen (Shaner NC et al. Nat Methods. 2013, May 10(5), 407-409.). GeNL protein is known to have a luminescence intensity which is about 1.8 times as high as that of Nluc due to forester resonance energy transfer (FRET) that occurs between Nluc and mNeonGreen (Suzuki K et al. Nat Commun. 2016, Dec 14; 7: 13718.). Therefore, by using a sequence of the *GeNL* gene as the gene sequence formed by SSA in the nucleic acid construct of the present invention, the presence or absence of MMR deficiency can be more sensitively measured compared to cases where a chemiluminescent gene is used alone. Specific examples of such use are shown below.

### A-V-1. Preparation of Vectors

### A-V-1-1. GeNL Expression Vector (Top Row of Fig. 20)

In order to prepare constructs that express the *GeNL* gene, a partial *GeNL* gene fragment in which a recognition sequence of XhoI is linked 5'-upstream, and in which the region of positions 16 to 79 (including an EcoNI recognition sequence) in the *Nluc* gene (SEQ ID NO:7) is linked 3'-downstream, of the mNeonGreen gene (the region of positions 1 to 678 in the mNeonGreen gene sequence shown in SEQ ID NO:172) was prepared by artificial gene synthesis (pGeNL). The prepared pGeNL was digested with XhoI and EcoNI, and a fragment containing the partial *GeNL* gene fragment was recovered. Subsequently, pCMV-Nluc (Saito S, et al. Nature Commun. 8:16112, 2017) was digested with XhoI and EcoNI to remove the 5'-upstream untranslated region of the *Nluc* gene and positions 1 to 73 in the *Nluc* gene, and a fragment containing the CMV promoter, the poly-A addition signal, and part of the *Nluc* gene was recovered. Using a DNA Ligation Kit <Mighty Mix> (Takara Bio Inc.), the partial *GeNL* gene fragment was inserted into the recovered fragment, to obtain a vector that expresses GeNL, in which [CMV promoter] - [*GeNL* gene fragment] - [poly-A addition signal] were linked together (pCMV-GeNL) (top row of Fig. 20). The base sequence of the *GeNL* gene, and the amino acid sequence of the GeNL protein encoded thereby, are shown in SEQ ID NOs: 174 and 175, respectively. The GeNL protein prepared has a structure in which the 1st to 226th residues of mNeonGreen protein (SEQ ID NO: 173) was linked to the 6th to 171st residues of Nluc protein (SEQ ID NO:8) through two amino acid residues (GF).

### A-V-1-2. Divided-Type SA-GeNL-v2 (Bottom Row of Fig. 20)

In order to prepare pCMV-SceGeNL, the pCMV-SceNluc prepared in Example A-I was digested with EcoNI and XbaI, and a fragment containing a partial *SceNluc* gene fragment was recovered. Subsequently, pCMV-GeNL was digested with EcoNI and XbaI to remove positions 743 to 1182 and the stop codon in the *GeNL* gene, and a fragment containing the CMV promoter, the poly-A addition signal, and part of the *GeNL* gene was recovered. Using a DNA Ligation Kit <Mighty Mix> (Takara Bio Inc.), the partial *SceNluc* gene fragment was inserted into the recovered fragment, to obtain a vector that expresses SceGeNL, in which [CMV promoter] *-* [*SceGeNL* gene fragment] - [poly-A addition signal] were linked together (pCMV-SceGeNL). The *SceGeNL* gene is a DNA having a structure in which the region of positions 892 to 912 in the *GeNL* gene was replaced with "stop codon (TGA) + I-SceI recognition sequence". The base sequence of the *SceGeNL* gene is shown in SEQ ID NO: 176; the amino acid sequence encoded by positions 1 to 894 thereof (the region up to the first stop codon) is shown in SEQ ID NO: 177; and the amino acid sequence encoded by positions 904 to 1185 thereof (the region located downstream of the third stop codon) is shown in SEQ ID NO: 178 (it should be noted that expression of a polypeptide consisting of this sequence does not occur).

pUC-iNluc having sequences homologous to positions 685 to 891 and positions 913 to 1143 in the *SceGeNL* gene, and pUC-iNluc (90% homology) and pUC-iNluc (80% homology) with decreased homologies between the homologous sequences were prepared as described in A-I-1.

The pCMV-SceGeNL was purified using a Qiagen Plasmid Plus Midi Kit (Qiagen K.K.), and linearized by cleavage with I-SceI (New England Biolabs, Ipswich, MA, USA) before use in transfection. The pUC19, pUC-iNluc, pUC-iNluc (90% homology), and pUC-iNluc (80% homology) were purified using a Qiagen Plasmid Plus Midi Kit (Qiagen K.K.), and then linearized by cleavage with BamHI and EcoRI, followed by purification of the fragment containing iNluc using the Wizard SV Gel and PCR Clean-Up System (Promega) before use. Cleavage of pUC-iNluc with BamHI and EcoRI results in a DNA containing no additional sequence (bottom row of Fig. 20).

### A-V-2. Cells and Gene Transfection, and Luciferase Assay

The same operation as described in A-I-2. was carried out.

### A-V-3. Results

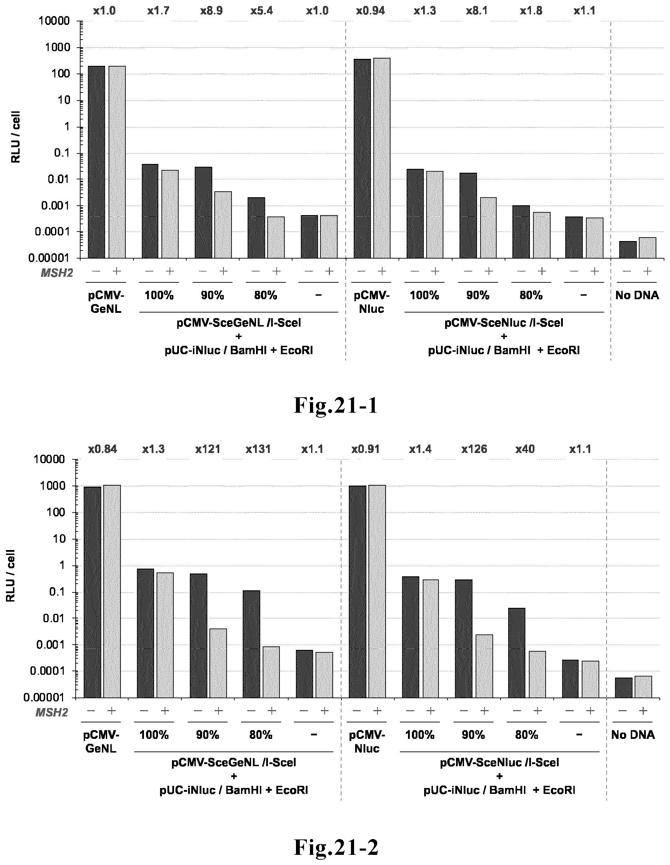

The constructs prepared in the present Example were designed such that a normal *GeNL* gene is generated when homologous recombination (HR) occurs between the SceGeNL fragment and the iNluc fragment, or when single-strand annealing (SSA) occurs at two sites, one in the 5'-terminal portion and one in the 3'-terminal portion of the respective fragments. In cases where the homologous regions have sufficiently high homology, HR occurs. However, in cases where the homologous regions have decreased homology, HR does not occur, and a normal *GeNL* gene is generated only when the SSA has occurred. At this time, the SSA reaction is inhibited by the MMR factor, so that the presence or absence of the MMR factor is thought to cause a difference in the expression level of the *GeNL* gene. Fig. 21-1 and Fig. 21-2 show examples of the results of luciferase assays of Nalm-6 cells and MSH2 revertant cells thereof into which the prepared constructs have been introduced. Fig. 21-1 shows the luciferase activity 4 hours after the gene transfection, and Fig. 21-2 shows the luciferase activity 24 hours after the gene transfection. The constructs having homologous regions with decreased homologies of 90% and 80% exhibited high Luc activity under the MSH2-deficient condition (in the WT Nalm-6 cells deficient in MMR). Since the luminescence intensity of GeNL protein is known to be about 1.8 times higher than that of Nluc protein, constructs using GeNL are thought to be capable of detecting the luciferase activity with higher sensitivity than constructs using Nluc. In fact, as shown in Fig. 21-1, in the cases of the GeNL-based constructs, there was a clear difference in luciferase activity between the wild-type line and MSH2 revertant cells at the time of 4 hours after the introduction, and an about 5-fold difference was observed. On the other hand, when the constructs using Nluc were introduced, the difference in the luciferase activity depending on the presence or absence of MSH2 deficiency was about 2-fold at the time of 2 hours after the introduction. At the time of 24 hours after the introduction of the constructs having homologous regions with decreased homologies of 90% and 80%, both the GeNL and Nluc constructs caused clear differences in luciferase activity between the wild-type line and the MSH2 revertant cells (Fig. 21-2). In particular, the GeNL construct having homologous regions with a homology of 80% showed an about 130-fold difference in the luciferase activity depending on the presence or absence of MSH2 deficiency. It could thus be confirmed that, similarly to Nluc-based constructs, GeNL-based constructs enable detection of the presence or absence of MMR deficiency in the cell.

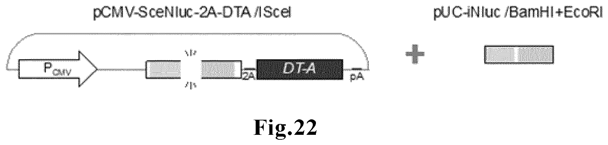

### Example B: Divided-Type DR-Nluc-Homeo DTA-Linked Type (SceNluc + iNluc) (Two-Molecule, Two-SSA-Reaction) (Fig. 22)

A two-molecule-type construct in which a normal *Nluc* gene is formed by SSA at two sites (two times of SSA) to cause polycistronic expression of Nluc and DTA linked downstream of Nluc was constructed, and evaluated for its cytocidal effect.

### B-1. Preparation of Vectors

In order to prepare pCMV-Nluc-2A-DTA, a DNA fragment in which the *Nluc* gene (SEQ ID NO:7) was linked to a sequence (SEQ ID NO:54) encoding 2A peptide (SEQ ID NO:55) derived from *Thosea asigna* virus was prepared by artificial gene synthesis (pNluc-2A, a plasmid having a structure in which a Nluc-2A fragment prepared by artificial gene synthesis was inserted into the EcoRI-HindIII site of pUC57 (GenScript Japan Inc., Tokyo, Japan)). The pNluc-2A prepared by the artificial gene synthesis was linearized by digestion with BamHI, and a *DTA* gene fragment obtained by digesting pCMV-DTA (a construct that expresses a normal *DT-A* gene (SEQ ID NO:56) under the control by the CMV promoter; see Example D-I for its preparation method) with BamHI was ligated to the linearized fragment using a DNA Ligation Kit <Mighty Mix>, to obtain pNluc-2A-DTA. The prepared pNluc-2A-DTA was digested with NheI and MfeI to cut out a DNA fragment in which the *Nluc* gene and the *DTA* gene were linked through a 2A peptide sequence. Subsequently, pIRES (Takara Bio Inc., Fig. 2) was digested with NheI and MfeI to cut out a fragment containing a CMV promoter and a poly-A addition signal, and the Nluc-2A-DTA fragment was inserted downstream of the CMV promoter using a DNA Ligation Kit <Mighty Mix> to obtain pCMV-Nluc-2A-DTA, in which [CMV promoter] - [Nluc] - [2A peptide sequence] - [DTA] - [poly-A addition signal] were linked together. The base sequence of the [Nluc] - [2A peptide sequence] - [DTA] portion is shown in SEQ ID NO:58, and the amino acid sequence encoded thereby is shown in SEQ ID NO:59.

Further, in order to prepare a vector in which the *SceNluc* gene and the *DTA* gene are linked through a 2A peptide sequence, pCMV-Nluc-2A-DTA was digested with EcoNI and PpuMI to cleave the *Nluc* gene at two internal sites to remove the central portion of the *Nluc* gene, and a fragment containing the CMV promoter, the *Nluc* gene lacking its central portion (the 5'-side portion and the 3'-side portion of the *Nluc* gene) and 2A-DTA was recovered. Subsequently, the pCMV-SceNluc prepared in A-I-1. was digested with EcoNI and PpuMI to cleave the *SceNluc* gene at two internal sites (upstream and downstream of the I-SceI recognition sequence) , and a partial fragment of the *SceNluc* gene containing the I-SceI recognition sequence was recovered. Using a DNA Ligation Kit <Mighty Mix>, the partial fragment of the *SceNluc* gene was inserted into the deleted central portion of the *Nluc* gene in the fragment recovered as described above, to obtain pCMV-SceNluc-2A-DTA, in which [CMV promoter] - [SceNluc] - [2A peptide sequence] - [DTA] - [poly-A addition signal] were linked together. The base sequence of the [SceNluc] - [2A peptide sequence] - [DTA] portion is shown in SEQ ID NO:60; the amino acid sequence encoded by positions 1 to 225 thereof (the region up to the first stop codon) is shown in SEQ ID NO:61; and the amino acid sequence encoded by positions 235 to 1209 thereof (the region located downstream of the third stop codon) is shown in SEQ ID NO:62 (it should be noted that expression of a polypeptide consisting of this sequence does not occur). In SEQ ID NO:60, positions 1 to 513 are the *SceNluc* gene sequence; positions 550 to 603 are the 2A peptide-coding sequence; and positions 619 to 1209 are the *DTA* gene sequence. The 106th to 123rd residues in SEQ ID NO:62 are the 2A peptide sequence. Since 2A peptide undergoes peptide bond skipping between glycine and proline at its C-terminus (between Gly at position 122 and Pro at position 123 in SEQ ID NO:62) during protein translation in the cell, inclusion of a 2A peptide sequence between two protein sequences allows polycistronic expression of the two proteins from one ORF.

The prepared plasmids were purified using a Qiagen Plasmid Plus Midi Kit (Qiagen K.K.). Thereafter, pCMV-Nluc-2A-DTA was used as it is in the circular form in transfection, while pCMV-SceNluc-2A-DTA was linearized by cleavage with I-SceI (New England Biolabs, Ipswich, MA, USA) before use in transfection. The iNluc used was prepared by linearizing, by cleavage with BamHI and EcoRI, the pUC-iNluc with a homology of 100% or with a decreased homology prepared in Example A-I.

### B-2. Cells and Gene Transfection, and Evaluation of Cytocidal Effect

For the cells and the gene transfection, see A-I-2. above.

Evaluation of the cytocidal effect was carried out as follows.

With each construct (1 µg), 2×10⁶ Nalm-6 cells (the wild-type line and Msh2 revertant cells thereof) were transfected. The cells after the gene transfection were plated on a 24-well dish at 1×10⁵ cells/ml, and cultured for 96 hours, followed by measurement of the survival rate of the cells using CellTiter-Glo (CellTiter-Glo (registered trademark) Luminescent Cell Viability Assay, Promega).

### B-3. Results

In the present constructs, a normal *Nluc* gene is formed by recombination reaction, and then transcription proceeds to the *DTA* gene portion, followed by expression of Nluc protein and DTA protein by the ribosome skipping mechanism of 2A peptide. By investigating the activity of Nluc by a luciferase assay, cells that will eventually be killed by the action of the DTA protein can be detected.

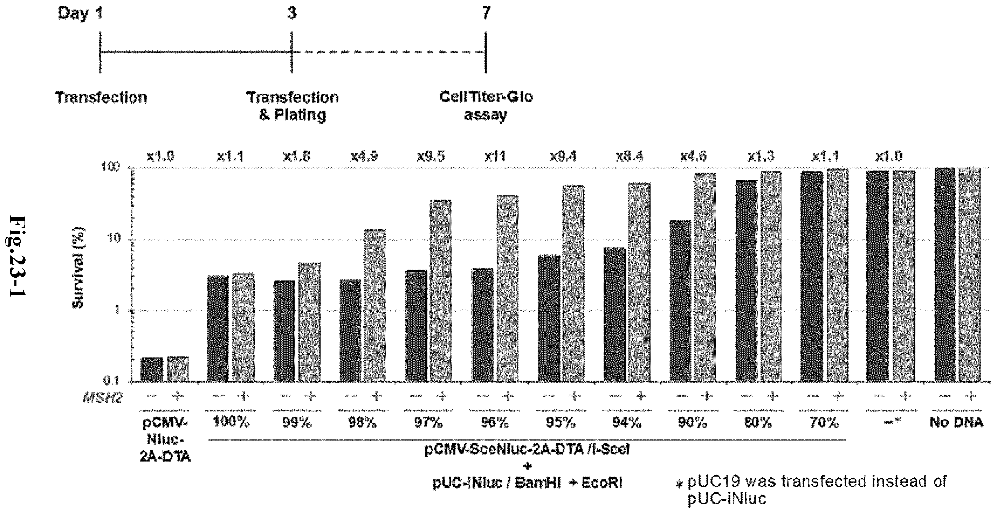

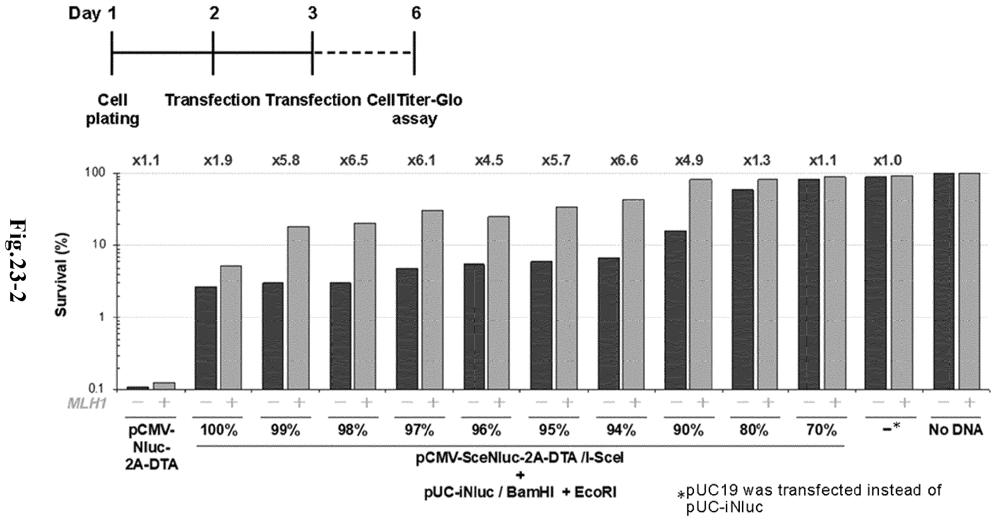

Fig. 23-1 shows the results of comparison of the survival rate 96 hours after introduction of the constructs, between Nalm-6 cells and MSH2 revertant cells thereof. Fig. 23-2 shows the results of comparison of the survival rate 72 hours after introduction of the constructs, between HCT116 cells and MLH1 revertant cells thereof. The constructs having homologous regions with decreased homologies were found to be capable of selectively killing MMR-deficient cells (WT Nalm-6 cells and WT HCT116 cells). Although the introduction of the construct with a homology of 90% resulted in survival of about 20% of the MMR-deficient cells (WT Nalm-6 cells and WT HCT116 cells), the construct hardly showed toxicity to the normal-MMR cells (MSH2 revertant cells (Nalm-6 cells) and MLH1 revertant cells (HCT116 cells)), so that the construct was suggested to be a potential anticancer drug with lower side effects.

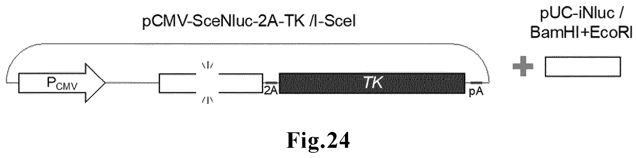

### Example C: Divided-Type DR-Nluc-Homeo TK-Linked Type (SceNluc + iNluc) (Two-Molecule, Two-SSA-Reaction) (Fig. 24)

A two-molecule-type construct in which a normal *Nluc* gene is formed by SSA at two sites (two times of SSA) to cause polycistronic expression of Nluc and HSV-TK linked downstream of Nluc was constructed, and evaluated for its cytocidal effect.

### C-1. Preparation of Vectors

In order to prepare a vector (pCMV-TK) for expressing the *HSV-TK* gene, the ORF of the *HSV-TK* gene was amplified by PCR using, as a template, an *HSV-TK* gene fragment (GenBank:V00470.1, Kobayashi et al. (2001) Decreased topoisomerase II alpha expression confers increased resistance to ICRF-193 as well as VP-16 in mouse embryonic stem cells. Cancer Lett. 166(1): 71-77; the base sequence of the coding region of the *HSV-TK* gene, and the amino acid sequence of the encoded TK protein, are shown in SEQ ID NOs: 63 and 64). PrimeSTAR HS (trade name) DNA Polymerase (Takara Bio Inc.) was used as a PCR polymerase, and TK-Fw (TAATACGACTCACTATAGGCTAGCCTCGAGATCCACCGGTCATGGCTTCGT ACCCCGGCCATC, SEQ ID NO:65) and TK-Rv (CCGCCCCGACTCTAGAGTCGCGGTCAGTTAGCCTCCCCCATCTCC, SEQ ID NO:66) were used as primers. Subsequently, pIRES (Takara Bio Inc., Fig. 2) was digested with NheI and XbaI, and a fragment containing the CMV promoter and the poly-A addition signal was recovered. Using an In-Fusion HD Cloning Kit (Takara Bio Inc.), the *TK* gene fragment was inserted downstream of the CMV promoter to obtain pCMV-TK, in which [CMV promoter] - [*HSV-TK* gene fragment] - [poly-A addition signal] were linked together. The prepared plasmid was purified using a Qiagen Plasmid Plus Midi Kit (Qiagen K.K.) and then used in transfection.

In order to prepare pCMV-Nluc-2A-TK, the pCMV-Nluc-2A-DTA prepared in B-1. was digested with AgeI and MfeI. The *DT-A* gene and the poly-A addition signal were removed, and a fragment containing the CMV promoter, the *Nluc* gene, and the 2A peptide sequence was recovered. Subsequently, pCMV-TK was digested with AgeI and MfeI, and a fragment containing the *HSV-TK* gene and the poly-A addition signal was recovered. Using a DNA Ligation Kit <Mighty Mix>, the *HSV-TK* gene and the poly-A addition signal were inserted downstream of the 2A peptide sequence, to obtain pCMV-Nluc-2A-TK, in which [CMV promoter] - [Nluc] - [2A peptide sequence] - [*HSV-TK* gene] - [poly-A addition signal] were linked together.

Further, in order to prepare a vector in which the *SceNluc* gene and the *HSV-TK* gene are linked through the 2A peptide sequence, the pCMV-SceNluc-2A-DTA prepared in B-1. was digested with AgeI and MfeI. The *DT-A* gene and the poly-A addition signal were removed, and a fragment containing the CMV promoter, the *SceNluc* gene, and the 2A peptide sequence was recovered. Subsequently, pCMV-TK was digested with AgeI and MfeI, and a fragment containing the *HSV-TK* gene and the poly-A addition signal was recovered. Using a DNA Ligation Kit <Mighty Mix>, the *HSV-TK* gene and the poly-A addition signal were inserted downstream of the 2A peptide sequence, to obtain pCMV-SceNluc-2A-TK, in which [CMV promoter] - [SceNluc] - [2A peptide sequence] - [*HSV-TK* gene] - [poly-A addition signal] were linked together. The base sequence of the DNA fragment in which the *SceNluc* gene, the 2A peptide sequence, and the *TK* gene were linked together (SceNluc-2A-TK portion) is shown in SEQ ID NO:67; and the amino acid sequences encoded by positions 1 to 225 (the region up to the first stop codon) and positions 235 to 1749 (the region located downstream of the third stop codon) thereof are shown in SEQ ID NOs:68 and 69, respectively (it should be noted that expression of a polypeptide consisting of the amino acid sequence of SEQ ID NO:69 does not occur). In SEQ ID NO:67, positions 1 to 513 are the *SceNluc* gene sequence; positions 550 to 603 are the 2A peptide-coding sequence; and positions 619 to 1749 are the *TK* gene sequence. The 106th to 123rd residues in SEQ ID NO:69 are the 2A peptide sequence. Since 2A peptide undergoes peptide bond skipping between glycine and proline at its C-terminus (between Gly at position 122 and Pro at position 123 in SEQ ID NO:69) during protein translation in the cell, inclusion of a 2A peptide sequence between two protein sequences allows polycistronic expression of the two proteins from one ORF.

The prepared plasmids were purified using a Qiagen Plasmid Plus Midi Kit (Qiagen K.K.). Thereafter, pCMV-Nluc-2A-TK was used as it is in the circular form in transfection, while pCMV-SceNluc-2A-TK was linearized by cleavage with I-SceI (New England Biolabs, Ipswich, MA, USA) before use in transfection. The iNluc used was prepared by linearizing, by cleavage with BamHI and EcoRI, the pUC-iNluc with a homology of 100% or with a decreased homology prepared in Example A-I.

### C-2. Cells and Gene Transfection, and Evaluation of Cytocidal Effect

For the cells and the gene transfection, see A-I-2. above.

Evaluation of the cytocidal effect was carried out as follows.

With each construct (1 µg), 2×10⁶ Nalm-6 cells (the wild-type line and Msh2 revertant cells thereof) were transfected. After the gene transfection, the cells were cultured for 48 hours, and then transfected again with each construct (1 µg), followed by plating the cells on a 24-well dish at 1×10⁵ cells/ml, and adding ganciclovir (Fujifilm Wako Pure Chemical Corporation) to the medium to a final concentration of 500 nM. After 96 hours of culture, the cell survival rate was measured using CellTiter-Glo (CellTiter-Glo (registered trademark) Luminescent Cell Viability Assay, Promega).

### C-3. Results

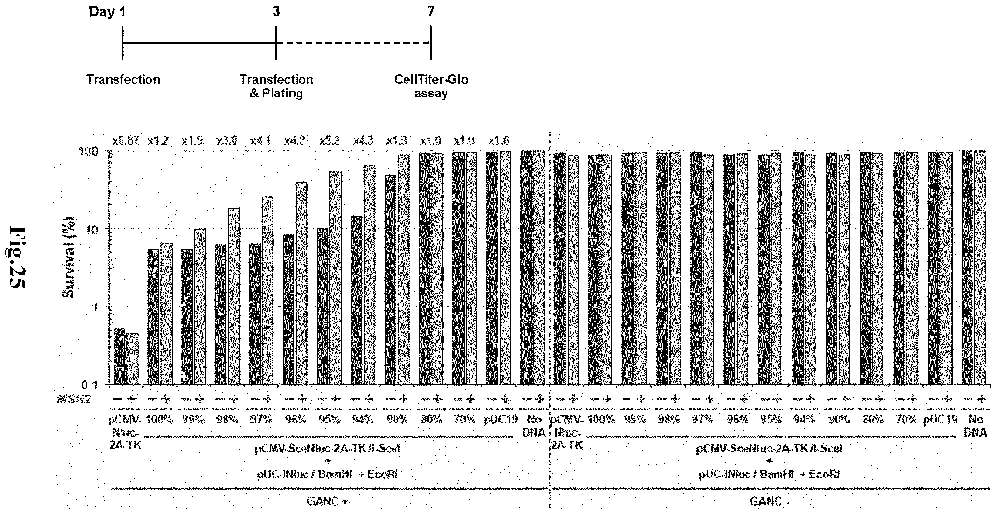

Fig. 25 shows the results of comparison of the survival rate 96 hours after the introduction of the constructs, between the Nalm-6 cells and the MSH2 revertant cells thereof. The survival rate was expressed as a relative value calculated by taking, as 100%, the survival rate of cells not transfected with DNA (No DNA) after culture in the presence of ganciclovir (GANC). The constructs having homologous regions with homologies of 98% to 94% were found to be capable of selectively killing MMR-deficient cells (WT Nalm-6 cells and WT HCT116 cells).

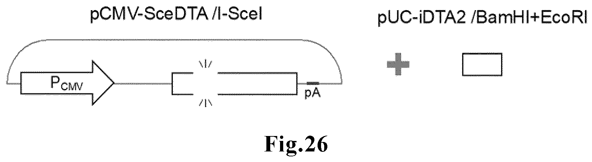

### Example D-I: Divided-Type DR-DTA-Homeo (SceDTA + iDTA) (Two-Molecule, Two-SSA-Reaction) (Fig. 26)

Constructs in which a normal *DT-A* gene is formed by SSA at two sites (two times of SSA) were prepared, and evaluated for their cytocidal effects.

### D-I-1. Preparation of Vectors

A 588-bp SceDTA fragment (a DNA having a structure in which the region of positions 133 to 153 in the *DT-A* gene was replaced with "stop codon (TGA) + I-SceI recognition sequence", having a stop codon added to the 3'-end; SEQ ID NO:76) to be used for pCMV-Sce-DTA was obtained by PCR amplification using pMC1DT-ApA (KURABO, Osaka, Japan) as a template. As a polymerase for the PCR, PrimeSTAR HS (trade name) DNA Polymerase (Takara Bio Inc.) was used. The primers used are shown in Table 4 below. The SceDTA fragment was prepared by separately amplifying two fragments: a 5'SceDTA fragment and a 3'SceDTA fragment. In the amplification of the 5'SceDTA fragment, DTA-Fw (SEQ ID NO:70) and Sce-5'DTA-Rv (SEQ ID NO:71) were used to amplify a DNA fragment having a structure in which a sequence for In-Fusion reaction was added to the 5'-end of the 5'SceDTA fragment, and in which "stop codon (TGA) + I-SceI recognition sequence" was added to the 3'-end of the 5' SceDTA fragment. In the amplification of the 3' SceDTA fragment, Sce-3'DTA-Fw (SEQ ID NO:72) and DTA-Rv (SEQ ID NO:73) were used to amplify a DNA fragment having a structure in which an I-SceI recognition sequence was added to the 5'-end of the 3'SceDTA fragment, and in which a stop codon (TGA) and a sequence for In-Fusion reaction were added to the 3'-end of the 3' SceDTA fragment. Subsequently, pIRES (Takara Bio Inc., Fig. 2) was digested with NheI and XbaI to remove the IRES region, and a fragment containing the CMV promoter and the poly-A addition signal was recovered. Using an In-Fusion HD Cloning Kit (Takara Bio Inc.), the 5'SceDTA fragment and the 3' SceDTA fragment were inserted downstream of the recovered CMV promoter, to obtain pCMV-Sce-DTA, in which [CMV promoter] - [SceDTA fragment] - [poly-A addition signal] were linked together. The amino acid sequences encoded by positions 1 to 135 (the region up to the first stop codon) and positions 145 to 591 (the region located downstream of the third stop codon) of the SceDTA fragment (SEQ ID NO:76) are shown in SEQ ID NO:77 and SEQ ID NO:78, respectively (it should be noted that expression of a polypeptide consisting of the latter sequence does not occur).

A *DT-A* gene fragment of 588 bp to be used for pCMV-DTA was obtained by PCR amplification using pMC1DT-ApA (KURABO, Osaka, Japan) as a template. As a polymerase for the PCR, PrimeSTAR HS (trade name) DNA Polymerase (Takara Bio Inc.) was used. By using DTA-Fw (SEQ ID NO:70) and DTA-Rv (SEQ ID NO:73) as primers, a DNA fragment in which sequences for In-Fusion reaction were added to the 5'-end and the 3'-end was amplified. Subsequently, pIRES (Takara Bio Inc., Fig. 2) was digested with NheI and XbaI to remove the IRES region, and a fragment containing the CMV promoter and the poly-A addition signal was recovered. Using an In-Fusion HD Cloning Kit (Takara Bio Inc.), the *DTA* gene fragment was inserted downstream of the CMV promoter in the recovered fragment, to obtain pCMV-DTA, in which [CMV promoter] - [*DTA* gene] - [poly-A addition signal] were linked together.

An iDTA2 fragment of 221 bp (the region of positions 33 to 253 in the *DT-A* gene; SEQ ID NO:79) having a region homologous to pCMV-Sce-DTA was obtained by PCR amplification using pCMV-DTA as a template. As a polymerase for the PCR, PrimeSTAR HS (trade name) DNA Polymerase (Takara Bio Inc.) was used. As primers, iDTA2-Fw (SEQ ID NO: 74) and iDTA2-Rv (SEQ ID NO:75) were used. Subsequently, pUC19 (Takara Bio Inc.) was digested at SmaI in the multicloning site, and the iDTA2 fragment was inserted thereto using a DNA Ligation Kit <Mighty Mix> (Takara Bio Inc.), to obtain pUC-iDTA2.

**[Table 4]**

| Primer name | Sequence (5'→3') | SEQ ID NO |
|---|---|---|
| DTA-Fw | ACTCACTATAGGCTAGCGTCCTCGCCATGGATCCTGATGATGTTG | 70 |
| Sce-5'DTA-Rv | ATTACCCTGTTATCCCTA**TCA**TTGTGTACCAGATTTTGGC | 71 |
| Sce-3'DTA-Fw | TAGGGATAACAGGGTAATAAAGGGTTTTATAGTACCGACAA | 72 |
| DTA-Rv | CCGCCCCGACTCTAGAA**TCA**CAAAGATCGCCTGACACGATTTCCTG | 73 |
| iDTA2-Fw | ATCTTTTGTGATGGAAAACTTTTCTTCG | 74 |
| iDTA2-Rv | TCACTTTGACCACGCCTCCAGC | 75 |

| | | |
|---|---|---|
| Single-underline: I-Scel recognition sequence Double-underline: A site that anneals to the target gene sequence Bold: Sequence for introduction of a stop codon (TGA) | | |

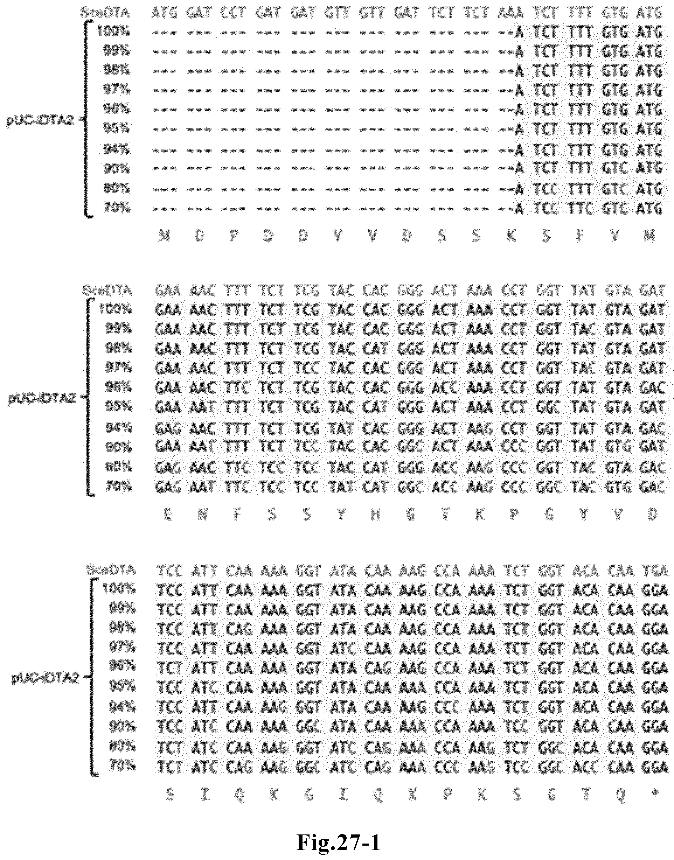

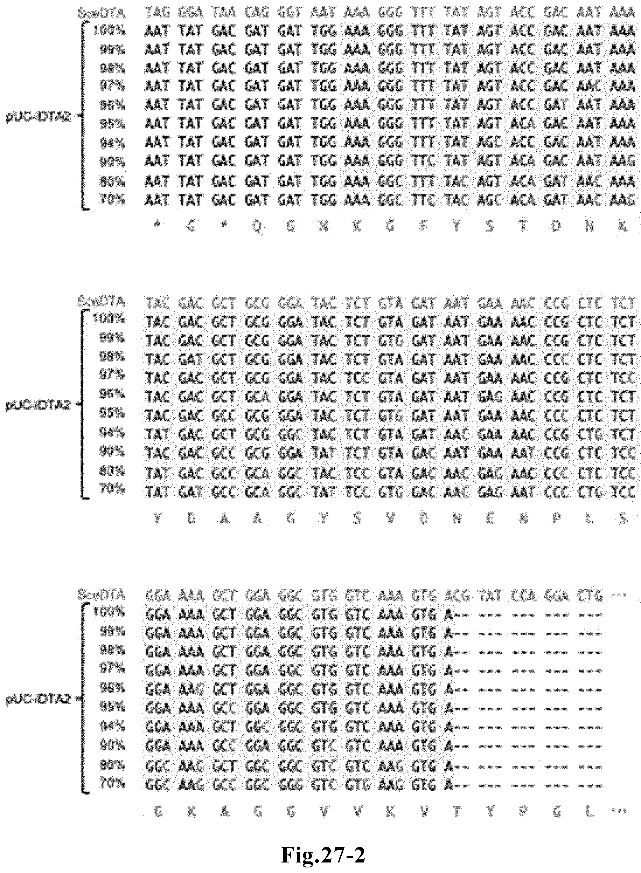

For preparation of vectors having decreased homologies between homologous sequences, artificial gene synthesis was performed to prepare DNA fragments in which silent mutations were introduced in the regions of positions 1 to 100 and positions 122 to 221 of the iDTA2 sequence (221 bp). The silent mutations were introduced such that the homology to SceDTA was 99% (2 sites; 5'-side 1/100, 3'-side 1/100), 98% (4 sites; 5'-side 2/100, 3'-side 2/100), 97% (6 sites; 5'-side 3/100, 3'-side 3/100), 96% (8 sites; 5'-side 4/100, 3'-side 4/100), 95% (10 sites; 5'-side 5/100, 3'-side 5/100), 94% (12 sites; 5'-side 6/100, 3'-side 6/100), 90% (20 sites; 5'-side 10/100, 3'-side 10/100), 80% (20 sites; 5'-side 20/100, 3'-side 20/100), or 70% (62 sites; 5'-side 31/100, 3'-side 31/100), and such that the silent mutations were dispersed as uniformly as possible throughout the sequence (Fig. 27-1, Fig. 27-2). The sequences of the iDTA2 fragments with homologies of 99%, 98%, 97%, 96%, 95%, 94%, 90%, 80%, and 70% are shown in SEQ ID NOs:81 to 89, respectively. Vectors in which the nine iDTA2 fragments having different mutations prepared by the artificial gene synthesis had been cloned were digested with BamHI (Takara Bio Inc.) and EcoRI (Takara Bio Inc.), to cut out each fragment containing each mutated iDTA2. Subsequently, pUC19 (Takara Bio Inc.) was digested with BamHI and EcoRI, and each mutated iDTA2 fragment was inserted therein using a DNA Ligation Kit <Mighty Mix> (Takara Bio Inc.), to obtain pUC-iDTA2 (99% homology), pUC-iDTA2 (98% homology), pUC-iDTA2 (97% homology), pUC-iDTA2 (96% homology), pUC-iDTA2 (94% homology), pUC-iDTA2 (90% homology), pUC-iDTA2 (80% homology), and pUC-iDTA2 (70% homology).

The pCMV-Sce-DTA was purified using a Qiagen Plasmid Plus Midi Kit (Qiagen K.K.), and linearized by cleavage with I-SceI (New England Biolabs) before use in transfection. The pCMV-DTA was purified using a Qiagen Plasmid Plus Midi Kit (Qiagen K.K.), and used in transfection as it is in the form of the circular molecule. The pUC-iDTA2 was purified using a Qiagen Plasmid Plus Midi Kit (Qiagen K.K.), and then linearized by cleavage with BamHI and EcoRI, followed by purification of the fragment containing iDTA2 using the Wizard SV Gel and PCR Clean-Up System (Promega) before use.

### D-I-2. Cells and Gene Transfection, and Evaluation of Cytocidal Effect

For the cells and the gene transfection, see A-I-2. above.

Evaluation of the cytocidal effect on Nalm-6 cells (the wild-type line and Msh2 revertant cells thereof) was carried out as follows. With each construct (1 µg), 2×10⁶ cells were transfected. The cells after the gene transfection were cultured for 48 hours, and transfected again with each construct (1 µg), followed by plating the cells on a 24-well dish at 1×10⁵ cells/ml, performing culture for 96 hours, and then measuring the survival rate of the cells using CellTiter-Glo (CellTiter-Glo (registered trademark) Luminescent Cell Viability Assay, Promega).

Evaluation of the cytocidal effect on HCT116 cells (the wild-type line and Mlh1 revertant cells thereof) was carried out as follows. On a 24-well dish, 5×10⁴ cells were plated and cultured overnight, followed by transfecting the cells with each construct (1 µg). The cells after the gene transfection were cultured for 24 hours, and then transfected again with each construct (1 µg). The cells were cultured for 72 hours, and subjected to measurement of the cell survival rate using CellTiter-Glo (CellTiter-Glo (registered trademark) Luminescent Cell Viability Assay, Promega).

### D-I-3. Results

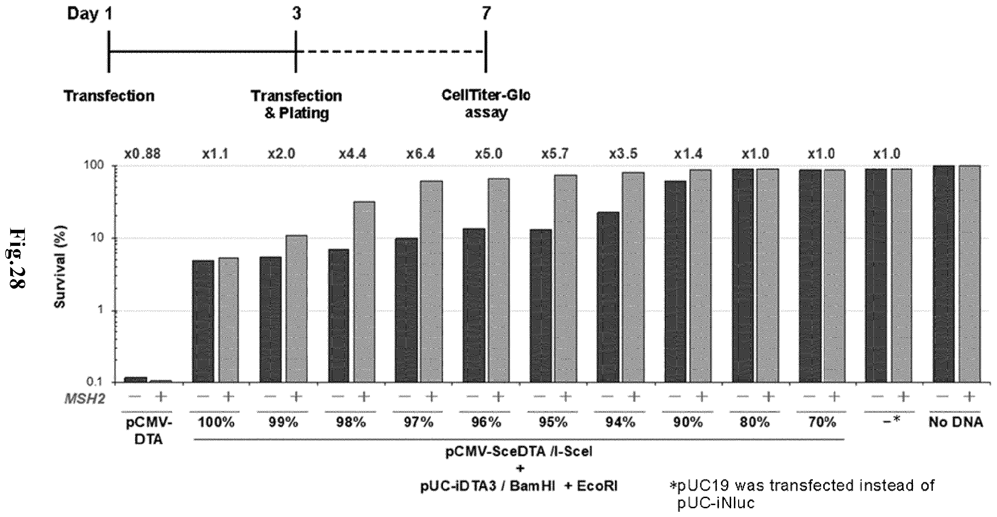

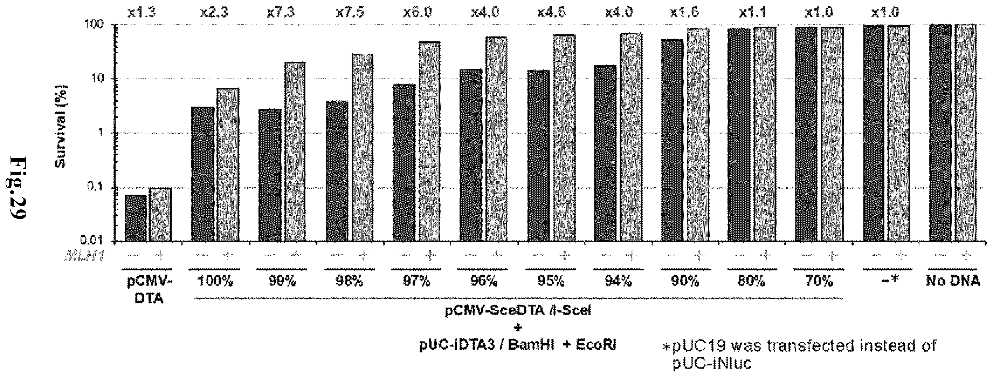

Fig. 28 shows the results of comparison of the survival rate 96 hours after the introduction of the constructs, between the Nalm-6 cells and the MSH2 revertant cells thereof. Fig. 29 shows the results of comparison of the survival rate 72 hours after the introduction of the constructs, between the HCT 116 cells and the MLH1 revertant cells thereof. It was confirmed that, also in cases where the *DT-A* gene is used as the substrate for the recombination, MMR-deficient cells (WT Nalm-6 cells and WT HCT116 cells) can be selectively killed by decreasing the homology of the homologous regions.

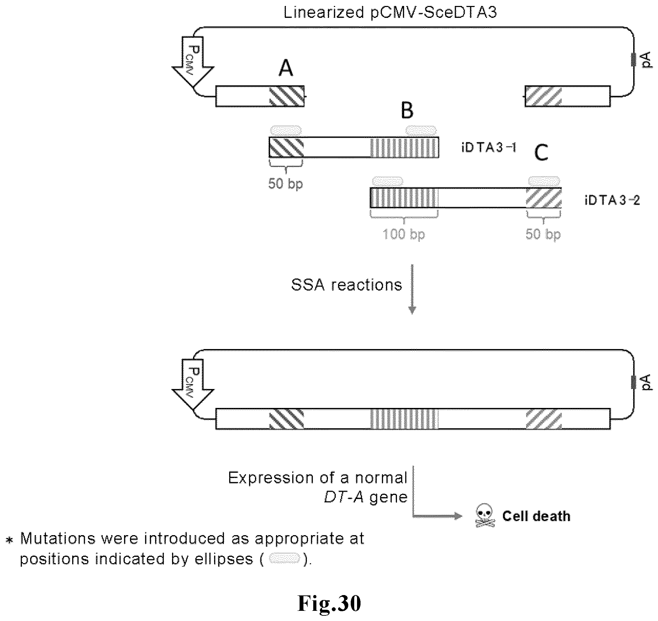

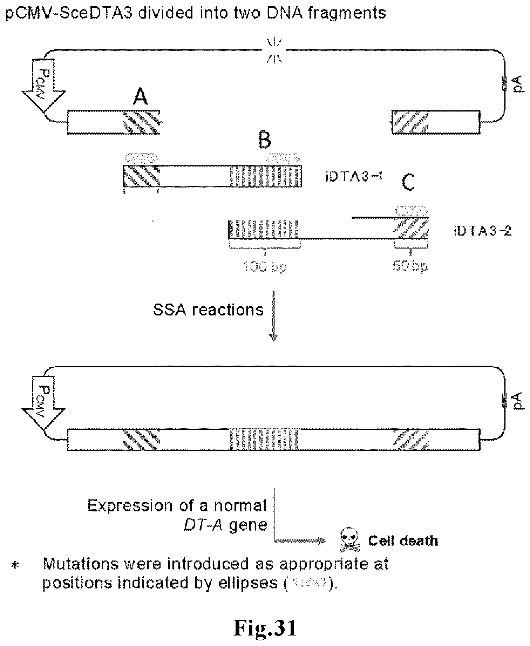

### Example D-II: Divided-Type SA-DTA-v3 (Three-Molecule & Four-Molecule, Three-SSA-Reaction Type) (Fig. 30, Fig. 31)

Divided-type constructs in which a normal *DT-A* gene is formed by SSA at three sites (three times of SSA) were prepared, and evaluated for their cytocidal effects.

### D-II-1-1. Preparation of Vectors (Three-Molecule, Three-SSA-Reaction Type) (Fig. 30)

A 288-bp SceDTA3 fragment (a DNA having a structure in which the region of positions 131 to 464 in the *DT-A* gene sequence was replaced with "stop codon (TGA) + I-SceI recognition sequence", and having a stop codon added to the 3'-end; SEQ ID NO:108) to be used for pCMV-SceDTA3, a 251-bp iDTA3-1-100 fragment (the region of positions 81 to 331 in the *DT-A* gene sequence; SEQ ID NO:112), and a 283-bp iDTA3-2-100 fragment (the region of positions 232 to 514 in the *DT-A* gene sequence; SEQ ID NO:113) were obtained by PCR amplification using pMC1DT-ApA (KURABO, Osaka, Japan) as a template. As a polymerase for the PCR, PrimeSTAR HS (trade name) DNA Polymerase (Takara Bio Inc.) was used. The primers used are shown in Table 5 below. The SceDTA3 fragment was prepared by separately amplifying two fragments: a 5'SceDTA3 fragment and a 3' SceDTA3 fragment. In the amplification of the 5'SceDTA3 fragment, DTA-Fw (SEQ ID NO:70) and 5' SceDTA3-Rv (SEQ ID NO:90) were used to amplify a DNA fragment having a structure in which a sequence for In-Fusion reaction was added to the 5'-end of the 5'SceDTA3 fragment, and in which "stop codon (TGA) + I-SceI recognition sequence" was added to the 3'-end of the 5' SceDTA3 fragment. In the amplification of the 3'SceDTA3 fragment, 3'SceDTA3-Fw (SEQ ID NO:91) and DTA-Rv (SEQ ID NO:73) were used to amplify a DNA fragment having a structure in which "stop codon (TGA) + I-SceI recognition sequence" was added to the 5'-end of the 3'SceDTA3 fragment, and in which a sequence for In-Fusion reaction was added to the 3'-end of the 3' SceDTA3 fragment. For the iDTA3-1-100 fragment, iDTA3-1-100-Fw (SEQ ID NO:92) and iDTA3-1-100-Rv (SEQ ID NO:93) were used. The 50-bp base sequence in the 5'-end region of the iDTA3-1-100 fragment amplified by this PCR has a homology of 100% to the 50 bp in the 3'-end region of pCMV-SceDTA3 digested with I-SceI, and the 100-bp base sequence in the 3'-end region of the iDTA3-1-100 fragment has a homology of 100% to the base sequence in the 5'-end region of the iDTA3-2-100 fragment. For the iDTA3-2-100 fragment, iDTA3-2-100-Fw (SEQ ID NO:94) and iDTA3-2-100-Rv (SEQ ID NO:95) were used. The 100-bp base sequence in the 5'-end region of the iDTA3-2-100 fragment amplified by this PCR has a homology of 100% to the base sequence in the 3'-end region of the iDTA3-1-100 fragment, and the 50-bp base sequence in the 3'-end region of the iDTA3-2-100 fragment has a homology of 100% to the 50 bp in the 5'-end region of pCMV-SceDTA3 digested with I-SceI.

Subsequently, pIRES (Takara Bio Inc., Fig. 2) was digested with NheI and XbaI to remove the IRES region, and a fragment containing the CMV promoter and the poly-A was recovered. Using an In-Fusion HD Cloning Kit (Takara Bio Inc.), the 5' SceDTA3 fragment and the 3' SceDTA3 fragment were inserted downstream of the CMV promoter in the recovered fragment, to obtain pCMV-SceDTA3, in which [CMV promoter] - [SceDTA3 fragment] - [poly-A addition signal] were linked together. The amino acid sequences encoded by positions 1 to 135 (the region up to the first stop codon), positions 136 to 156 (the region up to the second stop codon), and positions 157 to 288 (the region located downstream of the third stop codon) of the SceDTA3 fragment (SEQ ID NO: 108) are shown in SEQ ID NOs: 109 to 111, respectively (it should be noted that expression of polypeptides consisting of the sequences of SEQ ID NOs:110 and 111 does not occur).

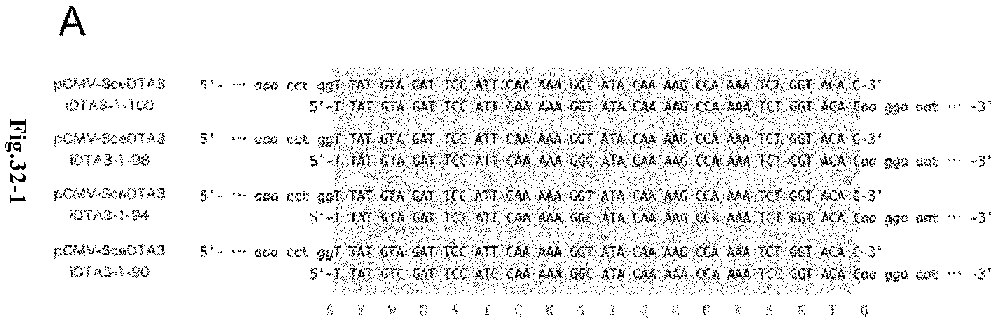

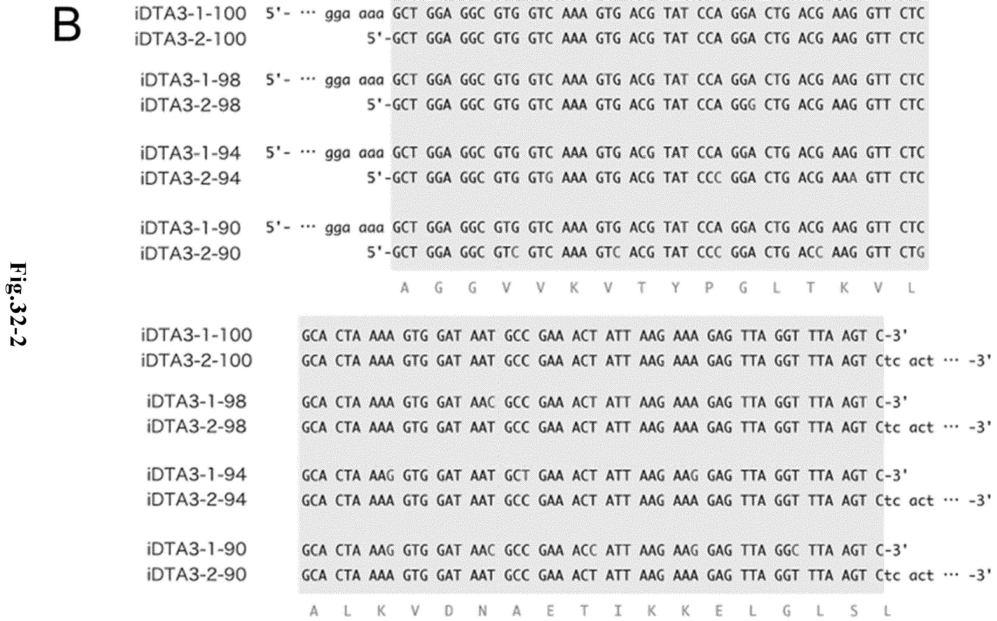

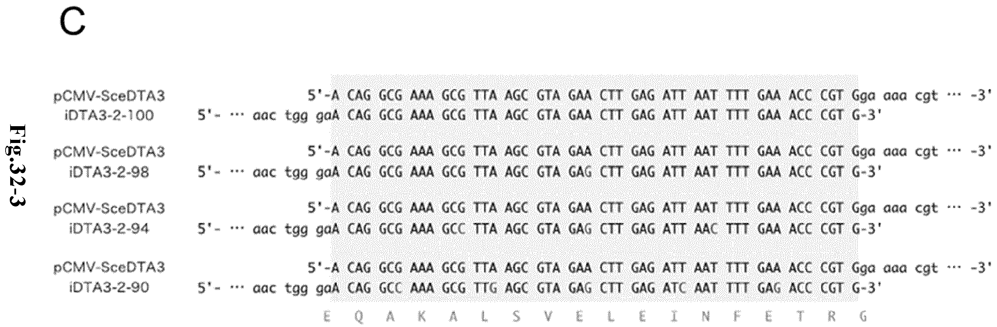

iDTA3-1 fragments and iDTA3-2 fragments having decreased homologies between the homologous sequences were obtained by PCR amplification using the pCMV-DTA prepared in Example D-I as a template. As a polymerase for the PCR, Tks Gflex (trade name) DNA Polymerase (Takara Bio Inc.) was used. Silent mutations were introduced such that the homology was 98% (1 site for a homologous region with a length of 50 bp, or 2 sites for a homologous region with a length of 100 bp), 94% (3 sites for a homologous region with a length of 50 bp, or 6 sites for a homologous region with a length of 100 bp), or 90% (5 sites for a homologous region with a length of 50 bp, or 10 sites for a homologous region with a length of 100 bp) (Fig. 32-1 to Fig. 32-3). The primers used are shown in Table 5 below. iDTA3-1-98-Fw (SEQ ID NO:96) and iDTA3-1-98-Rv (SEQ ID NO:97) were used for the amplification of an iDTA3-1-98 fragment (98% homology, SEQ ID NO: 114); iDTA3-1-94-Fw (SEQ ID NO:98) and iDTA3-1-94-Rv (SEQ ID NO:99) were used for the amplification of an iDTA3-1-94 fragment (94% homology, SEQ ID NO: 115); and iDTA3-1-90-Fw (SEQ ID NO:100) and iDTA3-1-90-Rv (SEQ ID NO:101) were used for the amplification of an iDTA3-1-90 fragment (90% homology, SEQ ID NO:116). iDTA3-2-98-Fw (SEQ ID NO:102) and iDTA3-2-98-Rv (SEQ ID NO:103) were used for the amplification of an iDTA3-2-98 fragment (98% homology, SEQ ID NO:117); iDTA3-2-94-Fw (SEQ ID NO:104) and iDTA3-2-94-Rv (SEQ ID NO:105) were used for the amplification of an iDTA3-2-94 fragment (94% homology, SEQ ID NO:118); and iDTA3-2-90-Fw (SEQ ID NO:106) and iDTA3-2-90-Rv (SEQ ID NO:107) were used for the amplification of an iDTA3-2-90 fragment (90% homology, SEQ ID NO:119).

The pCMV-SceDTA3 was purified using a Qiagen Plasmid Plus Midi Kit (Qiagen K.K.), and linearized by cleavage with I-SceI (New England Biolabs, Ipswich, MA, USA) before use in transfection. The iDTA3-1-100 fragment, iDTA3-2-100 fragment, iDTA3-1-98 fragment, iDTA3-1-94 fragment, iDTA3-1-90 fragment, iDTA3-2-98 fragment, iDTA3-2-94 fragment, and iDTA3-2-90 fragment were purified with the Wizard SV Gel and PCR Clean-Up System (Promega) before use in transfection.

**[Table 5]**

| Primer name | Sequence (5'→3') | SEQ ID NO |
|---|---|---|
| DTA-Fw | ACTCACTATAGGCTAGCGTCCTCGCCATGGATCCTGATGATGTTG | 70 |
| 5'SceDTA3-Rv | CATTACCCTGTTATCCCTACGCG**TCA**CAGTGTACCAGATTTTGGCTTTTG | 90 |
| 3'SceDTA3-Fw | GTAGGGATAACAGGGTAATGCATACAGGCGAAAGCGTTAAGCGTAG | 91 |
| DTA-Rv | CCGCCCCGACTCTAGAA**TCA**CAAAGATCGCCTGACACGATTTCCTG | 73 |
| iDTA3-1-100-Fw | TTATGTAGATTCCATTCAAAAAGGTATAC | 92 |
| iDTA3-1-100-Rv | GACTTAAACCTAACTCTTTCTTAATAG | 93 |
| iDTA3-2-100-Fw | GCTGGAGGCGTGGTCAAAGTGAC | 94 |
| iDTA3-2-100-Rv | CACGGGTTTCAAAATTAATCTCAAG | 95 |
| iDTA3-1-98-Fw | TTATGTAGATTCCATTCAAAAAGGCATACAAAAGC | 96 |
| iDTA3-1-98-Rv | GACTTAAACCTAACTCTTTCTTAATAGTTTCGGCGTTATCCAC | 97 |
| iDTA3-1-94-Fw | TTATGTAGATTCTATTCAAAAAGGCATACAAAAGCCCAAATCTGG | 98 |
| iDTA3-1-94-Rv | GACTTAAACCTAACTCCTTCTTAATAGTTTCAGCATTATCCACCTTTAGTGC | 99 |
| iDTA3-1-90-Fw | TTATGTCGATTCCATCCAAAAAGGCATACAAAAACCAAAATCCGGTAC | 100 |
| iDTA3-1-90-Rv | GACTTAAGCCTAACTCCTTCTTAATGGTTTCGGCGTTATCCACCTTTAGTGC | 101 |
| iDTA3-2-98-Fw | GCTGGAGGCGTGGTCAAAGTGACGTATCCAGGGCTGACG | 102 |
| iDTA3-2-98-Rv | CACGGGTTTCAAAATTAATCTCAAGCTCTACG | 103 |
| iDTA3-2-94-Fw | GCTGGAGGCGTGGTGAAAGTGACGTATCCCGGACTGACGAAAGTTCTCG | 104 |
| iDTA3-2-94-Rv | CACGGGTTTCAAAGTTAATCTCAAGCTCTACGCTTAAGGCTTTCG | 105 |
| iDTA3-2-90-Fw | GCTGGAGGCGTCGTCAAAGTCACGTATCCCGGACTGACCAAGGTTCTGGCAC | 106 |
| iDTA3-2-90-Rv | CACGGGTCTCAAAATTGATCTCAAGCTCTACGCTCAACGCTTTGGCCTG | 107 |

| | | |
|---|---|---|
| Single-underline: I-Scel recognition sequence Double-underline: A site that anneals to the target gene sequence Bold: Sequence for introduction of a stop codon (TGA) | | |

### D-II-1-2. Preparation of Vectors (Four-Molecule, Three-SSA-Reaction Type) (Fig. 31)

pCMV-SceDTA3 was divided into two molecules by cleavage at two sites using I-SceI and AhdI (a recognition sequence for AhdI is present in the ampicillin resistance gene in the backbone (vector sequence)), and combined with various iDTA3-1 and iDTA3-2 fragments to prepare four-molecule-type constructs.

### D-II-2. Cells and Gene Transfection, and Evaluation of Cytocidal Effect See D-I-2.

### D-II-3. Results

The three-molecule-type and four-molecule-type constructs prepared in the present Example were designed such that a normal *Nluc* gene is generated when single-strand annealing (SSA) occurs at the following three sites: between the 3'-side region in the SceDTA3 fragment and the 5'-side region in the iDTA3-1 fragment; between the 3'-side region in the iDTA3-1 fragment and the 5'-side region in the iDTA3-2 fragment; and between the 3'-side region in the iDTA3-2 fragment and the 5'-side region in the SceDTA3 fragment (Fig. 30, Fig. 31).

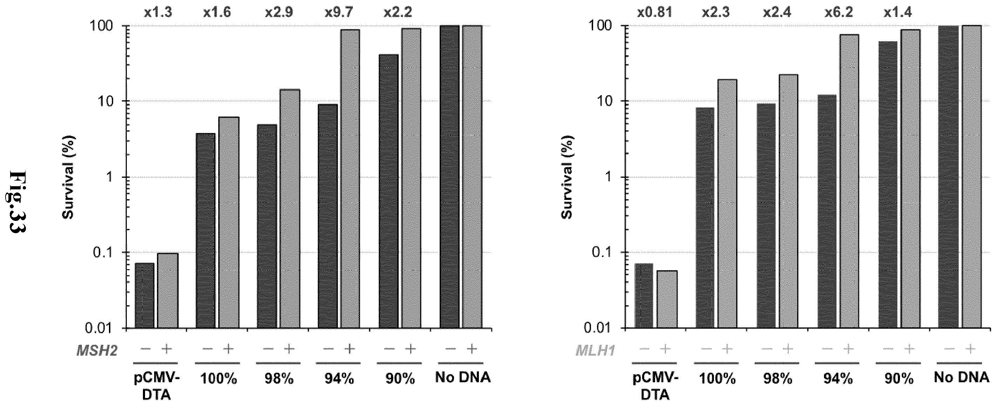

Fig. 33 left panel shows the results of comparison of the survival rate 96 hours after introduction of three-molecule, three-SSA-reaction type constructs, between Nalm-6 cells and MSH2 revertant cells thereof. Fig. 33 right panel shows the results of comparison of the survival rate 72 hours after the introduction of the constructs, between HCT116 cells and MLH1 revertant cells thereof. The constructs having homologous regions with decreased homologies showed differences in the survival rate depending on the presence or absence of the MMR factor. In particular, in the case where the construct with a homology of 94% was introduced, the construct hardly showed toxicity to the normal-MMR cells (MSH2 revertant cells (Nalm-6 cells) and MLH1 revertant cells (HCT116 cells)), demonstrating that the construct of the present invention can selectively kill MMR-deficient cells (WT Nalm-6 cells and WT HCT116 cells).

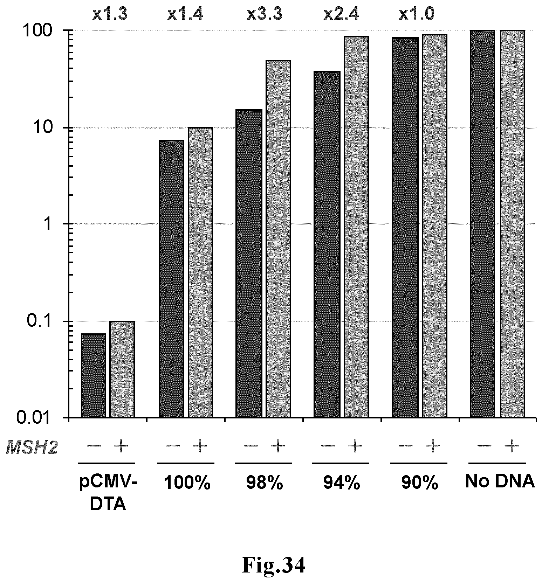

Fig. 34 shows the results of comparison of the survival rate 96 hours after introduction of the four-molecule, three-SSA-reaction type constructs into Nalm-6 cells and MSH2 revertant cells thereof. Although the killing effect was lower than that of the three-molecule constructs, the construct with a homology of 98% showed a 3.3-fold difference in the survival rate depending on the presence or absence of the MSH2 deficiency (MMR deficiency). These results showed that MMR-deficient cells can be selectively killed even in cases where the number of gene fragments required for the formation of a normal *DTA* gene is increased.

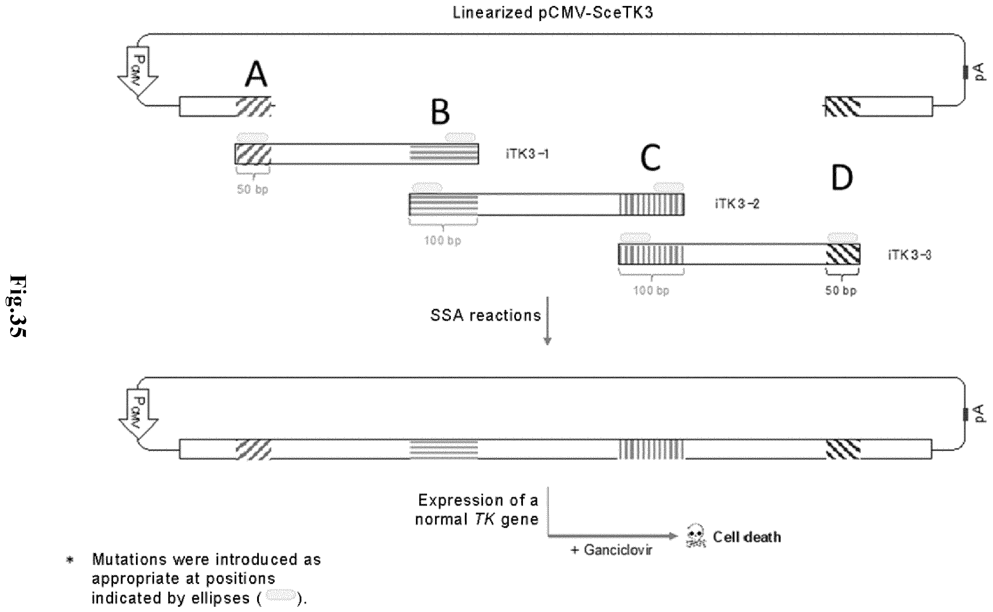

### Example E-I: Divided-Type SA-TK-v3 (Four-Molecule, Four-SSA-Reaction Type) (Fig. 35)

Divided-type constructs in which a normal *HSV-TK* gene is formed by SSA at four sites (four times of SSA) were prepared, and evaluated for their cytocidal effects.

### E-I-1. Preparation of Vectors (Fig. 35)

A 330-bp SceTK3 fragment (a DNA having a structure in which the region of positions 137 to 968 in the *HSV-TK* gene sequence was replaced with "stop codon (TGA) + I-SceI recognition sequence", having a stop codon added to the 3'-end; SEQ ID NO: 148) to be used for pCMV-SceTK3, a 362-bp iTK3-1-100 fragment (the region of positions 87 to 448 in the *HSV-TK* gene sequence; SEQ ID NO:152), a 412-bp iTK3-2-100 fragment (the region of positions 349 to 760 in the *HSV-TK* gene sequence; SEQ ID NO:153), and a 358-bp iTK3-3-100 fragment (the region of positions 661 to 1018 in the *HSV-TK* gene sequence; SEQ ID NO:154) were obtained by PCR amplification using an *HSV-TK* gene fragment (GenBank: V00470.1, Kobayashi et al. (2001) Decreased topoisomerase II alpha expression confers increased resistance to ICRF-193 as well as VP-16 in mouse embryonic stem cells. Cancer Lett. 166(1): 71-77; the base sequence of the coding region of the *HSV-TK* gene, and the amino acid sequence of the TK protein encoded thereby are shown in SEQ ID NOs: 63 and 64) as a template. As a polymerase for the PCR, PrimeSTAR HS (trade name) DNA Polymerase (Takara Bio Inc.) was used. The primers used are shown in Table 6 below. The SceTK3 fragment was prepared by separately amplifying two fragments: a 5'SceTK3 fragment fragment and a 3'SceTK3 fragment. In the amplification of the 5'SceTK3 fragment, Sce-5'TK-Fw (SEQ ID NO:120) and 5'SceTK3-Rv (SEQ ID NO:121) were used to amplify a DNA fragment having a structure in which a sequence for In-Fusion reaction was added to the 5'-end of the 5' SceTK3 fragment, and in which "stop codon (TGA) + I-SceI recognition sequence" was added to the 3'-end of the 5' SceTK3 fragment. In the amplification of the 3' SceTK3 fragment, 3' SceTK3-Fw (SEQ ID NO:122) and Sce-3'TK-Rv (SEQ ID NO:123) were used to amplify a DNA fragment having a structure in which "stop codon (TGA) + I-SceI recognition sequence" was added to the 5'-end of the 3' SceTK3 fragment, and in which a sequence for In-Fusion reaction was added to the 3'-end of the 3'SceTK3 fragment. For the iTK3-1-100 fragment, iTK3-1-100-Fw (SEQ ID NO:124) and iTK3-1-100-Rv (SEQ ID NO:125) were used. The 50-bp base sequence in the 5'-end region of the iTK3-1-100 fragment amplified by this PCR has a homology of 100% to the 50 bp in the 3'-end region of pCMV-SceTK3 digested with I-SceI, and the 100-bp base sequence in the 3'-end region of the iTK3-1-100 fragment has a homology of 100% to the base sequence in the 5'-end region of the iTK3-2-100 fragment. For the iTK3-2-100 fragment, iTK3-2-100-Fw (SEQ ID NO:126) and iTK3-2-100-Rv (SEQ ID NO:127) were used. The 100-bp base sequence in the 5'-end region of the iTK3-2-100 fragment amplified by this PCR has a homology of 100% to the base sequence in the 3'-end region of the iTK3-1-100 fragment, and the 100-bp base sequence in the 3'-end region of the iTK3-2-100 fragment has a homology of 100% to the base sequence of the 5'-end region of the iTK3-3-100 fragment. For the iTK3-3-100 fragment, iTK3-3-100-Fw (SEQ ID NO:128) and iTK3-3-100-Rv (SEQ ID NO:129) were used. The 100-bp base sequence in the 5'-end region of the iTK3-3-100 fragment amplified by this PCR has a homology of 100% to the base sequence in the 3'-end region of the iTK3-3-100 fragment, and the 50-bp base sequence in the 3'-end region of the iTK3-3-100 fragment has a homology of 100% to the 50 bp in the 5'-end region of pCMV-SceTK3 digested with I-SceI.

Subsequently, pIRES (Takara Bio Inc., Fig. 2) was digested with NheI and BamHI, and a fragment containing the CMV promoter was recovered. Using an In-Fusion HD Cloning Kit (Takara Bio Inc.), the 5'SceTK3 fragment and the 3'SceTK3 fragment were inserted downstream of the CMV promoter in the recovered fragment, to obtain pCMV-SceTK3, in which [CMV promoter] - [SceTK3 fragment] - [poly-A addition signal] were linked together. The amino acid sequences encoded by positions 1 to 141 (the region up to the first stop codon), positions 142 to 162 (the region up to the second stop codon), and positions 163 to 330 (the region located downstream of the third stop codon) of the SceTK3 fragment (SEQ ID NO:148) are shown in SEQ ID NOs: 149 to 151, respectively (it should be noted that expression of polypeptides consisting of the sequences of SEQ ID NOs: 150 and 151 does not occur).

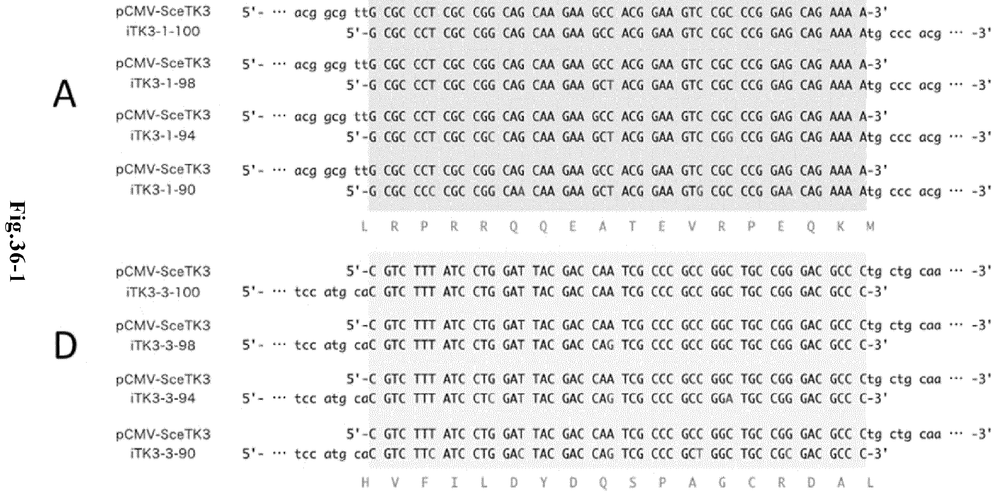

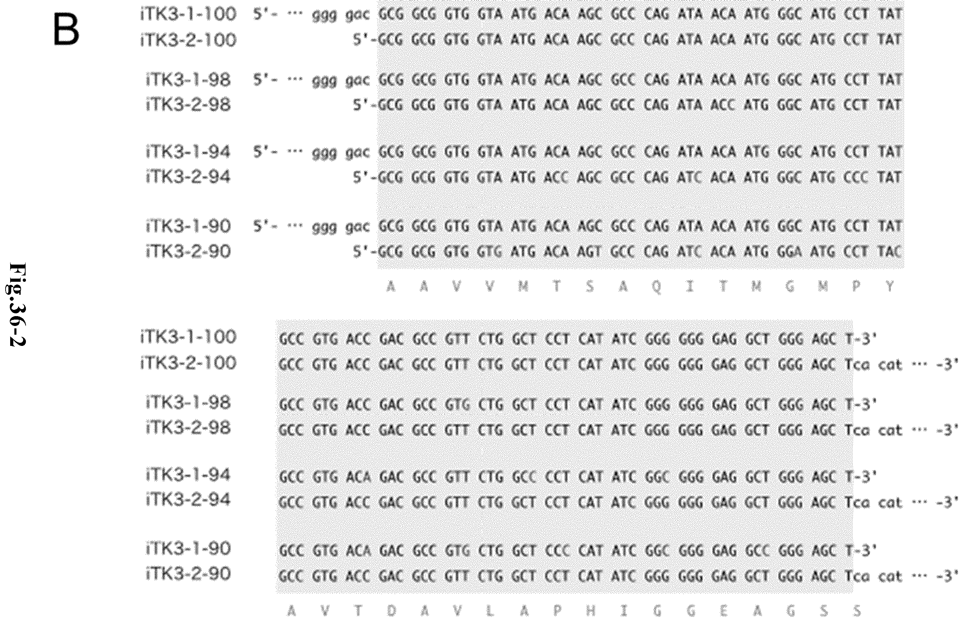

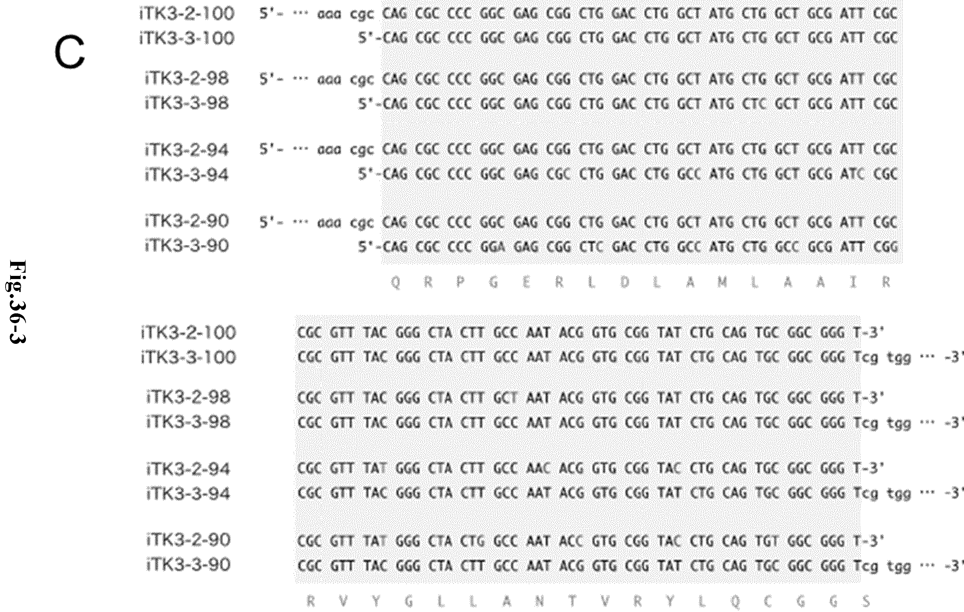

iTK3-1 fragments, iTK3-2 fragments, and iTK3-3 fragments having decreased homologies between the homologous sequences were obtained by PCR amplification using the *HSV-TK* gene fragment as a template. As a polymerase for the PCR, Tks Gflex (trade name) DNA Polymerase (Takara Bio Inc.) was used. Silent mutations were introduced such that the homology was 98% (1 site for a homologous region with a length of 50 bp, or 2 sites for a homologous region with a length of 100 bp), 94% (3 sites for a homologous region with a length of 50 bp, or 6 sites for a homologous region with a length of 100 bp), or 90% (5 sites for a homologous region with a length of 50 bp, or 10 sites for a homologous region with a length of 100 bp) (Fig. 36-1 to Fig. 36-3). The primers used are shown in Table 6 below. iTK3-1-98-Fw (SEQ ID NO:130) and iTK3-1-98-Rv (SEQ ID NO:131) were used for the amplification of an iTK3-1-98 fragment (98% homology, SEQ ID NO:155); iTK3-1-94-Fw (SEQ ID NO:132) and iTK3-1-94-Rv (SEQ ID NO:133) were used for the amplification of an iTK3-1-94 fragment (94% homology, SEQ ID NO:156); and iTK3-1-90-Fw (SEQ ID NO:134) and iTK3-1-90-Rv (SEQ ID NO:135) were used for the amplification of an iTK3-1-90 fragment (90% homology, SEQ ID NO:157). iTK3-2-98-Fw (SEQ ID NO:136) and iTK3-2-98-Rv (SEQ ID NO:137) were used for the amplification of an iTK3-2-98 fragment (98% homology, SEQ ID NO:158); iTK3-2-94-Fw (SEQ ID NO:138) and iTK3-2-94-Rv (SEQ ID NO:139) were used for the amplification of an iTK3-2-94 fragment (94% homology, SEQ ID NO:159); and iTK3-2-90-Fw (SEQ ID NO:140) and iTK3-2-90-Rv (SEQ ID NO:141) were used for the amplification of an iTK3-2-90 fragment (90% homology, SEQ ID NO:160). iTK3-3-98-Fw (SEQ ID NO:142) and iTK3-3-98-Rv (SEQ ID NO:143) were used for the amplification of an iTK3-3-98 fragment (98% homology, SEQ ID NO:161); iTK3-3-94-Fw (SEQ ID NO:144) and iTK3-3-94-Rv (SEQ ID NO:145) were used for the amplification of an iTK3-3-94 fragment (94% homology, SEQ ID NO:162); and iTK3-3-90-Fw (SEQ ID NO:146) and iTK3-3-90-Rv (SEQ ID NO:147) were used for the amplification of an iTK3-3-90 fragment (90% homology, SEQ ID NO:163).

The pCMV-SceTK3 was purified using a Qiagen Plasmid Plus Midi Kit (Qiagen K.K.), and linearized by cleavage with I-SceI (New England Biolabs, Ipswich, MA, USA) before use in transfection. The iTK3-1-100 fragment, iTK3-2-100 fragment, iTK3-3-100 fragment, iTK3-1-98 fragment, iTK3-1-94 fragment, iTK3-1-90 fragment, iTK3-2-98 fragment, iTK3-2-94 fragment, iTK3-2-90 fragment, iTK3-3-98 fragment, iTK3-3-94 fragment, and iTK3-3-90 fragment were purified with the Wizard SV Gel and PCR Clean-Up System (Promega) before use in transfection.

**[Table 6]**

| Primer name | Sequence (5'→3') | SEQ ID NO |
|---|---|---|
| Sce-5'TK-Fw | CTTAATACGACTCACTATAGGCTAGC | 120 |
| 5'SceTK3-Rv | CATTACCCTGTTATCCCTACGCG**TCA**CTTTTTCTGCTCCGGGCGGACTTC | 121 |
| 3'SceTK3-Fw | GTAGGGATAACAGGGTAATGCATCGTCTTTATCCTGGATTACGACC | 122 |
| Sce-3'TK-Rv | GAGTGCACCATACGCGGATC | 123 |
| iTK3-1-100-Fw | GCGCCCTCGCCGGCAGCAAGAAG | 124 |
| iTK3-1-100-Rv | AGCTCCCAGCCTCCCCCCCGATATG | 125 |
| iTK3-2-100-Fw | GCGGCGGTGGTAATGACAAGCG | 126 |
| iTK3-2-100-Rv | ACCCGCCGCACTGCAGATACCG | 127 |
| iTK3-3-100-Fw | CAGCGCCCCGGCGAGCGGCTGGA | 128 |
| iTK3-3-100-Rv | GGGCGTCCCGGCAGCCGGCGGG | 129 |
| iTK3-1-98-Fw | GCGCCCTCGCCGGCAGCAAGAAGCTACGGAAG | 130 |
| iTK3-1-98-Rv | AGCTCCCAGCCTCCCCCCCGATATGAGGAGCCAGCACGGC | 131 |
| iTK3-1-94-Fw | GCGCCCTCGCCGCCAGCAAGAAGCTACGGAAGTCCGGCCGGAG | 132 |
| iTK3-1-94-Rv | AGCTCCCAGCCTCCCCGCCGATATGAGGGGCCAGAACGGCGTCTGTCACG | 133 |
| iTK3-1-90-Fw | GCGCCCCCGCCGGCAACAAGAAGCTACGGAAGTGCGCCCGGAACAGAA | 134 |
| iTK3-1-90-Rv | AGCTCCCGGCCTCCCCGCCGATATGGGGAGCCAGCACGGCGTCTGTCACG | 135 |
| iTK3-2-98-Fw | GCGGCGGTGGTAATGACAAGCGCCCAGATAACCATGGG | 136 |
| iTK3-2-98-Rv | ACCCGCCGCACTGCAGATACCGCACCGTATTAGCAAGTAGC | 137 |
| iTK3-2-94-Fw | GCGGCGGTGGTAATGACCAGCGCCCAGATCACAATGGGCATGCCCTATGCC | 138 |
| iTK3-2-94-Rv | ACCCGCCGCACTGCAGGTACCGCACCGTGTTGGCAAGTAGCCCATAAACGC | 139 |
| iTK3-2-90-Fw | GCGGCGGTGGTGATGACAAGTGCCCAGATCACAATGGGAATGCCTTACGCCGTG | 140 |
| iTK3-2-90-Rv | ACCCGCCACACTGCAGGTACCGCACGGTATTGGCCAGTAGCCCATAAACGC | 141 |
| iTK3-3-98-Fw | CAGCGCCCCGGCGAGCGGCTGGACCTGGCTATGCTCGCTGC | 142 |
| iTK3-3-98-Rv | GGGCGTCCCGGCAGCCGGCGGGCGACTGGTC | 143 |
| iTK3-3-94-Fw | CAGCGCCCCGGCGAGCGCCTGGACCTGGCCATGCTGGCTGCGATCCGCCG | 144 |
| iTK3-3-94-Rv | GGGCGTCCCGGCATCCGGCGGGCGACTGGTCGTAATCGAGGATA | 145 |
| iTK3-3-90-Fw | CAGCGCCCCGGAGAGCGGCTCGACCTGGCCATGCTGGCCGCGATTCGGCGCGT | 146 |
| iTK3-3-90-Rv | GGGCGTCGCGGCAGCCAGCGGGCGACTGGTCGTAGTCCAGGATGAAGAC | 147 |

| | | |
|---|---|---|
| Single-underline: I-Scel recognition sequence Double-underline: A site that anneals to the target gene sequence (Sce-5'TK-Fw and Sce-3'TK-Rv target vector sequences located upstream and downstream of an *HSV-TK* gene fragment, respectively.) Bold: Sequence for introduction of a stop codon (TGA) | | |

### E-I-2. Cells and Gene Transfection, and Evaluation of Cytocidal Effect

For the cells and the gene transfection, see A-I-2. above.

Evaluation of the cytocidal effect on Nalm-6 cells (the wild-type line and Msh2 revertant cells thereof) was carried out as follows. With each construct (1 µg), 2×10⁶ cells were transfected. After the gene transfection, the cells were cultured for 48 hours, and then transfected again with each construct (1 µg), followed by plating the cells on a 24-well dish at 1×10⁵ cells/ml, and adding ganciclovir (Fujifilm Wako Pure Chemical Corporation) to the medium to a final concentration of 500 nM. After 96 hours of culture, the cell survival rate was measured using CellTiter-Glo (CellTiter-Glo (registered trademark) Luminescent Cell Viability Assay, Promega).

Evaluation of the cytocidal effect on HCT116 cells (the wild-type line and Mlh1 revertant cells thereof) was carried out as follows. On a 24-well dish, 5×10⁴ cells were plated and cultured overnight, followed by transfecting the cells with each construct (1 µg). After the gene transfection, the cells were cultured for 24 hours, and then transfected again with each construct (1 µg), followed by adding ganciclovir (Fujifilm Wako Pure Chemical Corporation) to the medium to a final concentration of 1 µM. After 72 hours of culture, the cell survival rate was measured using CellTiter-Glo (CellTiter-Glo (registered trademark) Luminescent Cell Viability Assay, Promega).

### E-I-3. Results

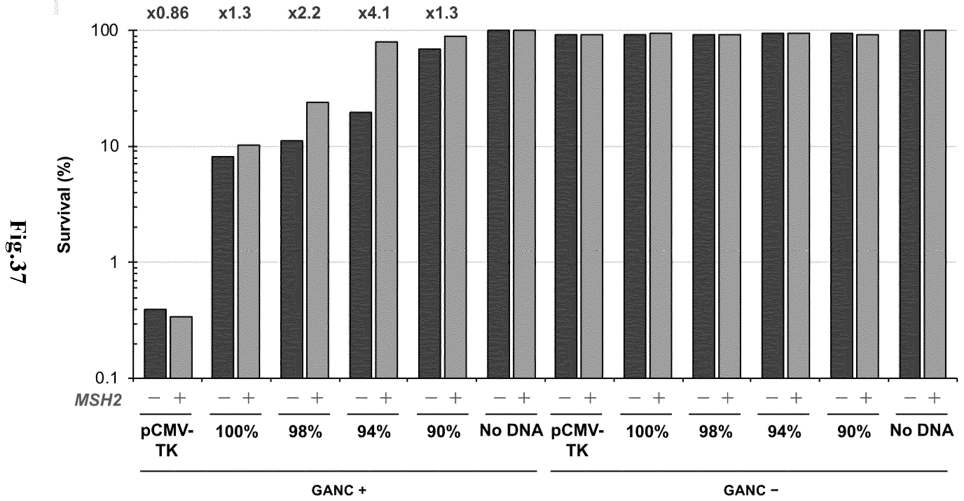

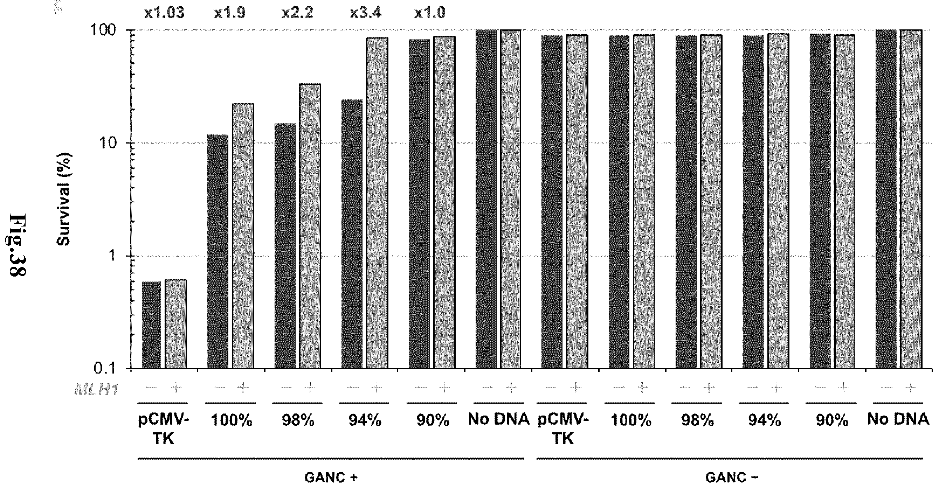

Fig. 37 shows the results of comparison of the survival rate 96 hours after introduction of the constructs, between Nalm-6 cells and MSH2 revertant cells thereof; and Fig. 38 shows the results of comparison of the survival rate 72 hours after introduction of the constructs, between HCT116 cells and MLH1 revertant cells thereof (expressed as relative values calculated by taking, as 100%, the survival rate of cells not transfected with DNA (No DNA) after culture in the presence of ganciclovir (GANC)). In both cases, the constructs having homologous regions with decreased homologies of 98% and 94% showed differences in the survival rate depending on the presence or absence of the MMR factor. In particular, in the case where the construct with a homology of 94% was introduced, the construct hardly showed toxicity to the normal-MMR cells (MSH2 revertant cells (Nalm-6 cells) and MLH1 revertant cells (HCT 116 cells)), demonstrating that the construct of the present invention can selectively kill MMR-deficient cells (WT Nalm-6 cells and WT HCT116 cells).

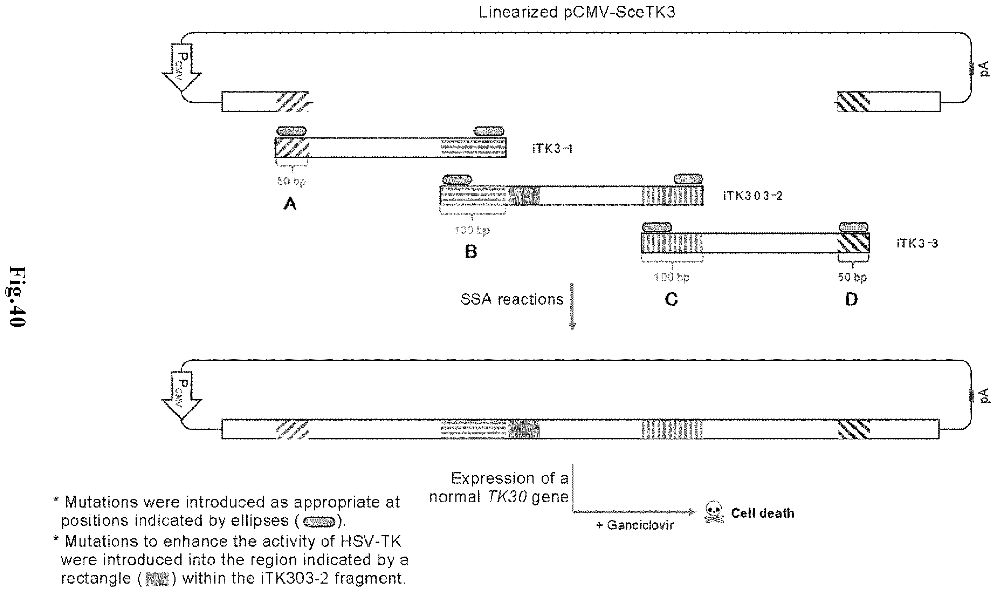

### Example E-II: Divided-Type SA-TK30 v3 (Four-Molecule, Four-SSA-Reaction Type) (Fig. 40)

The *TK30* gene is a mutant of the *HSV-TK* gene, prepared by introducing mutations of amino acids near the active center of the *HSV-TK* gene so as to enhance the action on ganciclovir. The *TK30* gene exhibits a stronger cytotoxicity than that of the wild-type HSV-TK (Kokoris MS et al. Gene Ther. 1999, Aug; 6(8): 1415-1426.). In the present Example, constructs of the present invention were prepared using the *TK30* gene as a suicide gene, and their cytocidal effects were evaluated.

### E-II-1. Preparation of Vectors

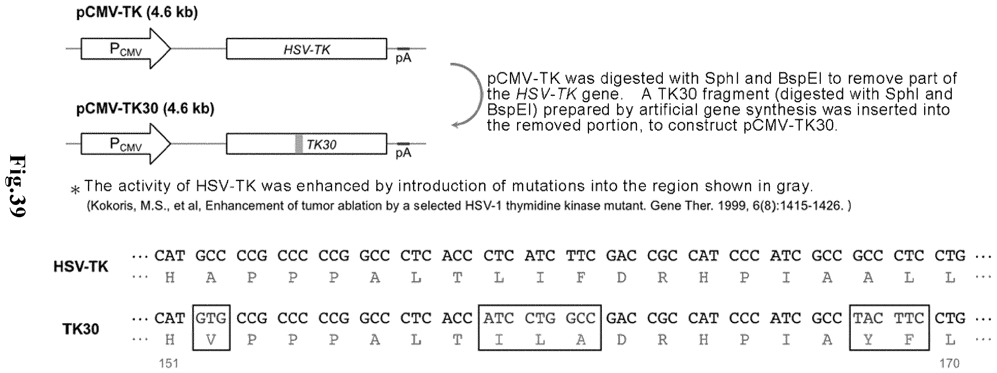

In order to prepare a construct pCMV-TK30 that expresses the *TK30* gene, mutations were introduced into codons in the *HSV-TK* gene such that the alanine at position 152 was substituted with valine; the leucine at position 159 was substituted with isoleucine; the isoleucine at position 160 was substituted with leucine; the phenylalanine at position 161 was substituted with alanine; the alanine at position 168 was substituted with tyrosine; and the leucine at position 169 was substituted with phenylalanine (Fig. 39). As an *HSV-TK* gene fragment containing these mutations (*TK30* gene, SEQ ID NO: 179), the portion from the SphI recognition sequence site (position 386 in the *HSV-TK* gene), located 5'-upstream of the mutation sites, to the BspEI recognition sequence site (position 629 in the *HSV-TK* gene), located 3'-downstream of the mutation sites, was prepared by artificial gene synthesis (pTK30). The prepared pTK30 was digested with SphI and BspEI, and a fragment containing the *TK30* gene was recovered. Subsequently, pCMV-TK was digested with SphI and BspEI to remove positions 391 to 624 in the *HSV-TK* gene, and a fragment containing the CMV promoter, the poly-A addition signal, and part of the *HSV-TK* gene was recovered, followed by inserting the TK30 fragment thereto using a DNA Ligation Kit <Mighty Mix> (Takara Bio Inc.), to obtain a vector that expresses TK30, in which [CMV promoter] - [TK30 fragment] - [poly-A addition signal] were linked together (pCMV-TK30). The amino acid sequence of the TK30 protein encoded by the TK30 fragment (SEQ ID NO:179) is shown in SEQ ID NO:180.

### pCMV-SceTK3, in which [CMV promoter] - [SceTK3 fragment] - [poly-A addition signal] were linked together, was prepared as described in E-I-1.

A 362-bp iTK3-1-100 fragment (the region of positions 87 to 448 in the *TK30* gene sequence; SEQ ID NO:152), a 412-bp iTK303-2-100 fragment (the region of positions 349 to 760 in the *TK30* gene sequence; SEQ ID NO:181), and a 358-bp iTK3-3-100 fragment (the region of positions 661 to 1018 in the *TK30* gene sequence; SEQ ID NO:154) were obtained by PCR amplification using pCMV-TK30 as a template. As a polymerase for the PCR, Tks Gflex (trade name) DNA Polymerase (Takara Bio Inc.) was used. For the amplification of the iTK3-1-100 fragment, iTK3-1-100-Fw (SEQ ID NO:124) and iTK3-1-100-Rv (SEQ ID NO:125) were used. The 50-bp base sequence in the 5'-end region of the iTK3-1-100 fragment amplified by this PCR has a homology of 100% to the 50 bp in the 3'-end region of pCMV-SceTK3 digested with I-SceI, and the 100-bp base sequence in the 3'-end region of the iTK3-1-100 fragment has a homology of 100% to the base sequence in the 5'-end region of the iTK303-2-100 fragment. For the amplification of the iTK303-2-100 fragment, iTK3-2-100-Fw (SEQ ID NO:126) and iTK3-2-100-Rv (SEQ ID NO:127) were used. The 100-bp base sequence in the 5'-end region of the iTK303-2-100 fragment amplified by this PCR has a homology of 100% to the base sequence in the 3'-end region of the iTK3-1-100 fragment, and the 100-bp base sequence in the 3'-end region of the iTK303-2-100 fragment has a homology of 100% to the base sequence of the 5'-end region of the iTK3-3-100 fragment. For the amplification of the iTK3-3-100 fragment, iTK3-3-100-Fw (SEQ ID NO:128) and iTK3-3-100-Rv (SEQ ID NO:129) were used. The 100-bp base sequence in the 5'-end region of the iTK3-3-100 fragment amplified by this PCR has a homology of 100% to the base sequence in the 3'-end region of the iTK303-2-100 fragment, and the 50-bp base sequence in the 3'-end region of the iTK3-3-100 fragment has a homology of 100% to the 50 bp in the 5'-end region of pCMV-SceTK3 digested with I-SceI (Fig. 40).

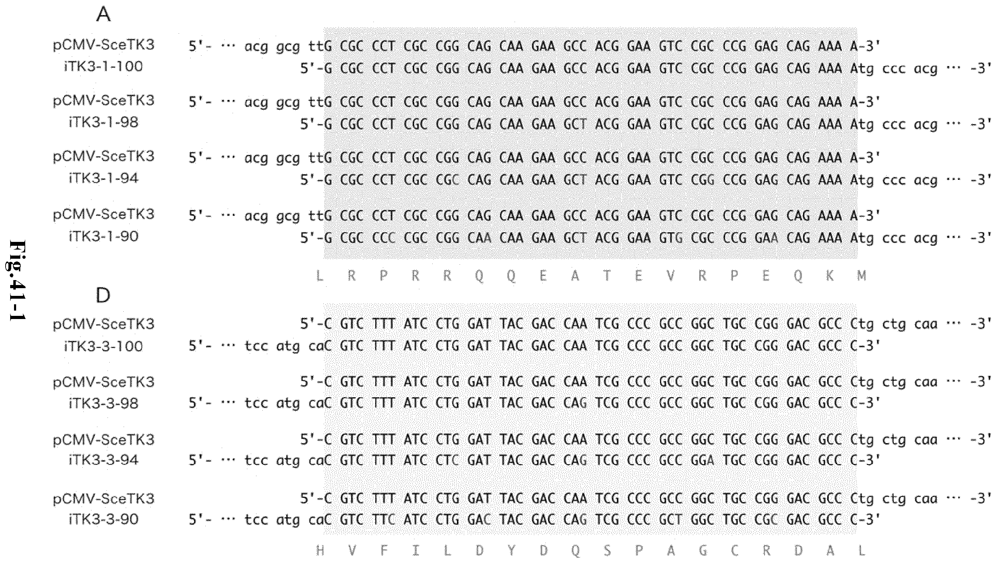

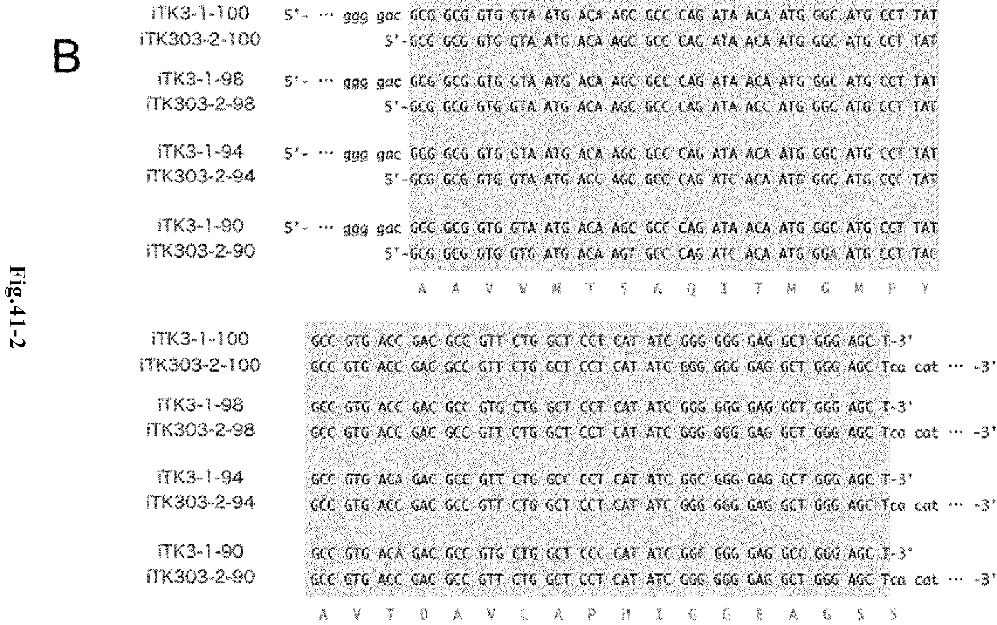

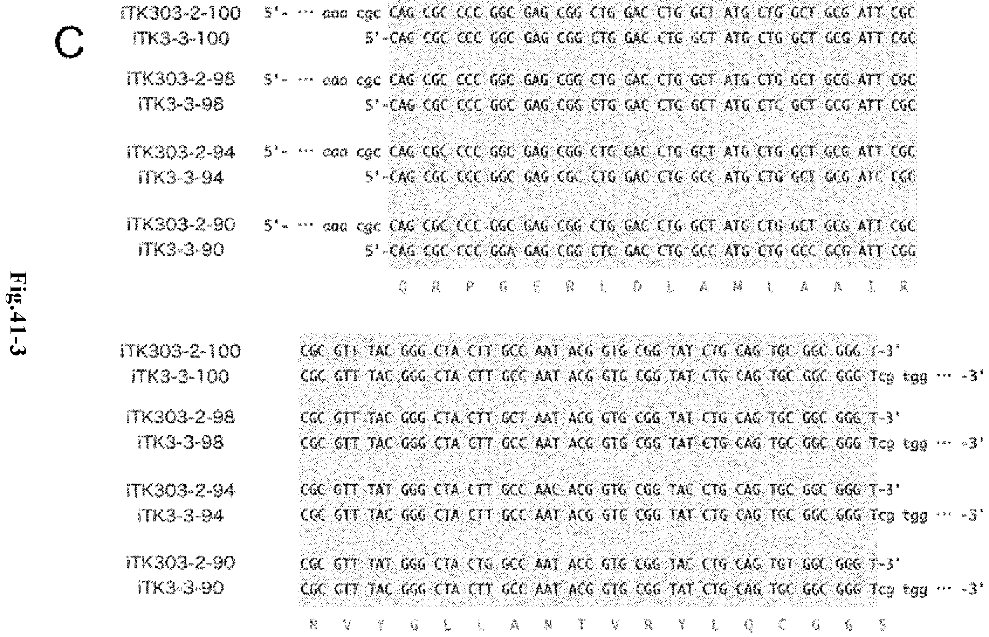

iTK3-1 fragments, iTK303-2 fragments, and iTK3-3 fragments having decreased homologies between the homologous sequences were obtained by PCR amplification using the *HSV-TK* gene fragment as a template. As a polymerase for the PCR, Tks Gflex (trade name) DNA Polymerase (Takara Bio Inc.) was used. Silent mutations were introduced such that the homology was 98% (1 site for a homologous region with a length of 50 bp, or 2 sites for a homologous region with a length of 100 bp), 94% (3 sites for a homologous region with a length of 50 bp, or 6 sites for a homologous region with a length of 100 bp), or 90% (5 sites for a homologous region with a length of 50 bp, or 10 sites for a homologous region with a length of 100 bp) (Fig. 41-1 to Fig. 41-3). The primers used are shown in Table 6 above. iTK3-1-98-Fw (SEQ ID NO:130) and iTK3-1-98-Rv (SEQ ID NO:131) were used for the amplification of an iTK3-1-98 fragment (98% homology, SEQ ID NO:155); iTK3-1-94-Fw (SEQ ID NO:132) and iTK3-1-94-Rv (SEQ ID NO:133) were used for the amplification of an iTK3-1-94 fragment (94% homology, SEQ ID NO:156); and iTK3-1-90-Fw (SEQ ID NO:134) and iTK3-1-90-Rv (SEQ ID NO:135) were used for the amplification of an iTK3-1-90 fragment (90% homology, SEQ ID NO:157). iTK3-2-98-Fw (SEQ ID NO:136) and iTK3-2-98-Rv (SEQ ID NO:137) were used for the amplification of an iTK303-2-98 fragment (98% homology, SEQ ID NO:182); iTK3-2-94-Fw (SEQ ID NO:138) and iTK3-2-94-Rv (SEQ ID NO:139) were used for the amplification of an iTK303-2-94 fragment (94% homology, SEQ ID NO:183); and iTK3-2-90-Fw (SEQ ID NO:140) and iTK3-2-90-Rv (SEQ ID NO:141) were used for the amplification of an iTK303-2-90 fragment (90% homology, SEQ ID NO:184). iTK3-3-98-Fw (SEQ ID NO:142) and iTK3-3-98-Rv (SEQ ID NO:143) were used for the amplification of an iTK3-3-98 fragment (98% homology, SEQ ID NO:161); iTK3-3-94-Fw (SEQ ID NO:144) and iTK3-3-94-Rv (SEQ ID NO:145) were used for the amplification of an iTK3-3-94 fragment (94% homology, SEQ ID NO:162); and iTK3-3-90-Fw (SEQ ID NO:146) and iTK3-3-90-Rv (SEQ ID NO:147) were used for the amplification of an iTK3-3-90 fragment (90% homology, SEQ ID NO:163).

The pCMV-SceTK3 was purified using a Qiagen Plasmid Plus Midi Kit (Qiagen K.K.), and linearized by cleavage with I-SceI (New England Biolabs, Ipswich, MA, USA) before use in transfection. The iTK3-1-100 fragment, iTK303-2-100 fragment, iTK3-3-100 fragment, iTK3-1-98 fragment, iTK3-1-94 fragment, iTK3-1-90 fragment, iTK303-2-98 fragment, iTK303-2-94 fragment, iTK303-2-90 fragment, iTK3-3-98 fragment, iTK3-3-94 fragment, and iTK3-3-90 fragment were purified with the Wizard SV Gel and PCR Clean-Up System (Promega) before use in transfection.

### E-II-2. Cells and Gene Transfection, and Evaluation of Cytocidal Effect

For the cells and the gene transfection, see A-I-2. above.

For the evaluation of the cytocidal effect, see E-I-2. above.

### E-II-3. Results

The four-molecule, four-SSA-reaction type constructs prepared in the present Example were designed such that a normal *TK30* gene (modified *HSV-TK* gene) is generated when single-strand annealing (SSA) occurs at the following four sites: between the 3'-side region in the SceTK3 fragment and the 5'-side region in the iTK3-1 fragment; between the 3'-side region in the iTK3-1 fragment and the 5'-side region in the iTK303-2 fragment; between the 3'-side region in the iTK303-2 fragment and the 5'-side region in the iTK3-3 fragment; and between the 3'-side region in the iTK3-3 fragment and the 5'-side region in the SceTK3 fragment (Fig. 40).

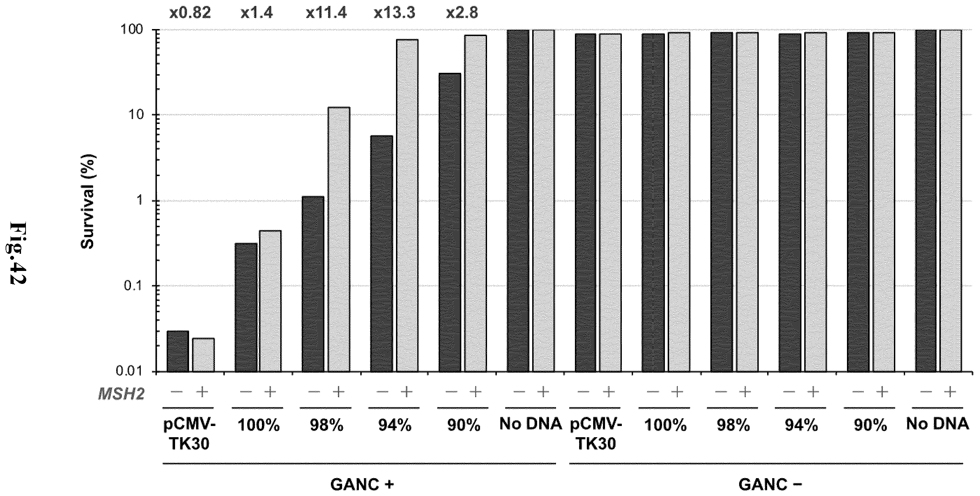

Fig. 42 shows the results of comparison of the survival rate 96 hours after introduction of the constructs prepared in the present Example (Fig. 40) into Nalm-6 cells and MSH2 revertant cells thereof (the survival rate was expressed as a relative value calculated by taking, as 100%, the survival rate of non-introduced cells (No DNA) after culture in the presence of ganciclovir (GANC)). The constructs having homologous regions with decreased homologies of 98%, 94%, and 90% showed differences in the survival rate depending on the presence or absence of MSH2. In particular, when the construct with a homology of 94% was introduced, the construct hardly showed toxicity to the MSH2 revertant cells, resulting in an about 13-fold difference in the survival rate.

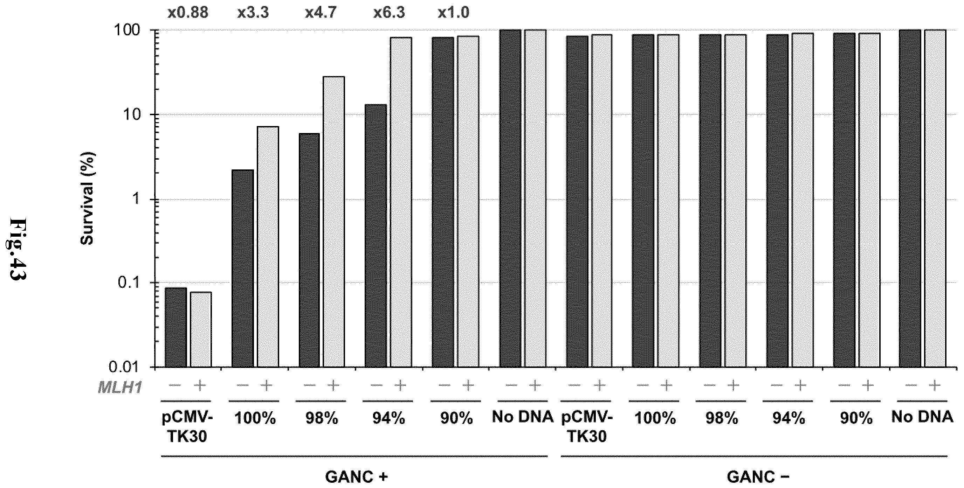

Fig. 43 shows the results of comparison of the survival rate 72 hours after introduction of the constructs prepared in the present Example (Fig. 40) into HCT 116 cells and MLH1 revertant cells thereof. It could be confirmed that, when the homologous regions have decreased homology, the difference in the survival rate increases depending not only on the presence or absence of MSH2, but also on the presence or absence of MLH1.

From these results, the four-molecule, four-SSA-reaction type constructs were found to be capable of selectively killing the MMR-deficient cells (WT Nalm-6 cells and WT HCT116 cells) also in the case where TK30 is used as a suicide gene.

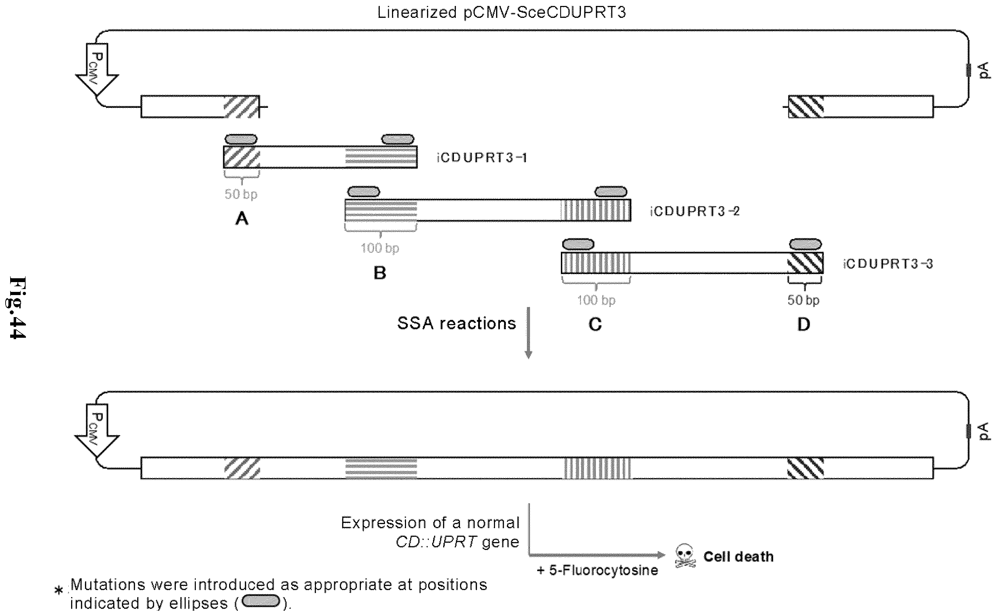

### Example E-III: Divided-Type SA-CDUPRT v3 (Four-Molecule, Four-SSA-Reaction Type) (Fig. 44)

In the present Example, the *CD::UPRT* gene was used as a gene encoding a protein that acts to decrease the survival rate of cells. The *CD::UPRT* gene is a fusion gene of the *Fcy1* gene (which encodes CD (cytosine deaminase)) derived from budding yeast and the *Fur1* gene (which encodes UPRT (uracil phosphoribosyl transferase) derived from budding yeast. CD converts 5-fluorocytosine (5-FC) to 5-fluorouracil by deamination. 5-Fluorouracil is intracellularly metabolized to become a substance that inhibits DNA synthesis and RNA synthesis, thereby inducing cell death (Austin EA and Huber BE. Mol Pharmacol. 1993 Mar; 43(3): 380-387.). UPRT is known to catalyze a metabolic pathway that converts 5-fluorouracil to a toxic substance in the cell. By using UPRT in combination with CD, a cytotoxicity-enhancing effect and a bystander effect due to 5-fluorocytosine can be obtained (Tiraby M et al. FEMS Microbiol Lett. 1998 Oct 1; 167(1): 41-49., Bourbeau D et al. J Gene Med. 2004 Dec; 6(12): 1320-1332.).

### E-III-1. Preparation of Vectors

In order to prepare a construct pCMV-CDUPRT that expresses the fusion gene (*CD::UPRT* gene) of the *Fcy1* gene (CD) derived from budding yeast and the *Fur1* gene (UPRT) derived from budding yeast, the codons of the *CD::UPRT* gene were optimized for use in human cells, according to a past report (Ho YK et al. Sci Rep. 2020 Aug 31; 10(1): 14257). Further, in order to perform cloning using restriction enzymes, an XhoI recognition sequence was added 5'-upstream, and an XbaI recognition sequence was added 3'-downstream, of the *CD::UPRT* gene. The sequence of the *CD::UPRT* gene (SEQ ID NO: 185; which encodes a fusion protein (SEQ ID NO: 186) having a structure in which the full-length CD is linked to the 3rd to 216th residues of UPRT through one alanine residue) was prepared by artificial gene synthesis (pCDUPRT). The prepared pCDUPRT was digested with XhoI and XbaI, and a fragment containing the *CD::UPRT* gene was recovered. Subsequently, pIRES (Takara Bio Inc.) was digested with XhoI and XbaI to remove the IRES region, and a fragment containing the CMV promoter and the poly-A addition signal was recovered, followed by inserting the CD::UPRT fragment thereto using a DNA Ligation Kit <Mighty Mix> (Takara Bio Inc.), to obtain a CD::UPRT expression vector, in which [CMV promoter] - [CD::UPRT fragment] - [poly-A addition signal] were linked together (pCMV-CDUPRT).

A 400-bp SceCDUPRT3 fragment (a DNA having a structure in which the region of positions 169 to 917 in the *CD::UPRT* gene sequence was replaced with "stop codon (TGA) + cgcg + I-SceI recognition sequence", having a stop codon added to the 3'-end; SEQ ID NO: 187) to be used for pCMV-SceCDUPRT3 was obtained by PCR amplification using pCMV-CDUPRT as a template. As a polymerase for the PCR, PrimeSTAR HS (trade name) DNA Polymerase (Takara Bio Inc.) was used. The primers used are shown in Table 7 below. The SceCDUPRT3 fragment was prepared by separately amplifying two fragments: a 5'SceCDUPRT3 fragment and a 3'SceCDUPRT3 fragment. In the amplification of the 5'SceCDUPRT fragment, Sce-5'CDUPRT-Fw (SEQ ID NO:189) and Sce-5'CDUPRT3-Rv (SEQ ID NO:190) were used to amplify a DNA fragment having a structure in which a sequence for In-Fusion reaction was added to the 5'-end of the 5'SceCDUPRT3 fragment, and in which "stop codon (TGA) + cgcg + I-SceI recognition sequence" was added to the 3'-end of the 5'SceCDUPRT3 fragment. In the amplification of the 3'SceCDUPRT3 fragment, Sce-3'CDUPRT3-Fw (SEQ ID NO:191) and Sce-3'CDUPRT-Rv (SEQ ID NO:192) were used to amplify a DNA fragment having a structure in which "I-SceI recognition sequence" was added to the 5'-end of the 3'SceCDUPRT3 fragment, and in which a sequence for In-Fusion reaction was added to the 3'-end of the 3'SceCDUPRT3 fragment. Subsequently, pIRES (Takara Bio Inc.) was digested with NheI and BamHI to remove the IRES region and the poly-A addition signal, and a fragment containing the CMV promoter was recovered. Using an In-Fusion Snap Assembly Master Mix (Takara Bio Inc.; the former product name, In-Fusion HD Cloning Kit), the 5'SceCDUPRT3 fragment and the 3'SceCDUPRT3 fragment were inserted downstream of the CMV promoter in the recovered fragment, to obtain pCMV-SceCDUPRT3, in which [CMV promoter] - [SceCDUPRT3 fragment] - [poly-A addition signal] were linked together. In the reading frame of the SceCDUPRT3 fragment, there is the stop codon TGA introduced upstream of the I-SceI recognition sequence, and in addition, there are a large number of stop codons downstream thereof generated due to occurrence of frameshift. The amino acid sequence encoded by positions 1 to 168 (the region up to the first stop codon) of the SceCDUPRT3 fragment (SEQ ID NO:187) is shown in SEQ ID NO:188.

Subsequently, a 256-bp iCDUPRT3-1-100 fragment (the region of positions 119 to 374 in the *CD::UPRT* gene sequence; SEQ ID NO:217), a 399-bp iCDUPRT3-2-100 fragment (the region of positions 275 to 673 in the *CD::UPRT* gene sequence; SEQ ID NO:218), and a 394-bp iCDUPRT3-3-100 fragment (the region of positions 574 to 967 in the *CD::UPRT* gene sequence; SEQ ID NO:219) were obtained by PCR amplification using pCMV-CDUPRT as a template. As a polymerase for the PCR, Tks Gflex (trade name) DNA Polymerase (Takara Bio Inc.) was used. For the amplification of the iCDUPRT3-1-100 fragment (SEQ ID NO:217), iCDUPRT3-1-100-Fw (SEQ ID NO:193) and iCDUPRT3-1-100-Rv (SEQ ID NO:194) were used. The 50-bp base sequence in the 5'-end region of the iCDUPRT3-1-100 fragment amplified by this PCR has a homology of 100% to the 100 bp in the 3'-end region of pCMV-SceCDUPRT3 digested with I-SceI, and the 100-bp base sequence in the 3'-end region of the iCDUPRT3-1-100 fragment has a homology of 100% to the base sequence in the 5'-end region of the iCDUPRT3-2-100 fragment. For the amplification of the iCDUPRT3-2-100 fragment (SEQ ID NO:218), iCDUPRT3-2-100-Fw (SEQ ID NO:195) and iCDUPRT3-2-100-Rv (SEQ ID NO:196) were used. The 100-bp base sequence in the 5'-end region of the iCDUPRT3-2-100 fragment amplified by this PCR has a homology of 100% to the base sequence in the 3'-end region of the iCDUPRT3-1-100 fragment, and the 100-bp base sequence in the 3'-end region of the iCDUPRT3-2-100 fragment has a homology of 100% to the base sequence in the 5'-end region of the iCDUPRT3-3-100 fragment. For the amplification of the iCDUPRT3-3-100 fragment (SEQ ID NO:219), iCDUPRT3-3-100-Fw (SEQ ID NO:197) and iCDUPRT3-3-100-Rv (SEQ ID NO:198) were used. The 100-bp base sequence in the 5'-end region of the iCDUPRT3-3-100 fragment amplified by this PCR has a homology of 100% to the base sequence in the 3'-end region of the iCDUPRT3-2-100 fragment, and the 50-bp base sequence in the 3'-end region of the iCDUPRT3-3-100 fragment has a homology of 100% to the 100 bp of the 5'-end region of pCMV-SceCDUPRT3 digested with I-SceI (Fig. 44).

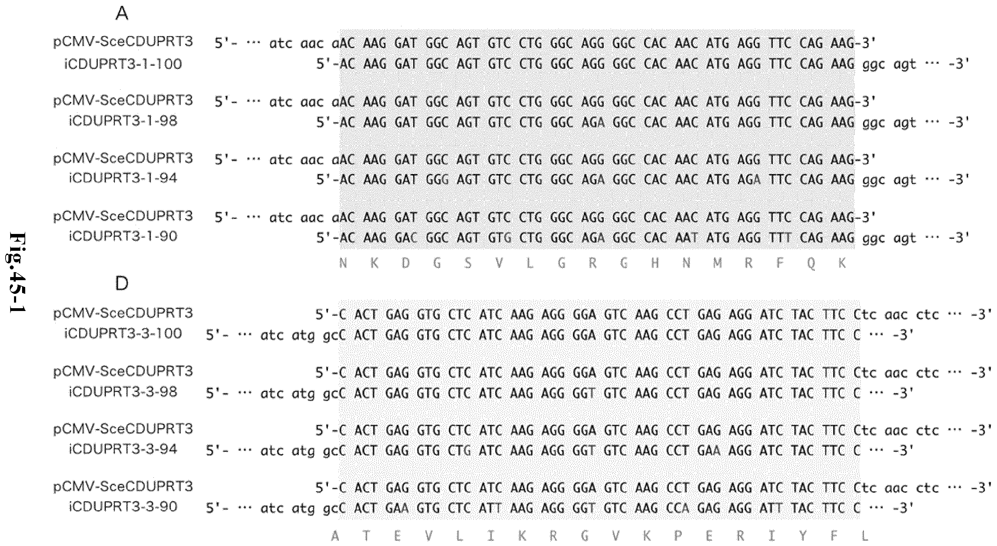

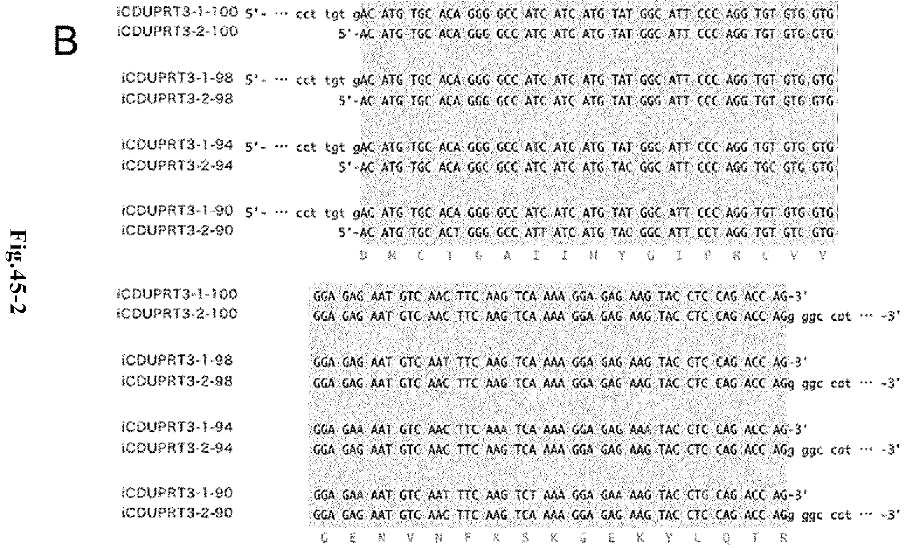

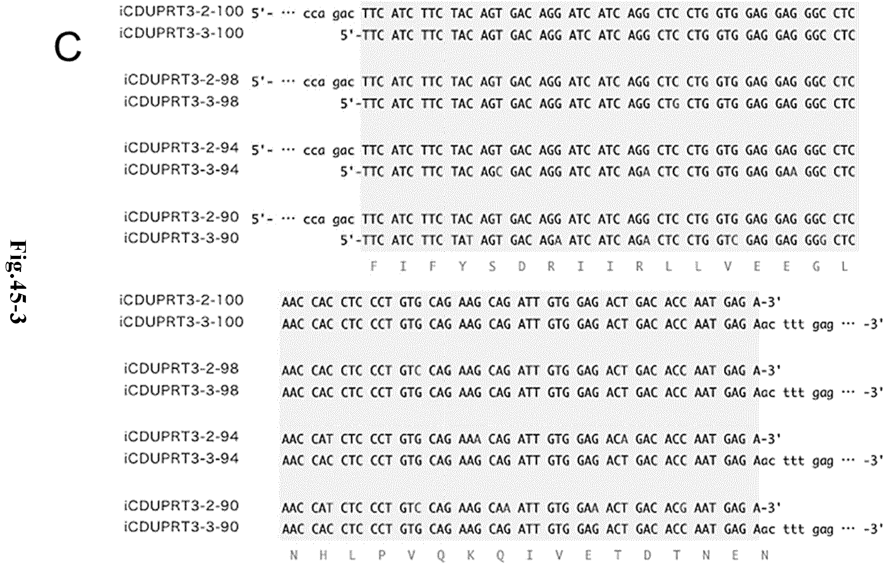

iCDUPRT3-1 fragments, iCDUPRT3-2 fragments, and iCDUPRT3-3 fragments having decreased homologies between the homologous sequences were obtained by PCR amplification using pCMV-CDUPRT as a template. As a polymerase for the PCR, Tks Gflex (trade name) DNA Polymerase (Takara Bio Inc.) was used. Silent mutations were introduced such that the homology was 98% (1 site for a homologous region with a length of 50 bp, or 2 sites for a homologous region with a length of 100 bp), 94% (3 sites for a homologous region with a length of 50 bp, or 6 sites for a homologous region with a length of 100 bp), or 90% (5 sites for a homologous region with a length of 50 bp, or 10 sites for a homologous region with a length of 100 bp (Fig. 45-1 to Fig. 45-3). The primers used are shown in Table 7 below. iCDUPRT3-1-98-Fw (SEQ ID NO:199) and iCDUPRT3-1-98-Rv (SEQ ID NO:200) were used for the amplification of an iCDUPRT3-1-98 fragment (98% homology, SEQ ID NO:220); iCDUPRT3-1-94-Fw (SEQ ID NO:201) and iCDUPRT3-1-94-Rv (SEQ ID NO:202) were used for the amplification of an iCDUPRT3-1-94 fragment (94% homology, SEQ ID NO:221); and iCDUPRT3-1-90-Fw (SEQ ID NO:203) and iCDUPRT3-1-90-Rv (SEQ ID NO:204) were used for the amplification of an iCDUPRT3-1-90 fragment (90% homology, SEQ ID NO:222). iCDUPRT3-2-98-Fw (SEQ ID NO:205) and iCDUPRT3-2-98-Rv (SEQ ID NO:206) were used for the amplification of an iCDUPRT3-2-98 fragment (98% homology, SEQ ID NO:223); iCDUPRT3-2-94-Fw (SEQ ID NO:207) and iCDUPRT3-2-94-Rv (SEQ ID NO:208) were used for the amplification of an iCDUPRT3-2-94 fragment (94% homology, SEQ ID NO:224); and iCDUPRT3-2-90-Fw (SEQ ID NO:209) and iCDUPRT3-2-90-Rv (SEQ ID NO:210) were used for the amplification of an iCDUPRT3-2-90 fragment (90% homology, SEQ ID NO:225). iCDUPRT3-3-98-Fw (SEQ ID NO:211) and iCDUPRT3-3-98-Rv (SEQ ID NO:212) were used for the amplification of an iCDUPRT3-3-98 fragment (98% homology, SEQ ID NO:226); iCDUPRT3-3-94-Fw (SEQ ID NO:213) and iCDUPRT3-3-94-Rv (SEQ ID NO:214) were used for the amplification of an iCDUPRT3-3-94 fragment (94% homology, SEQ ID NO:227); and iCDUPRT3-3-90-Fw (SEQ ID NO:215) and iCDUPRT3-3-90-Rv (SEQ ID NO:216) were used for the amplification of an iCDUPRT3-3-90 fragment (90% homology, SEQ ID NO:228).

The pCMV-SceCDUPRT3 was purified using a Qiagen Plasmid Plus Midi Kit (Qiagen K.K.), and linearized by cleavage with I-SceI (New England Biolabs, Ipswich, MA, USA) before use in transfection. The iCDUPRT3-1-100 fragment, iCDUPRT3-2-100 fragment, iCDUPRT3-3-100 fragment, iCDUPRT3-1-98 fragment, iCDUPRT3-1-94 fragment, iCDUPRT3-1-90 fragment, iCDUPRT3-2-98 fragment, iCDUPRT3-2-94 fragment, iCDUPRT3-2-90 fragment, iCDUPRT3-3-98 fragment, iCDUPRT3-3-94 fragment, and iCDUPRT3-3-90 fragment were purified with the Wizard SV Gel and PCR Clean-Up System (Promega) before use in transfection.

**[Table 7]**

| Primer name | Sequence (5'→3') | SEQ ID NO |
|---|---|---|
| Sce-5'CDUPRT-Fw | TAATACGACTCACTATAGGCTAG | 189 |
| Sce-5'CDUPRT3-Rv | GCATTACCCTGTTATCCCTACGCG**TCA**CTTCTGGAACCTCATGTTGTGG | 190 |
| Sce-3'CDUPRT3-Fw | GTAGGGATAACAGGGTAATGCATACACTGAGGTGCTCATCAAGAGG | 191 |
| Sce-3'CDUPRT-Rv | GAGTGCACCATACGCGGATC | 192 |
| iCDUPRT3-1-100-Fw | ACAAGGATGGCAGTGTCCTG | 193 |
| iCDUPRT3-1-100-Rv | CTGGTCTGGAGGTACTTCTC | 194 |
| iCDUPRT3-2-100-Fw | ACATGTGCACAGGGGCCATC | 195 |
| iCDUPRT3-2-100-Rv | TCTCATTGGTGTCAGTCTCC | 196 |
| iCDUPRT3-3-100-Fw | TTCATCTTCTACAGTGACAGG | 197 |
| iCDUPRT3-3-100-Rv | GGAAGTAGATCCTCTCAGG | 198 |
| iCDUPRT3-1-98-Fw | ACAAGGATGGCAGTGTCCTGGGCAGAGGCCAC | 199 |
| iCDUPRT3-1-98-Rv | CTGGTCTGGAGGTACTTCTCTCCTTTTGACTTGAAATTGACATTC | 200 |
| iCDUPRT3-1-94-Fw | ACAAGGATGGGAGTGTCCTGGGCAGAGGCCACAACATGAGATTCCAG | 201 |
| iCDUPRT3-1-94-Rv | CTGGTCTGGAGGTATTTCTCTCCTTTTGATTTGAAGTTGACATTTTCTCCC | 202 |
| iCOUPRT3-1-90-Fw | ACAAGGACGGCAGTGTGCTGGGCAGAGGCCACAATATGAGGTTTCAGAAG | 203 |
| iCDUPRT3-1-90-Rv | CTGGTCTGCAGGTACTTTTCTCCTTTAGACTTGAAATTGACATTTTCTCCC | 204 |
| iCDUPRT3-2-98-Fw | ACATGTGCACAGGGGCCATCATCATGTATGGGATTCCCAG | 205 |
| iCDUPRT3-2-98-Rv | TCTCATTGGTGTCAGTCTCCACAATCTGCTTCTGGACAGGGAG | 206 |
| iCDUPRT3-2-94-Fw | ACATGTGCACAGGCGCCATCATCATGTACGGCATTCCCAGGTGCGTGGTGG | 207 |
| iCDUPRT3-2-94-Rv | TCTCATTGGTGTCTGTCTCCACAATCTGTTTCTGCACAGGGAGATGGTTGAGG | 208 |
| iCDUPRT3-2-90-Fw | ACATGTGCACTGGGGCCATTATCATGTACGGCATTCCTAGGTGTGTCGTGGGAG | 209 |
| iCDUPRT3-2-90-Rv | TCTCATTCGTGTCAGTTTCCACAATTTGCTTCTGGACAGGGAGATGGTTGAGG | 210 |
| iCDUPRT3-3-98-Fw | TTCATCTTCTACAGTGACAGGATCATCAGGCTGCTGGTG | 211 |
| iCOUPRT3-3-98-Rv | GGAAGTAGATCCTCTCAGGCTTGACACCCCTC | 212 |
| iCDUPRT3-3-94-Fw | TTCATCTTCTACAGCGACAGGATCATCAGACTCCTGGTGGAGGAAGGCCTC | 213 |
| iCDUPRT3-3-94-Rv | GGAAGTAGATCCTTTCAGGCTTGACACCCCTCTTGATCAGCACC | 214 |
| iCDUPRT3-3-90-Fw | TTCATCTTCTATAGTGACAGAATCATCAGACTCCTGGTCGAGGAGGGGCTCAACC | 215 |
| iCDUPRT3-3-90-Rv | GGAAGTAAATCCTCTCTGGCTTGACACCCCTCTTAATGAGCACTTCAGTG | 216 |

| | | |
|---|---|---|
| Single-underline: I-Scel recognition sequence Double-underline: A site that anneals to the target gene sequence (Sce-5'CDUPRT-Fw and Sce-3'CDUF Rv target vector sequences located upstream and downstream of a CDUPRT gene fragment, respectively Bold: Sequence for introduction of a stop codon (TGA) | | |

### E-III-2. Cells and Gene Transfection, and Evaluation of Cytocidal Effect

For the cells and the gene transfection, see A-I-2. above.

Evaluation of the cytocidal effect on Nalm-6 cells and Msh2 revertant cells thereof was carried out as follows. With each construct (1 µg), 2×10⁶ cells were transfected. The cells after the gene transfection were cultured for 48 hours in a medium containing 5-fluorocytosine (Fujifilm Wako Pure Chemical Corporation) at a final concentration of 100 µM, and then transfected again with each construct (1 µg). The cells after the gene transfection were plated on a 24-well dish at 1×10⁵ cells/ml, and 5-fluorocytosine was added to the medium to a final concentration of 100 µM. After 96 hours of culture, the cell survival rate was measured using CellTiter-Glo (CellTiter-Glo (registered trademark) Luminescent Cell Viability Assay, Promega).

Evaluation of the cytocidal effect on HCT116 cells and MLH1 revertant cells thereof (MLH1 + HCT116 cells) was carried out as follows. On a 24-well dish, 5×10⁴ cells were plated and cultured overnight, followed by transfecting the cells with each construct (1 µg). After the gene transfection, the cells were cultured for 24 hours, and then transfected again with each construct (1 µg), followed by adding 5-fluorocytosine to the medium to a final concentration of 100 µM. After 72 hours of culture, the cell survival rate was measured using CellTiter-Glo (CellTiter-Glo (registered trademark) Luminescent Cell Viability Assay, Promega).

### E-III-3. Results

The four-molecule, four-SSA-reaction type constructs prepared in the present Example were designed such that a normal *CD::UPRT* gene (a fusion gene of the *Fcy1* gene (CD) derived from budding yeast and the *Fur1* gene (UPRT) derived from budding yeast) is generated when single-strand annealing (SSA) occurs at the following four sites: between the 3'-side region in the SceCDUPRT3 fragment and the 5'-side region in the iCDUPRT3-1 fragment; between the 3'-side region in the iCDUPRT3-1 fragment and the 5'-side region in the iCDUPRT3-2 fragment; between the 3'-side region in the iCDUPRT3-2 fragment and the 5'-side region in the iCDUPRT3-3 fragment; and between the 3'-side region in the iCDUPRT3-3 fragment and the 5'-side region in the SceCDUPRT3 fragment (Fig. 44).

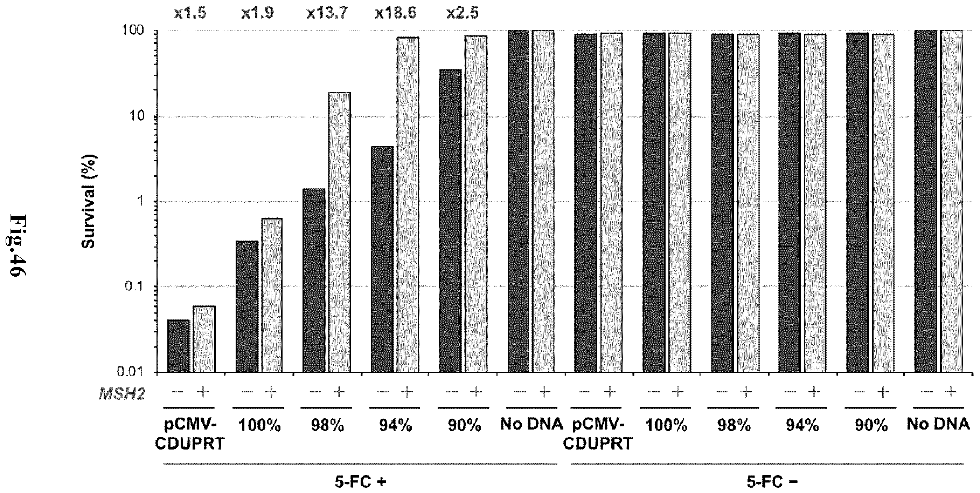

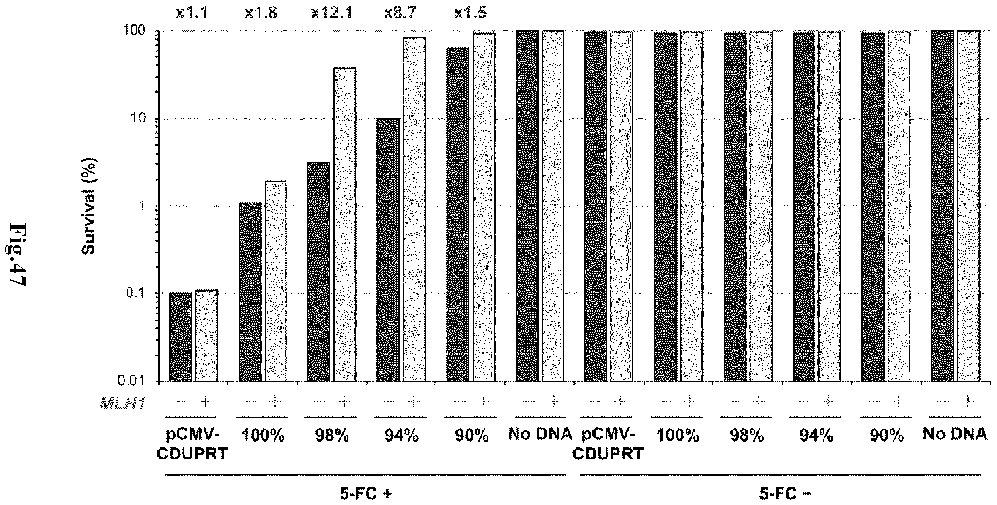

Fig. 46 shows the results of comparison of the survival rate 96 hours after the introduction of the constructs prepared in the present Example, between Nalm-6 cells and MSH2 revertant cells thereof. Fig. 47 shows the results of comparison of the survival rate 72 hours after the introduction of the constructs, between HCT 116 cells and MLH1 revertant cells thereof (each figure shows relative values calculated by taking, as 100%, the survival rate of non-introduced cells (No DNA), which were cultured in the presence of 5-FC (100 µM)). The constructs having homologous regions with decreased homologies were found to cause differences in the survival rate depending on the presence or absence of the MMR factor. In particular, in the case where the construct with a homology of 94% was introduced, the construct hardly showed toxicity to the normal-MMR cells (MSH2 revertant cells (Nalm-6 cells) and MLH1 revertant cells (HCT116 cells)). Thus, the four-molecule, four-SSA-reaction type constructs using *CD::UPRT* as a suicide gene were shown to be capable of selectively killing MMR-deficient cells (WT Nalm-6 cells and WT HCT116 cells).

## Claims

1. A nucleic acid construct comprising:
a promoter region;
a 5'-side region of a gene sequence encoding protein A;
a 3'-side region of a gene sequence encoding protein A; and
at least one complementary region comprising: a homologous region α for which a region comprising at least a 3'-end portion of the 5'-side region serves as a substrate in a recombination reaction by single-strand annealing; and a homologous region β for which a region comprising at least a 5'-end portion of the 3'-side region serves as a substrate in said recombination reaction;
the promoter region, the 5'-side region, the 3'-side region, and the at least one complementary region being placed in one nucleic acid molecule or in two or more different nucleic acid molecules,
wherein the homologous region α and the homologous region β are contained in the same complementary region or different complementary regions, and in the latter case, at least one complementary region comprises one or more additional sets of a homologous region and a substrate that undergo said recombination reaction,
wherein the homology between each homologous region and each region that serves as the substrate therefor is not less than 40% and less than 100%, and
wherein said recombination reaction forms a nucleic acid whose base sequence encodes protein A, or protein B which has the same activity as protein A, resulting in expression of protein A or protein B.

2. The nucleic acid construct according to claim 1, wherein the amino acid sequence of protein B has a sequence identity of not less than 80% and less than 100% to the amino acid sequence of protein A.

3. The nucleic acid construct according to claim 1, wherein the promoter region, the 5'-side region, and the 3'-side region are placed in the same nucleic acid molecule.

4. The nucleic acid construct according to claim 1, wherein the promoter region and the 5'-side region are placed in one nucleic acid molecule, and the 3'-side region is placed in another nucleic acid molecule.

5. The nucleic acid construct according to claim 1, wherein a poly-A addition signal is functionally linked downstream of the 3'-side region.

6. The nucleic acid construct according to claim 3, composed of two nucleic acid molecules and comprising one complementary region,
wherein:
a nucleic acid molecule 1 comprises the promoter region, the 5'-side region, and the 3'-side region;
a nucleic acid molecule 2 comprises a complementary region comprising the homologous region α and the homologous region β;
the homology between the homologous region α and the region that comprises at least the 3'-end portion of the 5'-side region and that serves as the substrate for the homologous region α in said recombination reaction is not less than 40% and less than 100%, and the homology between the homologous region β and the region that comprises at least the 5'-end portion of the 3'-side region and that serves as the substrate for the homologous region β in said recombination reaction is not less than 40% and less than 100%; and
the 5'-side region, the complementary region, and the 3'-side region, when arranged in this order, encode an amino acid sequence of protein A or protein B while each homologous region and the substrate therefor overlap each other, and the recombination reaction that occurs between each homologous region and the substrate therefor results in expression of protein A or protein B.

7. The nucleic acid construct according to claim 3, composed of *n* nucleic acid molecules and comprising (n-1) complementary regions,
wherein:
a nucleic acid molecule 1 comprises the promoter region, the 5'-side region, and the 3'-side region;
a nucleic acid molecule 2 comprises a first complementary region comprising a first homologous region and a second homologous region;
an *m*th nucleic acid molecule *m* comprises an (*m*-1)th complementary region comprising an (*m*-1)th substrate region and an *m*th homologous region;
the first homologous region is the homologous region α, and the homology between the first homologous region and the region that comprises at least the 3'-end portion of the 5'-side region and that serves as the substrate for the first homologous region in said recombination reaction is not less than 40% and less than 100%;
an (*m*-1)th homologous region is a region for which the (*m*-1)th substrate region serves as a substrate in said recombination reaction, and the homology between these regions is not less than 40% and less than 100%;
an nth homologous region contained in a nucleic acid molecule *n* is the homologous region β, and the homology between the *n*th homologous region and the region that comprises at least the 5'-end portion of the 3'-side region and that serves as the substrate for the *n*th homologous region in said recombination reaction is not less than 40% and less than 100%; and
the 5'-side region, the first complementary region, the (*m-*1)th complementary region, and the 3'-side region, when arranged in this order, encode an amino acid sequence of protein A or protein B while each homologous region and the substrate therefor overlap each other, and the recombination reaction that occurs between each homologous region and the substrate therefor results in expression of protein A or protein B
(wherein the integer *n* is a constant of 3 ≤ *n* ≤ 10, and the integer *m* is a variable of 3 ≤ *m ≤ n*).

8. The nucleic acid construct according to claim 6, wherein the nucleic acid molecule 1 is a linear nucleic acid molecule comprising, downstream of the 3'-side region, the promoter region and the 5'-side region in this order, or a circular nucleic acid molecule comprising, downstream of the promoter region, the 5'-side region and the 3'-side region in this order.

9. The nucleic acid construct according to claim 8, wherein the circular nucleic acid molecule comprises a cleavage site between the 5'-side region and the 3'-side region.

10. The nucleic acid construct according to claim 9, wherein the cleavage site is a restriction enzyme recognition site.

11. The nucleic acid construct according to claim 4, composed of three nucleic acid molecules and comprising one complementary region,
wherein:
a nucleic acid molecule 1-1 comprises the promoter region and the 5'-side region;
a nucleic acid molecule 1-2 comprises the 3'-side region;
a nucleic acid molecule 2 comprises a complementary region comprising the homologous region α and the homologous region β;
the homology between the homologous region α and the region that comprises at least the 3'-end portion of the 5'-side region and that serves as the substrate for the homologous region α in said recombination reaction is not less than 40% and less than 100%;
the homology between the homologous region β and the region that comprises at least the 5'-end portion of the 3'-side region and that serves as the substrate for the homologous region β in said recombination reaction is not less than 40% and less than 100%; and
the 5'-side region, the complementary region, and the 3'-side region, when arranged in this order, encode an amino acid sequence of protein A or protein B while each homologous region and the substrate therefor overlap each other, and the recombination reaction that occurs between each homologous region and the substrate therefor results in expression of protein A or protein B.

12. The nucleic acid construct according to claim 4, composed of (*n*+1) nucleic acid molecules and comprising (*n*-1) complementary regions,
wherein:
a nucleic acid molecule 1-1 comprises the promoter region and the 5'-side region;
a nucleic acid molecule 1-2 comprises the 3'-side region;
a nucleic acid molecule 2 comprises a first complementary region comprising a first homologous region and a second homologous region;
an *m*th nucleic acid molecule *m* comprises an (*m*-1)th complementary region comprising an (*m*-1)th substrate region and an *m*th homologous region;
the first homologous region is the homologous region α, and the homology between the first homologous region and the region that comprises at least the 3'-end portion of the 5'-side region and that serves as the substrate for the first homologous region in said recombination reaction is not less than 40% and less than 100%;
an (*m*-1)th homologous region is a region for which the (*m*-1)th substrate region serves as a substrate in said recombination reaction, and the homology between these regions is not less than 40% and less than 100%;
an *n*th homologous region contained in a nucleic acid molecule *n* is the homologous region β, and the homology between the *n*th homologous region and the region that comprises at least the 5'-end portion of the 3'-side region and that serves as the substrate for the *n*th homologous region in said recombination reaction is not less than 40% and less than 100%; and
the 5'-side region, the first complementary region, the (*m-*1)th complementary region, and the 3'-side region, when arranged in this order, encode an amino acid sequence of protein A or protein B while each homologous region and the substrate therefor overlap each other, and the recombination reaction that occurs between each homologous region and the substrate therefor results in expression of protein A or protein B
(wherein the integer *n* is a constant of 3 ≤ *n* ≤ 10, and the integer *m* is a variable of 3 ≤ *m* ≤ *n*).

13. The nucleic acid construct according to claim 3, composed of one circular nucleic acid molecule and comprising one complementary region,
wherein:
the circular nucleic acid molecule comprises, downstream of the promoter region, the 5'-side region, a cleavage site, and the 3'-side region in this order, and comprises, upstream of the promoter region but downstream of the 3'-side region, a complementary region comprising the homologous region α and the homologous region β;
the homology between the homologous region α and the region comprising at least the 3'-end portion of the 5'-side region which serves as a substrate for the homologous region α in said recombination reaction is not less than 40% and less than 100%;
the homology between the homologous region β and the region comprising at least the 5'-end portion of the 3'-side region which serves as a substrate for the homologous region β in said recombination reaction is not less than 40% and less than 100%;
the 5'-side region, the complementary region, and the 3'-side region, when arranged in this order, encode an amino acid sequence of protein A or protein B while each homologous region and the substrate therefor overlap each other, and the recombination reaction that occurs between each homologous region and the substrate therefor results in expression of protein A or protein B.

14. The nucleic acid construct according to claim 3, composed of one circular nucleic acid molecule and comprising (*n*-1) complementary regions,
wherein:
the circular nucleic acid molecule comprises, downstream of the promoter region, the 5'-side region, a cleavage site, and the 3'-side region in this order, and comprises, upstream of the promoter region but downstream of the 3'-side region, (*n-*1) complementary regions;
a first complementary region comprises a first homologous region and a second homologous region;
an (*m*-1)th complementary region comprises an (*m*-1)th substrate region and an mth homologous region;
the first homologous region is the homologous region α for which the region comprising at least the 3'-end portion of the 5'-side region serves as a substrate in said recombination reaction, and the homology between the substrate and the first homologous region is not less than 40% and less than 100%;
an (*m*-1)th homologous region is a region for which the (*m*-1)th substrate region serves as a substrate in said recombination reaction, and the homology between these regions is not less than 40% and less than 100%;
an *n*th homologous region contained in an (*n*-1)th complementary region is the homologous region β for which the region comprising at least the 5'-end portion of the 3'-side region serves as a substrate in said recombination reaction, and the homology between the substrate and the *n*th homologous region is not less than 40% and less than 100%;
the 5'-side region, the first complementary region, the (*m-*1)th complementary region, and the 3'-side region, when arranged in this order, encode an amino acid sequence of protein A or protein B while each homologous region and the substrate therefor overlap each other, and the recombination reaction that occurs between each homologous region and the substrate therefor results in expression of protein A or protein B
(wherein the integer *n* is a constant of 3 ≤ *n* ≤ 10, and the integer *m* is a variable of 3 ≤ *m ≤ n*).

15. The nucleic acid construct according to claim 13, wherein the cleavage site is a restriction enzyme recognition site.

16. The nucleic acid construct according to claim 3, composed of one linear nucleic acid molecule and comprising one complementary region,
wherein:
the linear nucleic acid molecule comprises, from upstream to downstream, the 3'-side region, the complementary region, the promoter region, and the 5'-side region in this order;
the complementary region comprises the homologous region α and the homologous region β;
the homology between the homologous region α and the region comprising at least the 3'-end portion of the 5'-side region which serves as a substrate for the homologous region α in said recombination reaction is not less than 40% and less than 100%;
the homology between the homologous region β and the region comprising at least the 5'-end portion of the 3'-side region which serves as a substrate for the homologous region β in said recombination reaction is not less than 40% and less than 100%;
the 5'-side region, the complementary region, and the 3'-side region, when arranged in this order, encode an amino acid sequence of protein A or protein B while each homologous region and the substrate therefor overlap each other, and the recombination reaction that occurs between each homologous region and the substrate therefor results in expression of protein A or protein B.

17. The nucleic acid construct according to claim 3, composed of one linear nucleic acid molecule and comprising (*n*-1) complementary regions,
wherein:
the linear nucleic acid molecule comprises, from upstream to downstream, the 3'-side region, the promoter region, and the 5'-side region in this order, and comprises, between the 3'-side region and the promoter region, (*n*-1) complementary regions;
a first complementary region comprises a first homologous region and a second homologous region;
an (*m*-1)th complementary region comprises an (*m*-1)th substrate region and an *m*th homologous region;
the first homologous region is the homologous region α for which the region comprising at least the 3'-end portion of the 5'-side region serves as a substrate in said recombination reaction, and the homology between the substrate and the first homologous region is not less than 40% and less than 100%;
an (*m*-1)th homologous region is a region for which the (*m*-1)th substrate region serves as a substrate in said recombination reaction, and the homology between these regions is not less than 40% and less than 100%;
an *n*th homologous region contained in an (*n*-1)th complementary region is the homologous region β for which the region comprising at least the 5'-end portion of the 3'-side region serves as a substrate in said recombination reaction, and the homology between the substrate and the *n*th homologous region is not less than 40% and less than 100%;
the 5'-side region, the first complementary region, the (*m-*1)th complementary region, and the 3'-side region, when arranged in this order, encode an amino acid sequence of protein A or protein B while each homologous region and the substrate therefor overlap each other, and the recombination reaction that occurs between each homologous region and the substrate therefor results in expression of protein A or protein B
(wherein the integer *n* is a constant of 3 ≤ *n* ≤ 10, and the integer *m* is a variable of 3 ≤ *m* ≤ *n*).

18. The nucleic acid construct according to claim 1, wherein the chain lengths of all homologous regions are at least 20 bases.

19. The nucleic acid construct according to claim 1, wherein the gene sequence encoding protein A is a gene sequence encoding a protein that acts to decrease cell survival rate, or a gene sequence encoding a protein whose intracellular expression is detectable.

20. The nucleic acid construct according to claim 19, wherein the gene sequence encoding protein A is a sequence of a suicide gene, a DNA damage-inducing gene, a DNA repair-inhibiting gene, a luminescent enzyme gene, a fluorescent protein gene, a cell surface antigen gene, a secretory protein gene, or a membrane protein gene.

21. A therapeutic agent for mismatch repair-deficient cancer, the agent comprising the nucleic acid construct according to any one of claims 1 to 20, wherein the gene sequence encoding protein A is a gene sequence encoding a protein that acts to decrease cell survival rate.

22. The therapeutic agent according to claim 21, wherein the gene sequence encoding protein A is a sequence of a suicide gene, a DNA damage-inducing gene, or a DNA repair-inhibiting gene.

23. A diagnostic agent for mismatch repair-deficient cancer, the agent comprising the nucleic acid construct according to any one of claims 1 to 20.

24. The diagnostic agent according to claim 23, wherein the gene sequence encoding protein A is a gene sequence encoding a protein whose intracellular expression is detectable.

25. A companion diagnostic agent for predicting an effect of an anticancer drug for mismatch repair-deficient cancer, the agent comprising the nucleic acid construct according to any one of claims 1 to 20.

26. The companion diagnostic agent according to claim 25, wherein the anticancer drug is an immune checkpoint inhibitor.

27. The companion diagnostic agent according to claim 25, wherein the gene sequence encoding protein A is a gene sequence encoding a protein whose intracellular expression is detectable.

28. A therapeutic method for mismatch repair-deficient cancer, the method comprising administering the nucleic acid construct according to any one of claims 1 to 20 to a patient with the mismatch repair-deficient cancer, wherein the gene sequence encoding protein A is a gene sequence encoding a protein that acts to decrease cell survival rate.

29. A diagnostic method for mismatch repair-deficient cancer, the method comprising:
introducing the nucleic acid construct according to any one of claims 1 to 20 into cancer cells of a cancer patient; and
measuring expression of protein A or protein B.

30. The method according to claim 29, wherein the expression of protein A or protein B is measured by directly or indirectly measuring the activity of protein A or protein B.

31. The method according to claim 29, wherein the cancer cells are cells separated from the cancer patient, and the introduction of the nucleic acid construct into the cancer cells is carried out *in vitro.*

32. The method according to claim 29, wherein: protein A or protein B is a protein whose intracellular expression is detectable as a signal; the introduction of the nucleic acid construct into the cancer cells is carried out by administering the nucleic acid construct to the cancer patient; and whether or not the signal of the protein is detected from a cancer lesion is investigated.

33. The method according to claim 29, wherein: protein A or protein B is a secretory protein whose intracellular expression is detectable; the introduction of the nucleic acid construct into the cancer cells is carried out by administering the nucleic acid construct to the cancer patient; and the activity of the protein in blood separated from the patient after the administration of the nucleic acid construct is measured.

34. A method of predicting an effect of an anticancer drug for mismatch repair-deficient cancer, the method comprising:
introducing the nucleic acid construct according to any one of claims 1 to 20 into cancer cells of a cancer patient; and
measuring expression of protein A or protein B.

35. The method according to claim 34, wherein the anticancer drug is an immune checkpoint inhibitor.

36. The method according to claim 34, wherein the cancer cells are cells separated from the patient, and the introduction of the nucleic acid construct into the cancer cells is carried out *in vitro.*

37. The method according to claim 34, wherein: protein A or protein B is a protein whose intracellular expression is detectable as a signal; the introduction of the nucleic acid construct into the cancer cells is carried out by administering the nucleic acid construct to the patient; and whether or not the signal of the protein is detected from a cancer lesion is investigated.

38. The method according to claim 34, wherein: protein A or protein B is a secretory protein whose intracellular expression is detectable; the introduction of the nucleic acid construct into the cancer cells is carried out by administering the nucleic acid construct to the cancer patient; and the activity of the protein in blood separated from the patient after the administration of the nucleic acid construct is measured.

39. A nucleic acid construct comprising:
a promoter region;
two substrate regions α and β;
a gene sequence encoding protein X; and
at least one complementary region comprising two homologous regions, wherein said two substrate regions serve as substrates for the two homologous regions, respectively, in a recombination reaction by single-strand annealing;
the promoter region, the two substrate regions, the gene sequence, and the at least one complementary region being placed in one nucleic acid molecule or in two or more different nucleic acid molecules,
wherein the two homologous regions are contained in the same complementary region or different complementary regions, and in the latter case, at least one complementary region comprises one or more additional sets of a homologous region and a substrate that undergo said recombination reaction,
wherein the homology between each substrate region and its corresponding homologous region is not less than 40% and less than 100%, and
wherein said recombination reaction results in expression of protein X.

40. The nucleic acid construct according to claim 39, wherein the promoter region, the substrate region α, the substrate region β, and the gene sequence are placed in the same nucleic acid molecule.

41. The nucleic acid construct according to claim 39, wherein the promoter region and the substrate region α are placed in one nucleic acid molecule, and the substrate region β and the gene sequence are placed in another nucleic acid molecule.

42. The nucleic acid construct according to claim 39, wherein a poly-A addition signal is functionally linked downstream of the gene sequence encoding protein X.

43. The nucleic acid construct according to claim 40, composed of two nucleic acid molecules and comprising one complementary region,
wherein:
a nucleic acid molecule 1 comprises the promoter region, the substrate region α, the substrate region β, and the gene sequence encoding protein X;
a nucleic acid molecule 2 comprises one complementary region comprising a first homologous region and a second homologous region;
the first homologous region is a region for which the substrate region α serves as a substrate in said recombination reaction, and the homology between these regions is not less than 40% and less than 100%;
the second homologous region is a region for which the substrate region β serves as a substrate in said recombination reaction, and the homology between these regions is not less than 40% and less than 100%; and
the recombination reaction that occurs between each homologous region and the substrate therefor results in expression of protein X.

44. The nucleic acid construct according to claim 40, composed of *n* nucleic acid molecules and comprising (*n*-1) complementary regions,
wherein:
a nucleic acid molecule 1 comprises the promoter region, the substrate region α, the substrate region β, and the gene sequence;
a nucleic acid molecule 2 comprises a first complementary region comprising a first homologous region and a second homologous region;
an *m*th nucleic acid molecule *m* comprises an (*m*-1)th complementary region comprising an (*m*-1)th substrate region and an *m*th homologous region;
the first homologous region is a region for which the substrate region α serves as a substrate in said recombination reaction, and the homology between these regions is not less than 40% and less than 100%;
an (*m*-1)th homologous region is a region for which the (*m*-1)th substrate region serves as a substrate in said recombination reaction, and the homology between these regions is not less than 40% and less than 100%;
an *n*th homologous region contained in a nucleic acid molecule *n* is a region for which the substrate region β serves as a substrate in said recombination reaction, and the homology between these regions is not less than 40% and less than 100%; and
the recombination reaction that occurs between each homologous region and the substrate therefor results in expression of protein X
(wherein the integer *n* is a constant of 3 ≤ *n* ≤ 10, and the integer *m* is a variable of 3 ≤ *m ≤ n*).

45. The nucleic acid construct according to claim 43, wherein the nucleic acid molecule 1 is a circular nucleic acid molecule comprising, downstream of the promoter region, the substrate region α, the substrate region β, and the gene sequence encoding protein X in this order, or a linear nucleic acid molecule comprising, from upstream to downstream, the substrate region β, the gene sequence encoding protein X, the promoter region, and the substrate region α in this order.

46. The nucleic acid construct according to claim 45, wherein the circular nucleic acid molecule comprises a cleavage site between the substrate region α and the substrate region β.

47. The nucleic acid construct according to claim 46, wherein the cleavage site is a restriction enzyme recognition site.

48. The nucleic acid construct according to claim 41, composed of three nucleic acid molecules and comprising one complementary region,
wherein:
a nucleic acid molecule 1-1 comprises the promoter region and the substrate region α;
a nucleic acid molecule 1-2 comprises the substrate region β and the gene sequence;
a first homologous region is a region for which the substrate region α serves as a substrate in said recombination reaction, and the homology between these regions is not less than 40% and less than 100%;
a second homologous region is a region for which the substrate region β serves as a substrate in said recombination reaction, and the homology between these regions is not less than 40% and less than 100%; and
the recombination reaction that occurs between each homologous region and the substrate therefor results in expression of protein X.

49. The nucleic acid construct according to claim 41, composed of (*n*+1) nucleic acid molecules and comprising (*n*-1) complementary regions,
wherein:
a nucleic acid molecule 1-1 comprises the promoter region and the substrate region α;
a nucleic acid molecule 1-2 comprises the substrate region β and the gene sequence;
a nucleic acid molecule 2 comprises a first complementary region comprising a first homologous region and a second homologous region;
an *m*th nucleic acid molecule *m* comprises an (*m*-1)th complementary region comprising an (*m*-1)th substrate region and an *m*th homologous region;
the first homologous region is a region for which the substrate region α serves as a substrate in said recombination reaction, and the homology between these regions is not less than 40% and less than 100%;
an (*m*-1)th homologous region is a region for which the (*m*-1)th substrate region serves as a substrate in said recombination reaction, and the homology between these regions is not less than 40% and less than 100%;
an *n*th homologous region contained in a nucleic acid molecule *n* is a region for which the substrate region β serves as a substrate in said recombination reaction, and the homology between these regions is not less than 40% and less than 100%; and
the recombination reaction that occurs between each homologous region and the substrate therefor results in expression of protein X
(wherein the integer *n* is a constant of 3 ≤ *n* ≤ 10, and the integer *m* is a variable of 3 ≤ *m* ≤ *n*).

50. The nucleic acid construct according to claim 40, composed of one circular nucleic acid molecule and comprising one complementary region,
wherein:
the circular nucleic acid molecule comprises, downstream of the promoter region, the substrate region α, a cleavage site, the substrate region β, and the gene sequence encoding protein X in this order, and comprises, upstream of the promoter region but downstream of the gene sequence, a complementary region comprising a first homologous region and a second homologous region;
the first homologous region is a region for which the substrate region α serves as a substrate in said recombination reaction, and the homology between these regions is not less than 40% and less than 100%;
the second homologous region is a region for which the substrate region β serves as a substrate in said recombination reaction, and the homology between these regions is not less than 40% and less than 100%; and
the recombination reaction that occurs between each homologous region and the substrate therefor results in expression of protein X.

51. The nucleic acid construct according to claim 40, composed of one circular nucleic acid molecule and comprising (*n*-1) complementary regions,
wherein:
the circular nucleic acid molecule comprises, downstream of the promoter region, the substrate region α, a cleavage site, the substrate region β, and the gene sequence encoding protein X in this order, and comprises, upstream of the promoter region but downstream of the gene sequence, (*n*-1) complementary regions;
a first complementary region comprises a first homologous region and a second homologous region;
an (*m*-1)th complementary region comprises an (*m*-1)th substrate region and an *m*th homologous region;
the first homologous region is a region for which the substrate region α serves as a substrate in said recombination reaction, and the homology between these regions is not less than 40% and less than 100%;
an (*m*-1)th homologous region is a region for which the (*m*-1)th substrate region serves as a substrate in said recombination reaction, and the homology between these regions is not less than 40% and less than 100%;
an *n*th homologous region contained in an (*n*-1)th complementary region is a region for which the substrate region β serves as a substrate in said recombination reaction, and the homology between these regions is not less than 40% and less than 100%; and
the recombination reaction that occurs between each homologous region and the substrate therefor results in expression of protein X
(wherein the integer *n* is a constant of 3 ≤ *n* ≤ 10, and the integer *m* is a variable of 3 ≤ *m* ≤ *n*).

52. The nucleic acid construct according to claim 50, wherein the cleavage site is a restriction enzyme recognition site.

53. The nucleic acid construct according to claim 40, composed of one linear nucleic acid molecule and comprising one complementary region,
wherein:
the linear nucleic acid molecule comprises, from upstream to downstream, the substrate region β, the gene sequence encoding protein X, the complementary region, the promoter region, and the substrate region α in this order;
the complementary region comprises a first homologous region and a second homologous region;
the first homologous region is a region for which the substrate region α serves as a substrate in said recombination reaction, and the homology between these regions is not less than 40% and less than 100%;
the second homologous region is a region for which the substrate region β serves as a substrate in said recombination reaction, and the homology between these regions is not less than 40% and less than 100%; and
the recombination reaction that occurs between each homologous region and the substrate therefor results in expression of protein X.

54. The nucleic acid construct according to claim 40, composed of one linear nucleic acid molecule and comprising (*n*-1) complementary regions,
wherein:
the linear nucleic acid molecule comprises, from upstream to downstream, the substrate region β, the gene sequence encoding protein X, the promoter region, and the substrate region α in this order, and comprises (*n*-1) complementary regions between the gene sequence and the promoter region;
a first complementary region comprises a first homologous region and a second homologous region;
an (*m*-1)th complementary region comprises an (*m*-1)th substrate region and an *m*th homologous region;
the first homologous region is a region for which the substrate region α serves as a substrate in said recombination reaction, and the homology between these regions is not less than 40% and less than 100%;
an (*m*-1)th homologous region is a region for which the (*m*-1)th substrate region serves as a substrate in said recombination reaction, and the homology between these regions is not less than 40% and less than 100%;
an nth homologous region contained in an (*n*-1)th complementary region is a region for which the substrate region β serves as a substrate in said recombination reaction, and the homology between these regions is not less than 40% and less than 100%; and
the recombination reaction that occurs between each homologous region and the substrate therefor results in expression of protein X
(wherein the integer *n* is a constant of 3 ≤ *n* ≤ 10, and the integer *m* is a variable of 3 ≤ *m* ≤ *n*).

55. The nucleic acid construct according to claim 39, wherein protein X is a protein that acts to decrease cell survival rate, or a protein whose intracellular expression is detectable.

56. The nucleic acid construct according to claim 55, wherein the gene sequence encoding protein X is a sequence of a suicide gene, a DNA damage-inducing gene, a DNA repair-inhibiting gene, a luminescent enzyme gene, a fluorescent protein gene, a cell surface antigen gene, a secretory protein gene, or a membrane protein gene.

57. The nucleic acid construct according to claim 39, wherein the base sequence generated by said recombination reaction between each substrate region and each homologous region is a gene sequence encoding protein Y, and the recombination reaction results in expression of protein X and protein Y.

58. The nucleic acid construct according to claim 57, wherein a sequence that enables polycistronic expression is placed between the substrate region β and the gene sequence encoding protein X.

59. The nucleic acid construct of claim 58, wherein the sequence that enables polycistronic expression is an IRES sequence or a 2A peptide-coding sequence.

60. The nucleic acid construct according to claim 57, wherein one of protein X and protein Y is a protein that acts to decrease cell survival rate, and the other is a protein whose intracellular expression is detectable.

61. The nucleic acid construct according to claim 60, wherein one of the gene sequence encoding protein X and the gene sequence encoding protein Y is a sequence of a suicide gene, a DNA damage-inducing gene, or a DNA repair-inhibiting gene, and the other is a sequence of a luminescent enzyme gene, a fluorescent protein gene, a cell surface antigen gene, a secretory protein gene, or a membrane protein gene.

62. A therapeutic agent for mismatch repair-deficient cancer, the agent comprising the nucleic acid construct according to any one of claims 39 to 61, wherein the gene sequence encoding protein X is a gene sequence encoding a protein that acts to decrease cell survival rate.

63. The therapeutic agent according to claim 62, wherein the gene sequence encoding protein X is a sequence of a suicide gene, a DNA damage-inducing gene, or a DNA repair-inhibiting gene.

64. An agent that detects and treats mismatch repair-deficient cancer, the agent comprising the nucleic acid construct according to claim 60 or 61.

65. A diagnostic agent for mismatch repair-deficient cancer, the agent comprising the nucleic acid construct according to any one of claims 39 to 61.

66. The diagnostic agent according to claim 65, wherein the gene sequence encoding protein X is a gene sequence encoding a protein whose intracellular expression is detectable.

67. A companion diagnostic agent for predicting an effect of an anticancer drug for mismatch repair-deficient cancer, the agent comprising the nucleic acid construct according to any one of claims 39 to 61.

68. The companion diagnostic agent according to claim 67, wherein the anticancer drug is an immune checkpoint inhibitor.

69. The companion diagnostic agent according to claim 67, wherein the gene sequence encoding protein X is a gene sequence encoding a protein whose intracellular expression is detectable.

70. A therapeutic method for mismatch repair-deficient cancer, the method comprising administering the nucleic acid construct according to any one of claims 39 to 61 to a patient with the mismatch repair-deficient cancer, wherein the gene sequence encoding protein X is a gene sequence encoding a protein that acts to decrease cell survival rate.

71. A method of detecting and treating mismatch repair-deficient cancer, the method comprising: administering, to a cancer patient, a nucleic acid construct according to claim 60 or 61 in which one of protein X and protein Y is a protein whose intracellular expression is detectable as a signal; and investigating whether or not the signal of the protein is detected from a cancer lesion.

72. A method of detecting and treating mismatch repair-deficient cancer, the method comprising: administering, to a cancer patient, a nucleic acid construct according to claim 60 or 61 in which one of protein X and protein Y is a secretory protein whose intracellular expression is detectable; and measuring the activity of the protein in blood separated from the patient after the administration.

73. A diagnostic method for mismatch repair-deficient cancer, the method comprising:
introducing the nucleic acid construct according to any one of claims 39 to 61 into cancer cells of a cancer patient; and
measuring expression of protein X.

74. The method according to claim 73, wherein the expression of protein X is measured by directly or indirectly measuring the activity of protein X.

75. The method according to claim 73, wherein the cancer cells are cells separated from the cancer patient, and the introduction of the nucleic acid construct into the cancer cells is carried out *in vitro.*

76. The method according to claim 73, wherein: protein X is a protein whose intracellular expression is detectable as a signal; the introduction of the nucleic acid construct into the cancer cells is carried out by administering the nucleic acid construct to the cancer patient; and whether or not the signal of the protein is detected from a cancer lesion is investigated.

77. The method according to claim 73, wherein: protein X is a secretory protein whose intracellular expression is detectable; the introduction of the nucleic acid construct into the cancer cells is carried out by administering the nucleic acid construct to the cancer patient; and the activity of the protein in blood separated from the patient after the administration of the nucleic acid construct is measured.

78. A method of predicting an effect of an anticancer drug for mismatch repair-deficient cancer, the method comprising:
introducing the nucleic acid construct according to any one of claims 39 to 61 into cancer cells of a cancer patient; and
measuring expression of protein X.

79. The method according to claim 78, wherein the anticancer drug is an immune checkpoint inhibitor.

80. The method according to claim 78, wherein the cancer cells are cells separated from the patient, and the introduction of the nucleic acid construct into the cancer cells is carried out *in vitro.*

81. The method according to claim 78, wherein: protein X is a protein whose intracellular expression is detectable as a signal; the introduction of the nucleic acid construct into the cancer cells is carried out by administering the nucleic acid construct to the patient; and whether or not the signal of the protein is detected from a cancer lesion is investigated.

82. The method according to claim 78, wherein: protein X is a secretory protein whose intracellular expression is detectable; the introduction of the nucleic acid construct into the cancer cells is carried out by administering the nucleic acid construct to the cancer patient; and the activity of the protein in blood separated from the patient after the administration of the nucleic acid construct is measured.
